Europäisches Patentamt

European Patent Office

Office européen des brevets

(19)

(11) Veröffentlichungsnummer: **0 334 841 B1**

(12) **EUROPÄISCHE PATENTSCHRIFT**

(45) Veröffentlichungstag der Patentschrift: **29.09.93**

(51) Int. Cl.⁵: **C12N 1/21**, C12N 15/31

(21) Anmeldenummer: **87905713.1**

(22) Anmeldetag: **28.08.87**

(86) Internationale Anmeldenummer:
**PCT/DE87/00392**

(87) Internationale Veröffentlichungsnummer:
**WO 88/01641 (10.03.88 88/06)**

(54) **MICROORGANISMEN AND PLASMIDE FÜR DIE 2,4-DICHLORPHENOXYESSIGSÄURE (2,4-D)-MONOOXIGENASE - BILDUNG UND VERFAHREN ZUR HERSTELLUNG DIESER PLASMIDE UND STÄMME.**

(30) Priorität: **29.08.86 DE 3629890**

(43) Veröffentlichungstag der Anmeldung:
**04.10.89 Patentblatt 89/40**

(45) Bekanntmachung des Hinweises auf die Patenterteilung:
**29.09.93 Patentblatt 93/39**

(84) Benannte Vertragsstaaten:
**AT BE CH DE FR GB IT LI LU NL SE**

(56) Entgegenhaltungen:

**Chemical Abstracts, vol. 103, no.3, 22 July 1985, (Columbus, Ohio, US), P.S. Amy et al "Characterization of aquatic bacteria and cloning of genes specifying partial degradation of 2, 4-dichlorophenoxyacetic acid", see page 154, abstract 17761s**

(73) Patentinhaber: **SCHERING AKTIENGESELL-SCHAFT Berlin und Bergkamen Müllerstrasse 170/178 Postfach 65 03 11 D-13303 Berlin(DE)**

(72) Erfinder: **STREBER, Wolfgang, R.
Bartningallee 24,
D-1000 Berlin 21(DE)**
Erfinder: **TIMMIS, Kenneth, N.
6a, avenue de la Foretaille
CH-1292 Chambésy(CH)**
Erfinder: **ZENK, Meinhart, H.
Pfeivestlstr. 17
D-8000 München 60(DE)**

Chemical Abstracts, vol. 99, no.9, 29 August 1983 (Columbus, Ohio, US), B. Friedrich et al "Transfer and expression of the herbicide-degrading plasmid pJP4 in aerobic autotrophic bacteria", see page 159, abstract 65163u

Chemical Abstracts, vol. 102, no.19, 13 May 1985 (Columbus, Ohio, US), R.H. Don et al "Transposon mutagenesis and cloning analysis of the pathways for degradation of 2, 4-dichlorophenoxyacetic acid and 3-chlorobenzoate in alcaligenes eutrophus JMP134 (pJP4), see page 157, abstract 161446q

Chemical Abstracts, vol. 102, no.13, April 1985 (Columbus, Ohio, US) R.H. Don et al "Genetic and physical map of the 2, 4-dichlorophenoxyacetic acid-degradative plasmid pJP4", see page 162, abstract 107177q

Biological Abstracts, vol. 84, no.7, 1987, (Philadelphia, Pa. US), W.R. Streber et al "Analysis, cloning and high-level expression of 2, 4-D monooxygenase gene tfdA of alcaligenes eutrophus JMP134", see abstract 66315

## Beschreibung

Die vorliegende Erfindung betrifft die gentechnische Herstellung von Plasmiden und Bakterienstämmen, die das Gen tfdA oder ein im Wesentlichen mit tfdA identisches Gen auf einem kurzen genau charakterisierbaren DNA-Abschnitt enthalten. Die neuen Plasmide und Mikroorganismen eignen sich besonders gut zur Produktion von 2,4-D-monooxigenase sowie als Ausgangsprodukt für die gentechnische Übertragung der 2,4-D-abbauenden Eigenschaften dieses Enzyms auf verschiedene Organismen (einschließlich der damit erreichbaren 2,4-D-Toleranz bei genetisch transformierten Pflanzen).

Die 2,4-D-monooxigenase ist ein Enzym, das in vielen 2,4-D-abbauenden Organismen den ersten Schritt bei der Metabo lisierung von 2, 4-Dichlorphenoxyessigsäure (2,4-D) katalysiert. Zu 2, 4-D-abbauenden Organismen gehören besonders Bodenbakterien wie z.B. Acinetobacter, Alcaligenes, Arthrobacter, Corynebacterium und Pseudomonas[(vgl. hierzu G.R. Bell, Can. J. Microbiol. 3:821 (1957); J.M. Bollag et al., J. Agric. Food Chem. 16:826 (1968); R.H. Don et al., J. Bacteriol. 145:681 (1981); W.C. Evans et al., Proc. Biochem. Soc. Biochem. J. 57:4 (1954); W.C. Evans et al., Biochem. J. 122:543 (1971); R.P. Fisher et al., J. Bacteriol. 135:798 (1978); T.I. Steenson et al., J. Gen. Microbiol. 16:146 (1957); J.M. Tiedje et al., J. Agric. Food Chem. 17:1080 (1969); J.E. Tyler et al., Appl. Microbiol. 28:181 (1974); J.M. Tiedje et al., J. Agric Food Chem. 17:1021 (1969)]. Sie spielen bei der Entgiftung des Erdbodens und der Abwässer von halogenierten aromatischen Verbindungen, wie sie gerade unter den landwirtschaftlich genützten Pestiziden und Herbiziden vorkommen, eine bedeutende Rolle.

In der Gruppe der 2,4-D-abbauenden Wildtypbakterien ist der Stamm Alcaligenes eutrophus JMP134 der bekannteste und am besten charakterisierte. Er beherbergt das etwa 80 Kilobasen große Plasmid pJP4, das sämtliche für den Abbau von 2,4-D wichtigen Gene enthält (R.H. Don et al., l.c.). Das Plasmid pJP4 wurde kürzlich auch isoliert und charakterisiert [R.H. Don et al., J. Bacteriol. 161:466 (1985)].

Fünf am Abbau von 2,4-D beteiligte Gene, bezeichnet als tfdB, tfdC,, tfdD, tfdE und tfdF, wurden durch Transposonmutagenese lokalisiert und in E. coli kloniert. Vier Genen konnte dabei von R.H. Don et al. [J. Bacteriol. 161:85 (1985)] durch biochemische Studien eine Einzymfunktion zugeordnet werden.

Es ist jedoch bisher noch nicht gelungen, das Gen tfdA auf dem Plasmid pJP4 oder auf einem Subfragment von diesem zu lokalisieren, zu isolieren und seine Struktur aufzuklären.

Durch die vorliegende Erfindung ist es nun erstmals gelungen, das Gen tfdA zu isolieren, klonieren und charakterisieren. Es ist damit möglich, dieses Gen für eine Übertragung auf andere Organismen verfügbar zu machen mit dem Ziel, die von tfdA kodierte 2,4-D-monooxigenase in diesen Organismen zur Expression zu bringen. Dabei kann es sich um Mikroorganismen handeln, aber auch höhere Organismen, wie z.B. Pflanzen, kommen dafür in Frage.

Die gezielte Übertragung von tfdA auf andere Mikroorganismen bietet die Möglichkeit, das Spektrum der von diesen Organismen abbaubaren Substanzen zu erweitern. Für Bakterien ist die Übertragung und Expression von Genen des gesamten 2,4-D-Abbaus bereits in J. Bacteriol. 145:681 (1981) und in Arch. Microbiol. 134:92 (1983) beschrieben worden. Es wurde jedoch noch kein Versuch unternommen, ein isoliertes tfdA-Gen in gramnegativen Bakterien wie Pseudonomas oder Alkaligenes zur Expression zu bringen. Ebensowenig ist ein solcher Versuch für Pflanzen bekannt. Die Metabolisierung von 2,4-D durch einige Pflanzenarten wurde verschiedentlich berichtet [Weed Science 24:557 (1976) und Z. Pflanzenphysiol. 110:395 (1983)]. In niederen Konzentrationen wirkt diese Substanz als auxinanaloges Pflanzenhormon und wird deshalb in der Zellkulturtechnik verwendet, in höheren Konzentrationen wirkt es auf die Pflanzenzelle wie auch auf die Gesamtpflanze als Wuchshemmstoff, was seinen Einsatz als Herbizid begründet. In einer haploiden Zellsuspensionzkultur von Nicotiana silvestris konnte durch Adaption an steigende Konzentrationen von 2,4-D eine Toleranz gegenüber diesem sythetischen Wuchsstoff erreicht werden, die auf einer erhöhten Metabolisierungsrate beruht [M.H. Zenk, 1974. Haploids in physiologicai and biochemical research. In Haploids in higher plants. Advances and potential. Proceedings of the First International Symposium, Guelph, Ontario, Canada. p339]. Da das Gen tfdA die Abspaltung der Seitenkette von 2,4-D vermittelt, könnte die Fähigkeit, 2,4-D zu inaktivieren, mit Hilfe des aus Bakterien gewonnenen Gens auf all diejenigen Pflanzen übertragen werden, für die eine derartige gentechnische Manipulation möglich ist. Verfahren zur Übertragung fremder Gene auf Pflanzen und deren Nachkommenschaft sind heute bereits für einige Pflanzenarten erprobt [M. De Block, L. Herrera-Estrella, M. Van Montagu, J. Schell, and P. Zambryski. 1984. Expression of foreign genes in regenerated plants and in their progeny. EMBO J. 3:1681 sowie R.D. Shillito, M.W. Saul, J. Paszkowski, M. Müller, and J. Potrykus, 1985. High efficiency direct gene transfer to plants. Bio/technology 3:1099] und werden in nächster Zeit eine breitere Anwendbarkeit erlangen.

In die genetische Transformation von Pflanzen werden große Hoffnungen gesetzt, insbesondere was die Erzeugung neuer, für den Menschen wichtiger Nutzpflanzen betrifft [J.L. Marx, 1985. Plant gene transfer becomes a fertile field. Science 230:1148]. Mit dem Gen tfdA stünde dafür eine neue genetisch übertragba-

EP 0 334 841 B1

re und in vivo selektierbare Eigenschaft in Form einer Toleranz gegenüber einem Wachstumshemmstoff zur Verfügung, wie sie bisher nur für wenige Antibiotika- bzw. Herbizidtoleranzen bekannt ist [R.T. Fraley et al. 1983. Expression of bacterial genes in plant cells. Proc. Natl. Acad. Sci. USA 80:4803 und Nature 317:741 (1985)].

Die vorliegende Erfindung besteht zunächst darin, daß unter Anwendung eines an sich bekannten Mutationsverfahrens von einem Bakterienstamm, der 2,4-D als Wachstumssubstrat verwenden kann, wie z.B. Alcaligenes eutrophus JMP134, eine bisher nicht bekannte Mutante hergestellt wird, in welcher das Strukturgen für die 2,4-D-monooxigenase inaktiviert ist. Diese Mutante wird unter Anwendung der bekannten Verfahren der DNA-Rekombination in vitro, der Transformation mit rekombinanter DNA und des konjugativen Transfers von DNA zur Selektion von rekombinanter DNA eingesetzt, welche tfdA oder mit tfdA im Wesentlichen identische Gene enthält.

Gegenstand der Erfindung sind Plasmide, die das Gen tfdA oder mit tfdA im Wesentlichen identische Gene enthalten, sowie Plasmide, weiche Teile dieser Gene einschließlich der Promotorregion enthalten.

Die erfindungsgemäßen Plasmide können hergestellt werden, indem man DNA aus Wildtypbakterien, welche Gene für die Metabolisierung von 2,4-D oder 2,4-D-ähnlichen Verbindungen besitzen, mit Restriktionsendonukleasen verdaut und die entstandenen DNA-Fragmente mit Hilfe einer DNA-Ligase mit einem Plasmidvektor verknüpft, welcher zuvor durch das gleiche Restriktionsenzym in seine lineare Form umgewandelt worden ist.

Unter den so entstandenen rekombinanten Plazmiden können die erfindungsgemäßen tfdA-haltigen Plasmide dadurch identifiziert werden, daß man die Plasmide in Bakterienstämme bringt, aus denen dann die tfdA-haltigen Klone direkt aufgrund einer von tfdA vermittelten Fähigkeit selektiert werden können.

Solche Bakterienstämme besitzen die Eigenschaft, die in einer enzymatischen Reaktion in vivo von der 2,4-D-monooxigenase gebildeten Produkte, nicht jedoch deren Substrate als Kohlenstoff- und Energiequellen verwenden zu können.

Beispiele für Stämme mit der genannten Eigenschaft sind die erfindungsgemäßen tfdA-Mutanten, in denen alle Gene für den Abbau von 2, 4-Dichlorphenol aktiv sind, ferner der Stamm Alcaligenes eutrophus JMP222, welcher Abbauwege für Phenol besitzt, sowie Pseudomonas sp. B13, der 4-Chlorphenol verwerten kann. Aber auch eine Reine weiterer Bakterienztämme, welche verschieden substituierte Phenole abbauen können und vorwiegend in der Gruppe der gram-negativen Baktreien angetroffen werden, sind als Rezipienten zur Selektion und Identifizierung von klonierten tfdA-Genen vorstellbar.

Als Klonierungsvektoren werden bevorzugt Plasmide mit breitem Wirtsbereich verwendet, die in der Lage sind, sich auch in anderen Bakterienstämmen als E. coli replikativ zu vermehren. Aber auch Plasmide, die ihre DNA oder Teile ihrer DNA durch Integration in das Genom der Wirtszelle weitervermehren können, kommen dafür in Frage.

Als Verfahren zur Einführung von DNA in die lebenden Zellen besagter Bakterienstämme eignet sich einmal die direkte Transformation dieser Stämme, sofern Methoden dafür bekannt sind. So gibt es z.B. Transformationsverfahren für Pseudomonas und verwandte gramnegative Bakterien [A.A. Mercer and J.S. Loutit. 1979. Transformation and transfection of Pseudomonas aeruginosa: effects of metal ions. J Bacteriol. 140:37-42; A.M. Chakrabarty, J.R. Mylroie, D.A. Friello, and J.G. Vacca. 1975. Transformation of Pseudomonas putida and Escherichia coli with plasmid-linked drug-resistance factor DNA. Proc. Natl. Acad. Sci. USA 72:3647-3651]. Für alle gramnegativen Bakterien anwendbar und wesentlich effektiver ist hingegen die Transformation eines E. coli Stammes nach einer literaturbekannten Methode mit anschließendem konjugativen Transfer der Plasmide in die betreffenden Bakterienstämme.

Für den konjugativen Transfer klonierter DNA werden bevorzugt mobilisierbare Plasmidvektoren verwendet. Die für den Transfer erforderlichen Gene werden dabei entweder von sogenannten Helferplasmiden oder von speziell dafür konstruierten mobilisierenden Stämmen zur Verfügung gestellt.

Aus den besagten Bakterienstämmen, welche direkt durch Selektion auf geeigneten Wachstumssubstraten gewonnen werden, oder aus Klonen von E. coli, welche durch das geschilderte Verfahren oder andere bekannte Testsysteme auf Expression von tfdA identifiziert werden, lassen sich die tfdA enthaltenden Plasmide nach bekannten Verfahren als eindeutig definierbare chemische Substanzen isolieren.

Der Vorteil der direkten Selektion tfdA-haltiger Stämme durch Wachztumstest besteht vor allem darin, daß er im Gegensatz zu den bisher bekannten Verfahren, die alle auf relativ aufwendigen Enzymtests beruhen, die Identifizierung eines tfdA-haltigen Plasmids unter einer sehr großen Anzahl verschiedener Plasmide ermöglicht, wie sie bevorzugt bei der Herstellung von Genbanken, d.h. der randomisierten Klonierung von DNA-Bruchstücken genomischer DNA entstehen. Der Vorteil besteht damit in der breiten Anwendbarkeit des Verfahrens, denn Klonierung von tfdA-Genen ist damit nicht mehr auf Wildtypstämme beschränkt, die das Gen tfdA auf einem gut isolierbaren Plasmid tragen, sondern ist aus beinahe allen Wildtypstämmen möglich, auch solchen, die das Gen auf einem schlecht zugänglichen Plasmid oder dem

4

Chromosom enthalten.

Die erfindungsgemäßen Plasmide werden erhalten, indem man z.B. das aus Alcaligenes eutrophus JMP134 stammende Plasmid pJP4, auf dem die Gene für den Abbau von 2,4-D liegen, mit der Restriktions-endonuklease HindIII schneidet, die entstandenen DNA-Fragmente elektrophoretisch trennt und die einzelnen isolierten Fragmente mit dem HindIII-geschnittenen und dephosphorylierten Vektor pVK101 ligiert. Der mobilisierende Stamm E. coli S17-1 wird mit der rekombinanten DNA transformiert und plasmidhaltige Stämme werden auf tetracyclin-haltigem Medium selektiert. Aus Stämmen, welche durch Restriktionsanalyse identifizierte rekombinante Plasmide enthalten, wird die Plasmid-DNA durch Kunjugation auf die oben erwähnte tfdA-Mutante JMP134:Tn5-2 übertragen und die Expression des klonierten tfdA-Gens durch Wachstum des Wirtsstammes auf 2,4-D-haltigem Minimalmedium nachgewiesen. Auf diese Weise kann das Plasmid pVJH21 identifiziert werden, welches das Gen tfdA auf einem 21 Kilobasen großen HindIII-Fragment, stammend aus pJP4, enthält. Durch Isolierung des Plasmids und anschließende Restriktionsanalyse kann die Identität des klonierten Fragments bestätigt werden.

Die erfindungsgemäßen Plasmide können außerdem durch Subklonieren aus rekombinanten Plasmiden hergestellt werden, die das Gen tfdA enthalten. Dazu werden diese Plasmide mit einer oder mehreren Restriktionsendonukleasen verdaut und die entstandenen Bruchstücke werden mit Hilfe einer DNA-Ligase mit einem Vektorplasmid verknüpft, das mit den gleichen Restriktionsendonukleasen in seine lineare Form überführt worden ist. Aus den entstandenen Plasmiden können tfdA-haltige auf die oben geschilderte Weise selektiert werden.

So kann z.B. das Plasmid pGJS3 dadurch hergestellt werden, däß mit SacI erzeugte DNA-Fragmente des Plasmids pVJH21 mit dem SacI-geschnittenen Vektorplasmid pGSS33 verknüpft werden. Aus der Anzahl der neukombinierten Plasmide können solche selektiert werden, die ein intaktes tfdA-Gen enthalten, indem die rekombinante Plasmid-DNA zuerst durch Transformation in den mobilisierenden Stamm E. coli S17-1 und dann von dort durch Konjugation in die tfdA-Mutante JMP134:Tn5-2 überführt wird. Durch Selektion auf 2,4-D-haltigem Medium werden Plasmide identifiziert, welche ein 3 Kilobasen großes SacI-Insert enthalten, dessen Herkunft durch Restriktionsanalyse eindeutig auf das 21 Kilobasen große HindIII-Fragment aus pVJH21 und damit auf pJP4 zurückgeführt werden kann.

Durch Subklonieren von tfdA enthaltenden DNA-Abschnitten können auch solche erfindungsgemäßen Plasmide hergestellt werden, die sich je nach dem verfolgten Anwendungsziel in der Art der verwendeten Plasmidvektoren unterscheiden.

So kann z.B. das 3 Kilobasen große SacI-Fragment aus pGJS3 in den Vektor pKT231 umkloniert werden, wobei die Plasmide pKJS31 und pKJS32 entstehen, die gegenüer pGJS3 durch eine günstigere Restriktionskarte und die in vielen gram-negativen Bakterien gut exprimierte Kanamycin-Resistenz Vorteile für die weitere Charakterisierung von tfdA bieten. Als Verfahren zum Nachweis der tfdA-Expression durch von pKT231 abgeleitete Plasmide, wie sie im Folgenden beschrieben werden, wird der konjugative Transfer aus E. coli S17-1 in den Stamm Alcaligenes eutrophus JMP222 mit anschließendem Test auf Verwertung von Phenoxyessigsäure als Wachstumssubstrat bevorzugt. Dieses System bietet gegenüber den tfdA-Mutanten die Vorteile, daß bei diesem Rezipientenstamm der konjugative Transfer mit höherer Effizienz stattfindet und mit der Kanamycin-Resistenz ein weiterer selektierbarer Marker zur Verfügung steht.

Durch Subklonieren können tfdA enthaltende DNA-Fragmente auch in Expresssionsvektoren eingebaut werden, auf denen tfdA-Gene mit Hilfe eines fremden Promotors exprimiert werden.

So können z.B. das 2.8 Kilobasen große SaCl/SalI-Fragment, das 2.0 Kilobasen große BamHI/SalI-Fragment und das 1.4 Kilobasen große XbaI/SalI-Fragment in die Expressionsvektoren pT7-5 und pT7-6 direkt im Anschluß an einen Phagenpromotor eingebaut werden, durch den die Genexpression mit Hilfe einer Promotor-spezifischen RNA-Polymerase gezielt angeschaltet werden kann. Das von den neu kombinierten Plasmiden pTJSS'035, pTJS'B435 und pTJS'X535, deren Herstellung in den Beispielen 8, 9 und 10 beschrieben wird, exprimierte tfdA-Genprodukt kann durch spzifische Markierung mit radioaktivem Methionin und anschließende Gelelektrophorese identifiziert werden. Das System kann ferner dazu benutzt werden, in einem E. coli-Stamm große Mengen an 2,4-D-monooxigenase zu erzeugen, was die Reinigung und die anschließende proteinchemische und enzymatische Charakterisierung des Genprodukts im Vergleich zu seiner Isolierung aus dem Wildtypstamm wesentlich erleichtert, wodurch weitere biotechnologische Anwendungsmöglichkeiten erst erforschbar werden.

Die Subklonierung von tfdA enthaltenden DNA-Fragmenten in Phagenvektoren vom Typ des M13-Phagen ermöglicht die Herstellung von Einzelstrang-DNA. Sie kann zur Bestimmung der Basensequenz nach dem Verfahren von Sanger [F` Sanger, S. Nicklen, and A.R. Coulson. 1977. DNA sequencing with chain-terminating inhibitors. Proc. Natl. Acad. Sci. USA 75:5463] eingesetzt werden, sie kann ferner zur gezielten Mutagenese einzelner Basen mittels Oligonukleotiden dienen, ein bekanntes Verfahren, das die Erzeugung von Restriktionsschnittstellen in einem Gen oder die Änderung der Aminosäureabfolge erlaubt.

Die Einfügung von Restriktionsstellen in Genen erweitert deren Anwendungsmöglichkeiten, weil dadurch Schnittstellen für den Einbau von fremden Promotoren und die Verknüpfung von Genfragmenten zur Bildung von Fusionsproteinen geschaffen werden, wie sie z.B. für die Expression prokaryotischer Gene in Eukaryonten nötig sind.

Die Subklonierung des 2.8 Kilobasen großen SaCl/Sall-Fragments aus pKJS32 in die Phagenvektoren M13tg130 und M13tg131, wie sie im Beispiel 16 beschrieben wird, stellt eine Möglichkeit dar, um das Gen tfdA für eine Sequenzierung zugänglich zu machen. Durch Modifikation der DoppelstrangDNA der rekombinanten Phagen MJSS'030 und MJSS'031 können weitere Veränderungen an der Insert-DNA vorgenommen werden.

Die erfindungsgemäßen Plasmide können ferner durch Modifikation aus tfdA-haltigen Plasmiden hervorgehen. Durch Behandlung solcher Plasmide mit einer oder mehreren Restriktionsendonukleasen, eventueller Behandlung mit Exonukleasen und anschließender Wiederverknüpfung der DNA-Enden zu einem ringförmigen Molekül, erhält man deletierte, d.h. um einen bestimmten DNA-Abschnitt verkleinerte Plasmide, welche ein weiterhin intaktes tfdA-Gen enthalten können.

So ist es z.B. möglich, durch Entfernen von definierten DNA-Fragmenten aus den Plasmiden pKJS31 und pKJS32 das darin enthaltene 3 Kilobasen große Insert bis zu der Xbal-Schnittstelle auf der einen Seite und bis zu der Sall-Schnittstelle auf der anderen Seite zu verkleinern, ohne daß die Aktivität von tfdA dabei verloren geht. Die so erzeugten, in den Beispielen 4, 5 und 7 beschriebenen Plasmide pKJSB330, pKJS(X)-630 und pKJS32RHΔS' sind in der Lage, tfdA-Genaktivität zu exprimieren. Auf diese Weise kann die Lage des Gens auf ein 1.4 Kilobasen großes Xbal/Sall-Fragment eingegrenzt werden.

Durch die in Beispiel 16 beschriebene Deletion von verschieden langen DNA-Abschnitten aus den doppelsträngigen Formen der rekombinanten Phagen MJSS' 030 und MJSS'031 mittels Exonuklease III, kann eine Reihe von Phagen erzeugt werden, bei denen jeweils verschiedene Bereiche der klonierten DNA dem Startpunkt für die Sequenzierung am nächsten liegen. Die Sequenz eines 2 Kilobesen großen BamHI/Sall-Fragments kann dann aus den überlappend sequenzierten Teilbereichen zusammengesetzt werden. Aus der Kenntnis der Basensequenz lassen sich dann weitere Eigenschaften der tfdA enthaltenden DNA ableiten, wie z. B. die Lage und Länge der kodierenden Region und die Lage von Restriktionsschnittstellen, was für die weitere Verwendung von tfdA von erheblichem Nutzen sein kann.

Durch Deletion von DA-Fragmenten aus tfdA enthaltenden Plasmiden bzw. durch Subklonieren von DNA-Fragmenten können auch weitere erfindungsgemäße Plasmide erhalten werden, die nur noch Teile eines tfdA-Gens enthalten. So entsteht z.B. durch Subklonieren des 1.5 Kilobasen großen EcoRI/BamHI-Fragments aus pKJS32 in pKT231 das Plasmid pKJEΔB130, das nur noch das 5'-Ende von tfdA, d.h. den Promotor und die für den N-Terminus kodierende Sequenz, enthält und das nicht mehr zur Expression eines enzymatisch aktiven Genprodukts befähigt ist. Teile von Genen können zur Konstruktion von Plasmiden Verwendung finden, bei denen das 5'-Ende einer kodierenden DNA einschließlich deren Promotor im gleichen Leseraster mit dem 3'-Ende einer anderen kodierenden DNA verknüpft sind und die somit zur Expression eines sogenannten Fusionsproteins in denjenigen Organismen führen, die den jeweiligen Promotor erkennen.

Eine weitere Möglichkeit der Modifikation der erfindungsgemäßen Plasmide besteht in der Insertion eines fremden DNA-Segments. Die Insertion kann zur Einfügung neuer funktioneller DNA-Sequenzen dienen, z.B. von Restriktionsschnittstellen, Promotoren, Resistenzgenen, Translations- und Transkriptionsstopsignalen. Die Insertion eines Teils einer fremden Gensequenz kann auch zur Bildung von Fusionsproteinen führen.

Ein Beispiel für die Insertion einer fremden DNA ist die Insertion des Omega-Fragments in die BglII-Schnittstelle des Plasmids pTJS'X535, die inmitten der kodierenden Region von tfdA gelegen ist (Beispiel 11). Mit diesem Fragment werden Translations-Stopkodons in allen Leserastern und Transkriptions-Stopsignale zusammen mit einer selektierbaren Antibiotikaresistenz eingebaut. Wie durch spezifische radioaktive Markierung des Genprodukts sowie durch in vivo Enzymetest auf 2.4-D-monooxigenase (Beispiel 15) nachgewiesen werden kann, führt dies zur Expression eines verkürzten, nicht mehr enzymatisch aktiven Proteins durch das rekombinante Plasmid pTJS'X535omega.

Klonierte Fragmente, die tfdA oder Teile von tfdA enthalten, können zur Detektion homologer DNA-Sequenzen und damit zum Auffinden von tfdA-Genen in anderen Organismen dienen. Dazu werden diese Fragmente aus den erfindungsgemäßen Plasmiden mit Hilfe geeigneter Restriktionsenzyme herausgeschnitten, isoliert und nach literaturbekannten Methoden z.B. durch Radionuklideinbau markiert. Durch das bekannte Verfahren der Hybridisierung können in der gesamten DNA eines Organismus oder in einer daraus hergestellten Genbank Fragmente identifiziert werden, die eine Homologie mit tfdA aufweisen. Auf diese Weise können auch unter einer Population verschiedener Organismen jene erkannt werden, die eine solche mit tfdA homologe Sequenz enthalten. Das Verfahren kann zum Aufspüren neuer 2,4-D abbauender Organismen sowie zum Auffinden von tfdA-Genen in diesen Organismen verwendet werden.

tfdA-Gene können entweder direkt mit Hilfe der erfindungsgemäßen Plasmide, soweit sie dafür geeignet sind, oder nach Veränderung mittels bekannter Verfahren der Gentechnik auf andere Organismen übertragen werden. Die Übertragung kann den Zweck verfolgen, die von tfdA kodierte 2,4-D-monooxigenase in diesen Organismen zu exprimieren und diesen damit die Eigenschaft zu verschaffen, 2,4-D oder 2,4-D-ähnliche Substanzen abbauen zu können.

So ist es z.B. möglich, durch den in Beispiel 14 beschriebenen konjugativen Transfer tfdA-haltiger Plasmide mit breitem Wirtsbereich in verschiedene gram-negative Bakterien diesen die Fähigkeit zu verleihen, 2,4-D oder 2,4-D-ähnliche Substanzen zu dem korrespondierenden Phenol umzusetzen. In Verbindung mit einer dem jeweiligen Stamm eigenen Abbauleistung kann dies zu einer Erweiterung des Spektrums der von diesem Stamm abbaubaren Verbindungen führen. Der Stamm Alcaligenes eutrophuz JMP222 besitzt z.B. Gene für die Metabolisierung von Phenol. Die Expression von tfdA vermittelt ihm somit die völlig neue Möglichkeit, Phenoxyessigsäure als Wachstumssubstrat zu nutzen. Der Stamm Pseudomonas B13 kann sowohl Phenol als auch 4-Chlorphenol metabolisieren, und erhält durch tfdA auf analoge Weise die Fähigkeit zum Wachstum auf Phenoxyessigsäure und 4-Chlorphenoxyessigsäure. Ebenso ist der Abbau verschieden substituierter Phenoxyessigsäuren wie 4-Chlor-2-methylphenoxyessigsäure, 2,4,5-Trichlorphenoxyessigsäure und ähnlicher Verbindungen durch Organismen denkbar, welche tfdA enthalten und die korrespondierenden Phenole umsetzen können. Wenn die damit erreichbare Erweiterung des Spektrums der Abbauleistungen eines Organismus auch nicht in jedem Falle zur Schaffung einer neuen, in der Natur bisher nicht verwirklichten Eigenschaft führt, so kann sie doch zu einer qualitativen Verbesserung dienen, insofern als damit Stämme mit einer im Vergleich zu Wildtypstämmen höheren Abbauleistung konstruiert werden können, oder solche mit einer höheren Toleranz gegenüber möglicherweise toxischen Substraten oder Produkten eines Abbauweges. Solche Stämme würden sich speziell unter Bedingungen als nützlich erweisen, wie sie nicht in natürlicher Umgebung, sondern in künstlichen Systemen wie z.B. in Kläranlagen zur Reinigung industrieller Abwässer vorherrschen. Eine Übersicht über die bisher auf dem Gebiet des mikrobiellen Abbaus halogenierter aromatischer Verbindungen mit Hilfe neukonstruierter Stämme erreichbaren Leistungen wurde kürzlich veröffentlicht [D. Ghosal, I.-S. You, D.K. Chatterjee, A.M. Chakrabarty. 1985. Microbial degradation of halogented compounds. Science 228:135-142].

Eine weitere Verwendungsmöglichkeit für das Gen tfdA stellt seine Übertragung auf Pflanzen dar. Methoden zur Transformation von Pflanzen mit fremder DNA sind in der Literatur bekannt und erprobt. Bisher sind im wesentlichen zwei Verfahren zu unterscheiden: Die eine bedient sich der Funktionen des Ti-Plasmids aus Agrobacterium tumefaciens, die andere basiert auf der Transformation pflanzlicher Protoplasten unter Zuhilfenahme physikalischer und chemischer Agentien wie Elektroporation, Hitzeschock, Kalziumchlorid- oder Polyethylenglykolbehandlung. Während die Transformation pflanzlicher Zellen durch die eine oder andere Methode prinzipiell immer möglich ist, gelingt nicht in jedem Fall die Regeneration ganzer Pflanzen aus einzelnen Zellen oder Zellgeweben. Dies stellt im Moment das Haupthindernis für die gentechnische Manipulation von monokotylen Pflanzen dar.

Bevorzugtes Ziel der Transformation von Pflanzen mit tfdA ist die Expression der Geneigenschaft, d.h. der 2,4-D-monooxigenase-Aktivität in der transformierfen Pflanze. Die Pflanze wäre damit in der Lage, die in niederen Konzentrationen als auxinanaloges Pflanzenhormon und in hohen Konzentrationen als Wuchshemmstoff wirkenden Derivate der Phenoxyessigsäure zu den phytochemisch unwirksamen Phenolen abzubauen. Somit käme dieser Eigenschaft eine Bedeutung als selektierbarer Marker für genetisch transformierte Pflanzen zu.

Damit ein prokaryotisches Gen wie tfdA in Pflanzenzellen exprimiert werden kann, muß es mit den für die Transkription und die Translation notwendigen pflanzenspezifischen Signalsequenzen gekoppelt sein. Es sind Hinweise bekannt, daß diese Koppelung durch die zufallsgesteuerte Integration eines Gens in das Genom einer damit transformierten Pflanze stattfinden kann. Prokaryotische Gene werden jedoch bevorzugt in vitro mit den geeigneten pflanzenspezifischen Expressionssignalen kombiniert. Ein allgemein anwendbares Verfahren besteht z.B. darin, däß das 5'-Ende eines Strukturgens einschließlich des dazugehörigen pflanzlichen Promotors mit der kodierenden Sequenz des prokaryotischen Gens in der Weise verknüpft wird, daß in einer damit transformierten Pflanzenzelle ein Fusionsprotein sythetisiert wird, das aus den N-terminalen Aminosäuren des Pflanzenproteins und einem überwiegenden Anteil des prokaryotischen Proteins besteht und das in seinen wesentlichen Eigenschaften dem prokaryotischen Protein gleicht [R.T. Fraley et al. 1983. Expression of bacterial genes in plant cells. Proc. Natl. Acad Sci. USA 80:4803, J. Paszkowski, and M.W. Saul, 1986. Direct gene transfer to plants. In S.P. Colowick and N.A. Kaplan (ed.), Methods in Enzymology 118:668]. Eine ähnliche Methode verwendet nur den pflanzlichen Promotor einschließlich eines Teils der 5'-untran sla tierten Region der transkribierten m-RNA und verzichtet auf das 5'-Ende der kodierenden Region eines pflanzlichen Gens. Bei der Expression in Pflanzen dient dann ein ATG-Kodon des prokaryotischen Genteils als Start für die Translation. In analoger Weise lassen sich die Expressionssignale von Pflanzenviren verwenden [N. Brisson, and T. Hohn. 1986, Plant virus vectors; cauliflower mosaic virus. In S. P. Colowick and N. O. Kaplan (ed.), Methods in Enzymology 118:659]. Vektoren für die Expression prokaryotischer Gene in Pflanzen sind literaturbekannt und stehen allgemein zur Verfügung.

Voraussetzung für die Konstruktion eines in Pflanzen exprimierbaren Gens aus einem prokaryotischen Gen mit Hilfe der genannten Vektorplasmide ist die Kenntnis der Basensequenz, wie sie für das Gen tfdA vorliegt. Sie erlaubt die exakte Lokalisation von Restriktionsschnittstellen und ermöglicht damit die basengenaue Verknüpfung der verschiedenen DNA-Segmente. Ferner ermöglicht sie erst die gezielte Mutagenese zur Schaffung neuer funktionell wichtiger DNA-Sequenzen, wie z.B. die Einführung von neuen Restriktionsschnittstellen.

Im Nachstehenden wird die Erfindung an Hand von Zeichnungen und Beispielen naher erläutert. In den Beispielen wurden in der Fachwelt allgemein bekannte und erprobte Methoden der Gentechnik benutzt. Die Klonierung von Genen und die Analyse von Genstrukturen wurden nach den gentrechnischen Standardverfahren durchgeführt, die bereits Eingang in die Lehrbücher von E.-L. Winnacker (Gene und Klone, Verlag Chemie, Weinheim, 1985) und von R. W. Old und S.B. Primrose (Principles of gene manipulation, Blackwell Scientific Publ., Oxford, 1985) gefunden haben. In I.K. Setlow und A. Hollaender (Genetic engineering: principles and methods. Plenum Publ. Corp., N.Y.) z.B. werden die Methoden und Techniken jeweils auf den neuesten Stand gebracht. Neben den speziellen Veröffentlichungen wurden vielfach auch allgemeine Arbeitsvorschriften in Form von Labörhandbüchern [R.W. Davis et al., Advanced bacterial genetics: a manual for genetic engineering; T. Maniatis et al., Molecular cloning; T.J. Silhavy et al., Experiments with gene fusions, alle aus Cold Spring Harbour Lab., N.Y.; A. Pühler and K.N. Timmis, Advanced molecular genetics, Springer, Berlin, 1984; D.M. Glover, DNA cloning: a practical approach, JRL Press, Oxford, Washington, D.C., 1985] herangezogen.

Am 28. August 1986 wurden bei der Deutschen Sammlung von Mikroorganismen (DSM) in Göttingen, Bundesrepublik Deutschland, folgende Mikroorganismen hinterlegt (Hinterlegungsnummer):

| | |
|---|---|
| E. coli HMS 174 (pT7-5) | (DSM 3829) |
| E. coli HMS 174 (pT7-6) | (DSM 3830) |
| E. coli JA 221 (pGSS33) | (DSM 3831) |
| E. coli LE 392 (pTJSS'035) | (DSM 3832) |
| E. coli HB101 (pVK101) | (DSM 3833) |
| E. coli SK1592 (pKT231) | (DSM 3834) |
| E. coli S17-1 (pKJS31) | (DSM 3835) |
| E. coli S17-1 (pKJS32 RHΔS') | (DSM 3836) |
| E. coli S17-1 (pKJS(X)630) | (DSM 3837) |
| E. coli SBC107 (pRME1) | (DSM 3838) |
| E. coli K38 (pGT1-2/pTJS'X535) | (DSM 3839) |
| Alcaligenes eutrophus JMP134 (pJP4) | (DSM 3840) |
| Alcaligenes eutrophus JMP222 | (DSM 3841) |
| Alcaligenes eutrophus JMP134:Tn5-2 (pJP4:Tn5-2) | (DSM 3842) |
| Alcaligenes eutrophus JMP134:Tn5-4 (pJP4:Tn5-4) | (DSM 3843) |

Beschreibung der Abbildungen:

Abkürzungen:

Ap      - Ampicillinresistenz
Km      - Kanamycinresistenz
kb      - Kilobasen
M      - Molekulargewichtsmarker
kD      - Kilodalton

Abbildungen:

| | |
|---|---|
| Abb. 1 | - zeigt die Herkunft und die Restriktionskarte des 21 kb großen, aus pJP4 klonierten HindIII-Fragments (Beispiel 1) und der davon abgeleiteten Subfragmente (Beispiel 3 bis 7), sowie die Expression des Phenoxyessigsäureabbaus in Alcaligenes eutrophus JMP222 durch die entsprechenden Plasmide mit breitem Wirtsbereich (Beispiel 14). Der Pfeil markiert die genaue Lage von tfdA im Plasmid pJP4. |
| Abb. 2, 3, 4 | - zeigen die von pKT231 abgeleiteten Plasmide, die tfdA oder Teile von tfdA enthalten (Beispiele 3 bis 7). Die dünnen Linien bezeichnen den vom Vektorplasmid stammenden Anteil, die dicken Linien bezeichnen das aus pJP4 stammende Insert. |
| Abb. 5, 6, 7 a | - zeigen die von den T7-Promotorplasmiden pT7-5 und pT7-6 abgeleiteten Plasmide mit tfdA enthaltenden Insertfragmenten (Beispiele 8 bis 11). Die dünnen Linien bezeichnen den vom Vektorplasmid stamenden Anteil, die dicken Linien bezeichnen das aus pJP4 stammende Insert. |
| Abb. 7 b | - zeigt die Insertion des Omegafragments in das Plasmid pTJS'X535 (Beispiel 11). |
| Abb. 8 | - zeigt spezifisch radioaktiv markierte Proteine auf dem Autoradiogramm eines Polyacrylamid gels, die von den tfdA enthaltenden Plasmiden mit Hilfe des T7-Promotors in hoher Ausbeute exprimiert werden (Beispiel 15). 1: pTJSS'035, 2: pTJS'B435, 3: pTJS'X535, 4: pTJSS'036, 5: pTJS'B436, 6: pTJS'X536. a bezeichnet vollständig markiertes Gesamtzellprotein, b bezeichnet nach Induktion des T7-Promotors spezifisch markiertes Protein. |
| Abb. 9 | - zeigt das von Plasmid pTJS'X535omega mit Hilfe des T7-Promotors exprimierte, verkürzte tfdA-Genprodukt (2b) im Vergleich zum unverkürzten, von pTJS'X535 exprimierten Protein (3b) und zum insertfreien Vektorplasmid pT7-5 (1b). a bezeichnet vollständig markiertes Gesamtzellprotein, b bezeichnet nach Induktion des T7-Promotors spezifisch markiertes Protein. |
| Abb. 10 a/ 10 b | - zeigt die Basensequenz des 2058 Basen langen BamHI/SalI-Fragments, auf dem das Gen tfdA vom 5'-Ende zum 3'-Ende hin transkribiert wird. Die Pfeile bezeichnen den Anfang und das Ende der kodierenden Region von tfdA (Beispiel 16). |

Beispiel 1: Herstellung des Plasmids pVJH21

a) Isolierung der Plasmide pJP4 und pVK101

Alcaligenes eutrophus JMP134 wird in 250 ml PYF-Medium (Pepton, 3 g/l; Hefeextrakt, 3 g/l; Fructose, 2 g/l) 16 Stunden bei 30 °C angezogen. Aus den abzentrifugierten Zellen wird das Plasmid pJP4 nach dem Verfahren aus J. Bacteriol. 135:227 (1978) isoliert. Das Plasmid pVK101 [V.C.Knauf et al., Plasmid 8:45 (1982)] wird aus E. coli HB101 nach dem allgemein bekannten Verfahren der alkalischen Lyse von T. Maniatis et al. (Molecular cloning: a laboratory manual. Cold Spring Harbour Laboratory, Cold Spring Harbour, New York, 1982) isoliert.

b) Klonierung des 21 Kilobasen HindIII-Fragments aus pJP4

Eine Reaktionsmischung, bestehend aus 20 μl (500 ng) pJP4, 2.5 μl TAS-Puffer (0.33 M Tris-acetat, 0.65 M Kaliumacetat, 0.1 M Magnesiumacetat, 5 mM Dithiothreitol (DTT), 30 mM Spermidin, pH 7.9) und 2.5 μl (1 Unit) einer verdünnten HindIII-Lösung, werden 120 min bei 37 °C inkubiert. Eine weitere Reaktionsmischung, bestehend aus 50 μl (14 μg) pVK101, 5.5 μl TAS-Puffer und 1 μl (20 Units) unverdünnter HindIII-Lösung, werden 90 min bei 37 °C inkubiert, danach wird 1 μl (30 Units) Calf Intestinal Alcaline Phospnatase (DIP, Boehringer Mannheim) zugesetzt und weitere 60 min inkubiert. 9 μl von jeder der beiden Reaktionsmischungen werden auf einem 0.7%igen Low-Melting-Agarose-Gel elektrophoretisch aufgetrennt. Das Gel wird anschließend 15 min in einer Ethidiumbromidlösung (5 μg/ml) gefärbt und die DNA-Banden im UV 300nm sichtbar gemacht. Die einzelne 20 kb Bande der pVK101-Restriktion sowie die zweitgrößte Bande (21 kb) der pJP4-Restriktion werden aus dem Gel ausgeschnitten, beide Banden werden vereinigt, und die DNA wird aus der Agarose nach dem Verfahren von T. Maniatis et al. (Molecular cloning: a laboratory manual. Cold Spring Harbour Laboratory, Cold Spring Harbour, New York, 1982) isoliert. Die ethanolgefällte DNA wird in 10 μl Ligationspuffer (66 mM Tris-HCl, 6.6 mM MgCl$_2$, 10 mM DTT, 1 mM ATP, pH 7.5) aufgenommen, mit 1 μl (0.1 Unit) verdünnter T4-DNA-Ligase versetzt und 16 Stunden bei 14 °C inkubiert (=Ligationsansatz). Kompetente Zellen von E. coli S17-1 [Biotechnology 1:784 (1983)] werden nach dem Verfahren von T.J. Silhavy et al. (Experiments with gene fusions. Cold Spring Harbour Laboratory, Cold Spring Harbour, New York, 1984) hergestellt 0.2 ml der so gewonnenen kompetenten Zellen werden mit 5 $\mu$l Ligationsansatz gemischt, 40 min auf Eis belassen, dann 3 min auf 42 °C erhitzt (=Transformation), anschließend mit 2 ml LB-Medium nach T. Maniatis et al. (vgl. oben) verdünnt und 60 min bei 30 °C inkubiert (= phänotypische Expression). Danach werden Portionen von je 0.2 ml auf LB-Agar-Platten, enthaltend 10 $\mu$l/ml Tetracyclin, ausgestrichen. Die Platten werden 16 Stunden bei 37 °C bebrütet. Es zeigen sich mehrere Tetracyclin-resistente Kolonien.

c) Identifizierung von pVJH2

Aus den so gewonnenen Tetracyclin-resistenten Kolonien wird nach dem Verfahren der alkalischen Lyse eine Schnellpräparation der Plasmid-DNA durchgeführt, wie sie von T. Maniatis et al. beschrieben wird. Durch das oben beschriebene Verfahren der Behandlung mit alkalischer Phosphatase wird gewährleistet, daß jeder der Tetracyclin-resistenten Klone das gewünschte Insert enthält. Durch Restriktionsenzymverdauung mit EcoRI und anschließender elektrophoretischer Trennung der DNA-Fragmente in einem 0.7%igen Agarosegel nach T. Maniatis et al. (vgl. oben) werden die rekombinanten Plasmide identifiziert. Durch Vergleich der Größen der erhaltenen EcoRI-Fragmente mit den Größenverhältnissen, die bezüglich EcoRI und HindIII für pJP4 und pVK101 aus der Literatur bekannt sind [R.H. Don, J. Bacteriol. 161:466 (1985) und V.C.Knauf et al. Plasmid 8:45 (1982)], gelingt eine genaue Identifizierung der Insert-DNA sowie seiner Orientierung im Vektorplasmid pVK101.

d) Restriktionskarte des HindIII-Inserts in pVJH21

Ein so gewonnener Stamm, der das Plasmid pVJH21 enthält, wird in 400 ml LB-Medium, das 20 $\mu$g/ml Tetracyclin enthält, 16 Stunden bei 37 °C angezogen. Aus den abzentrifugierten Zellen wird die Plasmid-DNA nach der Methode der alkalischen Lyse nach T. Maniatis et al. (vgl. oben) isoliert. Die isolierte Plasmid-DNA von pVJH21 wird in verschiedenen Ansätzen mit den Enzymen EcoRI, BamHI und SacI einzeln, sowie mit folgenden 9 Kombinationen mehrerer Enzyme verdaut: EcoRI/BamHI (1), EcoRI/SacI (2), BamHI/SacI (3), HindIII/EcoRI (4), HindIII/BamHI (5), HindIII/SacI (6), HindIII/EcoRI/BamHI (7), HindIII/EcoRI/SacI (8) und HindIII/BamHI/SacI (9). Die DNA-Fragmente werden in einem 0,7%igen Agarose-gel nach T. Mahiatis et al. (vgl. oben) aufgetrennt und ihre Größen werden durch Vergleich mit HindIII-geschnittener lambda-DNA ermittelt. Mit den daraus gewonnen Daten wird zusammen mit den für die Plasmide pJP4 [R.H. Don, J. Bacteriol. 161:466 (1985)] und pVK101 [V.C.Knauf et al. Plasmid 8:45 (1982)] bezüglich EcoRI, BamHI und HindIII bekannten Größenverhältnissen die Restriktionskarte erstellt.

Beispiel 2: Herstellung des Plasmids pGJS3

a) Isolierung der Plasmide pVJH21 und pGSS33

Der rekombinante Stamm E. coli S17-1, der das in Beispiel 1 hergestellte Plasmid pVJH21 enthält, und E. coli JA221 mit dem Vektorplasmid pGSS33 [G.S. Sharpe Gene 29:93 (1984)] werden in je 300 ml LB-Medium, das 20 $\mu$g/ml Tetracyclin enthält, 16 Stunden bei 37 °C angezogen. Aus den abzentrifugierten Zellen werden die beiden Plasmide nach der Methode der alkalischen Lyse nach T. Maniatis et al. (vgl. oben) isoliert.

b) Klonierung von SacI-Fragmenten aus pVJH21

Eine Reaktionsmischung, bestehend aus 10 $\mu$l (5 $\mu$g) pVJH21, 10 $\mu$l (1 $\mu$g) pGSS33, 4 $\mu$l TAS-Puffer, 16 $\mu$l Wasser und 0.5 $\mu$l (10 Units) SacI, wird 90 min bei 37 °C inkubiert. Das Restriktionsenzym wird anschließend durch 15 minütiges Erhitzen auf 68 °C inaktiviert. Die DNA wird mit Ethanol gefällt nach T. Maniatis et al. (vgl. oben) und der getrocknete Niederschlag in 40 $\mu$l Ligationspuffer aufgenommen. Nach Zusatz von 1 $\mu$l (1 Unit) T4-DNA-Ligase wird der Ligationsansatz 16 Stunden bei 14 °C inkubiert. E. coli LE392 [N.E. Murray et al. Mol. Gen. Genet. 150:53 (1977)] wird nach dem Verfahren von T.J. Silhavy et al. (Experiments with gene fusions. Cold Spring Harbour Laboratory. Cold Spring Harbour, New York, 1984) für die DNA-Aufnahme kompetent gemacht, 0.2 ml kompetente Zellen werden mit 5 $\mu$l Ligationsansatz gemischt, 40 min auf Eis belassen und durch 3minütiges Erhitzen auf 42 °C transformiert. Nach Verdünnen mit 2 ml LB-Medium und Inkubation bei 30 °C werden Portionen von 50 $\mu$l bis 200 $\mu$l auf LB-Agar-Platten, die 20 $\mu$g/ml Chloramphenicol enthalten, ausgestrichen. Die Platten werden 16 Stunden bei 37 °C inkubiert.

c) Identifizierung rekombinanter Plasmide

Die so erhaltenen Chloramphenicol-resistenten Kone werden parallel auf zwei LB-Agar-Platten ausgestrichen, wovon eine 25 $\mu$g/ml Streptomycin, die andere 20 $\mu$g/ml Chloramphenicol enthält. Aus Klonen, die sich bei diesem Test als Streptomycin-sensitiv erweisen, wird die Plasmid-DNA mit Hilfe des Schnellverfahrens der alkalischen Lyse nach T. Maniatis et al. (vgl. oben) isoliert. Durch Restriktion der isolierten Plasmide mit SacI, elektrophoretischer Auftrennung der DNA-Fragmente auf einem 0.7%igen Agarosegel und Vergleich der im UV 300 nm sichtbaren Banden mit bekannten Fragmenten einer HindIII-Restriktion der lambda-Phagen-DNA wird die Größe der im Vektor pVK101 enthaltenen Inserts ermittelt. Bei dem Plasmid pGJS3 handelt es sich um ein rekombinantes Plasmid aus pGSS33 und einem 3 Kilobasen großen DNA-Fragment. Da das Plasmid pVK101 selbst keine Schnittstelle für SacI besitzt, stammt das klonierte DNA-Fragment eindeutig aus dem 21 Kilobasen großen HindIII-Fragment von PJP4.

Beispiel 3: Herstellung der Plasmide-pKJS31 und pKJS32

a) Isolierung der Plasmide PGJS3 und pKT231

Der in Beispiel 2 hergestellte Stamm E. coli LE392, der das rekombinante Plasmid pGJS3 enthält, wird in 400 ml LB-Medium mit einem Zusatz von 20 $\mu$g/ml Chloramphenicol 16 Stunden bei 37 °C angezogen. E. coli SK1592 mit dem Vektorplasmid pKT231 [M. Bagdasarian et al., Current Topics in Microbiology and Immmnology 96:47 (1982)] wird in 400 ml LB-Medium mit einem Zusatz von 50 $\mu$g/ml Kanamycin unter den gleichen Bedingungen angezogen. Aus den abzentrifugierten Zellen werden die beiden Plasmide nach dem Verfahren der alkalischen Lyse nach T. Maniatis et al. (vgl. oben) isoliert.

b) Umklonierung des SacI-Fragmentes aus pGJS3 in pKT231

Eine Reaktionsmischung, bestehend aus 40 $\mu$l (400 ng) pGJS3, 4 $\mu$l (100 ng) pKT231, 6 $\mu$l TAS-Puffer, 9 $\mu$l Wasser und 1 $\mu$l (1 Unit) verdünnte SacI-Lösung, werden 120 min bei 37 °C inkubiert. Durch anschließendes Erhitzen der Reaktionsmischung auf 68 °C für 15 min werden die Restriktionsenzyme inaktiviert. Zu dem Ansatz werden 39 $\mu$l Wasser, 10 $\mu$l 10-fach konzentrierter Ligationspuffer und 1 $\mu$l (0.1 Unit) einer verdünnten T4-DNA-Ligase hinzugefügt und der Ligationsansatz 16 Stunden bei 14 °C inkkbiert. 0.2 ml kompetente Zellen von E. coli LE392, hergestellt nach dem Verfahren von T.J. Silhavy et al. (Experiments with gene fusions. Cold Spring Harbour Laboratory, Cold Spring Harbour, New York, 1984), werden mit 10 $\mu$l des Ligationsansatzes gemischt, 40 min auf Eis belassen, 3 min auf 42 °C erhitzt, mit 2 ml LB-Medium gemischt und 60 min bei 37 °C inkubiert. Portionen von 50 $\mu$l bis 200 $\mu$l werden auf LB-Agar-Platten ausgestrichen, die 50 $\mu$g/ml Kanamycin enthalten. Die Platten werden 16 Stunden bei 37 °C inkubiert.

c) Identifizierung der rekombinanten Plasmide

Die so erhaltenen Kanamcyin-resistenten Klone werden parallel auf drei verschiedenen LB-Agar-Platten ausgestrichen, wovon eine 25 $\mu$g/ml Streptomycin, die zweite 20 $\mu$g/ml Chloramphenicol und die dritte 50 $\mu$g/ml Kanamycin enthält. Klone, die sich in diesem Test als Streptomycin- und Chloramphenicol-sensitiv, aber als Kanamycin-resistent erweisen, enthalten im Regelfalle ein rekombinantes Plasmid aus dem Vektor pKT231 (Kanamycinresistenz) und dem 3 Kilobasen großen SacI-Fragment aus pGJS3. Durch Isolierung der Plasmid-DNA nach dem bekannten Schnellverfahren nach T. Maniatis et al. (vgl. oben), Restriktion der DNA mit SacI und anschließender Analyse der Fragmente durch Elektrophorese in einem 0.7%igen Agarosegel wird die Größe der inserierten DNA bestimmt. Durch eine in gleicher Weise ausgeführte Restriktionsanalyse mit EcoRI werden Plasmide mit unterschiedlicher Orientierung des Inserts in Relation zum Vektorplasmid identifiziert. Bei dem Plasmid pKJS32 handelt es sich um diejenige Form der beiden Möglichkeiten, bei der die EcoRI-Stelle des Inserts am nahesten bei der EcoRI-Stelle des Vektoranteils liegt. Dieses Plasmid ergibt zwei EcoRI-Fragmente der Größen 0.9 und 15 Kilobasen. Das Plasmid pKJS31 stellt die Konstruktion mit der entgegengesetzten Orientierung des Inserts dar, es liefert zwei EcoRI-Fragmente der Größen 2.3 und 13.5 Kilobasen.

Beispiel 4: Herstellung des Plasmids pKJSB330

a) Isolierung des Plasmids pKJS31

Der in Beispiel 3 erhaltene Klon von E. coli LE392, der das neukombinierte Plasmid pKJS31 enthält, wird in 400 ml LB-Medium mit einem Zusatz von 50 μg/ml Kanamycin 16 Stunden bei 37 °C angezogen und aus den abzentrifugierten Zellen wird die Plasmid-DNA mittels alkalischer Lyse nach T. Maniatis et al. (vgl. oben) isoliert.

b) Deletion des kleineren BamHI-Fragments aus pKJS31

Eine Reaktionsmischung, bestehend aus 10 μl (500 ng) pKJS31, 7 μl Wasser, 2 μl TAS-Puffer ind 1 μl (1 Unit) einer verdünnten BamHI-Lösung, wird 60 min bei 37 °C inkubiert und anschließend die enzymatische Reaktion durch Erhitzen auf 68 °C für 15 min gestoppt. Der Restriktionsansatz wird dann mit 50 μl Wasser, 8 μl 10-fach konzentriertem Ligationspuffer und 2 μl (2Units) T4-DNA-Ligase versetzt und 16 Stunden bei 14 °C inkubiert. 0.2 ml kompetente Zellen von E. coli S17-1, hergestellt nach dem bekannten Verfahren von T.J. Silhavy et al. (Experiments with gene fusions. Cold Spring Harbour Laboratory, Cold Spring Harbour, New York, 1984), werden mit 10 μl des Ligationsansatzes gemischt, 40 min auf Eis belassen, 3 min auf 42 °C erhitzt, mit 2 ml LB-Medium gemischt und 60 min bei 37 °C inkubiert. Portionen von 50 μl bis 200 μl werden auf LB-Agar-Platten ausgestrichen, die 50 μg/ml Kanamycin enthalten. Die Platten werden 16 Stunden bei 37 °C inkubiert.

c) Identifizierung der verkleinerten Plasmide

Aus den so erhaltenen Kanamycin-resistenten Klonen wird die Plasmid-DNA mittels des bekannten Schnellverfahrens der alkalischen Lyse nach T. Maniatis et al. (vgl. oben) isoliert. Durch Restriktion der Plasmide mit BglII und elektrophoretischer Auftrennung der DNA-Fragmente in einem 0.7%igen Agarosegel werden Plasmide identifiziert, die das kleine BaaHI-Fragment aus pKJS31 verloren haben. Diese Plasmide besitzen nur eine einzige BglII-Schnittstelle und liefern daher ein BglII-Fragment von 13.2 Kilobasen Größe. Sie lassen sich damit eindeutig von ihrem Ursprungsplasmid pKJS31 unterscheiden und werden als pKJSB330 bezeichnet.

Beispiel 5: Herstellung des Plasmids pKJS32RHΔS'

a) Isolierung der Plasmide pKJS32 und pRME1

Der in Beispiel 3 erhaltene Klon von E. coli LE392, der das neukombiniete Plasmid pKJS32 enthält, sowie E. coli SBC107 (pRME1) werden in je 400 ml LB-Medium mit einem Zusatz von 50 μg/ml Kanamycin 16 Stunden bei 37 °C angezogen. Aus den abzentrifugierten Zellen werden die Plasmide mittels alkalischer Lyse nach T. Maniatis et al. (vgl. oben) isoliert.

b) Deletion eines HindIII/SalI-Fragmentes aus pKJS32 und anschließende Insertion eines HindIII/SalI-Fragmentes aus pRME1

Eine Reaktionsmischung, bestehend aus 10 μl (500 ng) pKJS32, 10 μl (1 μg) pRME1, 15 μl Wasser, 4 μl TAS-Puffer, 0.5 μl (1 Unit) verdünnter HindIII-Lösung und 0.5 μl (1 Unit) verdünnter SalI-Lösung, werden 120 min bei 37 °C inkubiert und die enzymatische Reaktion durch Erhitzen auf 68 °C für 15 min gestoppt. Anschließend werden 10 μl des Restriktionsansatzes mit 25 μl Wasser, 4 μl 10-fach konzentriertem Ligationspuffer und 1 μl (1 Unit) T4-DNA-Ligase versetzt und 16 Stunden bei 14 °C inkubiert. Nach einem bekannten Verfahren T.J. Silhavy et al. (Experiments with gene fusions. Cold Spring Harbour Laboratory, Cold Spring Harbour, New York, 1984), kompetent gemachte Zellen von E. coli S17-1 werden mit 10 μl des Ligationsansatzes gemischt, 40 min auf Eis belassen, 3 min auf 42 °C erhitzt und nach Vermischen mit 2 ml LB-Medium 60 min bei LB-Agar-Platten ausgestrichen, die 50 μg/ml Kanamycin enthalten.

Die Platten werden 16 Stunden bei 37 °C inkubiert.

c) Identifizierung der rekombinanten Plasmide

Aus den so erhaltenen Kanamycin-resistenten Klonen wird die Plasmid-DNA mittels des bekannten Schnellverfahrens der alkalischen Lyse nach T. Maniatis et al. (vgl. oben) isoliert. Durch Restriktion mit BamHI und elektrophoretischer Auftrennung der erhaltenen DNA-Fragmente werden Plasmide identifiziert, bei denen ausgehend von pKJS32 der kleinere DNA-Abschnitt zwischen der auf dem Vektoranteil inmitten des Kanamycin-Resistenzgens gelegenen HindIII-Schnittstelle und der auf dem Insert gelegenen SalI-Schnittstelle entfernt worden ist und durch dasjenige HindIII/SalI-Fragment aus pRME1 ersetzt worden ist, welches das 3'-Ende der kodierenden Region des Kanamycin-Resistenzgens trägt. Diese Plasmide liefern zwei BanHI-Fragmente mit den Längen 13.3 und 2.0 Kilobasen und unterscheiden sich damit eindeutig von den Ausgangsprodukten und anderen möglichen Nebenprodukten der Ligation. Sie stellen Konstruktionen dar, bei denen ein 0,2 Kilobasen großer DNA-Abschnitt des 3.0 Kilobasen großen Inserts von pKJS32 entfernt wurde und bei denen ein Teil des Kanamycinresistenzgens gegen ein anderes Genfragment des gleichen genetischen Ursprungs ausgetauscht wurde. Diese Plasmide werden als pKJS32RHΔS' bezeichnet.

Beispiel 6: Herstellung des Plasmids pKJEΔB130

a) Isolierung der Plasmide pKT231 und pKJS32

Die Isolierung von pKT231 wurde in Beispiel 3, die von pKJS32 in Beispiel 5 beschrieben.

b) Klonierung des kleineren EcoRI/BamHI-Fragments aus pKJS32 in pKT231

Zwei Reaktionsmischungen, die eine bestehend aus 10 μl (500 ng) pKJS32, 5 μl Wasser, 2 μl TAS-Puffer und je 1 μl (1 Unit) von verdünnten Lösungen der Enzyme EcoRI, BamHI und PstI, die andere bestehend aus 10 μl (250 ng) pKT231, 6 μl Wasser, 2 μl TAS-Puffer und je 1 μl (1 Unit) von verdünnten Lösungen der Enzyme EcoRI und BamHI, werden 120 min bei 37 °C inkubiert und zum Abstoppen der Reaktion für 15 min auf 68 °C erhitzt. Anschließend werden je 10 μl der beiden Restriktionsansätze gemischt, mit 50 μl Wasser, 8 μl 10-fach konzentriertem Ligationspuffer und 2 μl (2 Units) T4-DNA-Ligase versetzt und 16 Stunden bei 14 °C inkubiert. 0.2 ml kompetente Zellen von E. coli LE392, hergestellt nach dem literaturbekannten Verfahren von T.J. Silhavy et al. (Experiments with gene fusions. Cold Spring Harbour Laboratory, Cold Spring Harbour, New York, 1984), werden mit 10 μl des Ligationsansatzes gemischt, 40 min auf Eis belassen, 3 min auf 42 °C erhitzt, mit 2 ml LB-Medium gemischt und 60 min bei 37 °C inkubiert. Portionen von 50 μl bis 200 μl werden auf LB-Agar-Platten ausgestrichen, die 50 μg/ml Kanamycin enthalten. Die Platten werden 16 Stunden bei 37 °C inkubiert.

c) Identifizierung rekombinanter Plasmide

Die so erhaltenen Kanamycin-resistenten Klone werden parallel auf zwei LB-Agar-Platten ausgestrichen, wovon eine 25 μg/ml Streptomycin, die andere 50 μg/ml Kanamycin enthält. Aus Klonen, die sich in diesem Test als Streptomycin-resistent erweisen, wird die Plasmid-DNA nach dem Schnellverfahren nach T. Maniatis et al. (vgl. oben) isoliert. Restriktion der DNA mit XhoI und anschließende elektrophoretische Auftrennung der DNA-Bruchstücke in einem 0.7%igen Agarosegel erlaubt die Identifizierung von neukombinierten Plasmiden, die zwei XhoI-Fragmente mit den Größen 3.0 und 9.7 Kilobasen ergeben. Werden die gleichen Plasmide gleichzeitig mit EcoRI und BamHI geschnitten, so ergibt die elektrophoretische Analyse zwei Bruchstücke der Größen 1.5 und 11.2 Kilobasen. Bei den so identifizierten Plasmiden handelt es sich um Rekombinanten aus dem großen EcoRI/BamHI-Fragment von pKT231 und dem 1.5 Kilobasen großen EcoRI/BamHI-Fragment, das aus der Insert-DNA des Plasmids pKJS32 stammt. Sie werden als pKJEΔB130 bezeichnet.

Beispiel 7: Herstellung des Plasmids pKJS(X)630

a) Isolierung des Plasmids pKJSB330

Der in Beispiel 4 erhaltene Klon von E. coli S17-1, der das neukombinierte Plasmid pKJSB330 enthält, wird in 400 ml LB-Medium mit einem Zusatz von 50 μg/ml Kanamycin 16 Stunden bei 37 °C angezogen und aus den abzentrifugierten Zellen wird die Plasmid-DNA mittels alkalischer Lyse nach T. Maniatis et al. (vgl. oben) isoliert.

b) Entfernen eines BamHI/XbaI-Fragments aus dem Plasmid pKJSB330

Eine Reaktionsmischung, bestehend aus 20 μl (1 μg) pKJSB330, 15 μl Wasser, 4 μl TAS-Puffer, 0.5 μl (2 Units) verdünnter XbaI-Lösung und 0.5 μl (2 Units) verdünnter BamHI-Lösung, wird 120 min bei 37 °C inkubiert. Anschließend werden 20 μl gepuffertes Phanol nach T. Maniatis et al. (vgl. oben) zugesetzt, gemischt, nach Zusatz von 20 μl Chloroform: Isoamylalkohol (24:1) wird nochmals gemischt und abzentrifugiert. Die obere wäßrige Phase wird abgenommen und mit 120 μl -20 °C kaltem Ethanol gemischt. Die Mischung wird 30 min bei -20 °C aufbewahrt und dann abzentrifugiert. Die gefällte DNA wird mit kaltem 70%igem Ethanol gewaschen, im Vakuum getrocknet und in 20 μl Klenow-Reaktionslösung (20 mM Tris-HCl, pH 8.0, 7 mM $MgCl_2$, 10 U/ml DNA-Polymerase I (= Klenowenzym)) aufgenommen. Nach 5 minütiger Vorinkubation bei 37 °C werden 2 μl einer Lösung aller 4 Desoxynukleotidtriphosphate (0.125 mM ATP, 0.125 mM CTP, 0.125 mM GTP, 0.125 mM TTP, Pharmacia) hinzugefügt und weitere 5 min inkubiert. Danach wird der Ansatz mit 80 μl Ligationspuffer, der 25 U/ml T4-DNA-Ligase enthält, gemischt und 6 Stunden bei Raumtemperatur belassen. Anschließend wird 16 Stunden bei 14 °C inkubiert. 0.2 ml einer Suspension kompetenter Zellen von E. coli S17-1, hergestellt nach dem Verfahren von T.J. Silhavy et al. (Experiments with gene fusions. Cold Spring Harbour Laboratory. Cold Spring Harbour, New York, 1984), werden mit 10 μl des Ligationsansatzes gemischt, nach 40 min Inkubation auf Eis durch Hitzebehandlung (3 min, 42 °C) transformiert, mit 2 ml LB-Medium gemischt und 60 min bei 37 °C inkubiert. Von der Zellsuspension werden dann Portionen von 50 bis 200 μl auf LB-Agar-Platten ausgestrichen, die 50 μg/ml Kanamycin enthalten. Die Platten werden 16 Stunden bei 37 °C inkubiert.

c) Identifizierung der verkleinerten Plasmide

Aus den so erhaltenen Kanamycin-resistenten Klonen wird die Plasmid-DNA mittels des bekannten Schnellverfahrens der alkalischen Lyse nach T. Maniatis et al. (vgl. oben), isoliert. Durch Restriktion der Plasmide mit SmaI und anschließender elektrophoretischer Trennung der DNA-Bruchstücke in einem 0.7 %igen Agarosegel werden Plasmide identifiziert, die den DNA-Abschnitt zwischen der BamHI-Schittstelle und der XbaI-Schnittstelle auf dem Insert von pKJSB330 verloren haben. Diese Plasmide liefern in der Restriktionsanalyse zwei SmaI-Fragmente mit einer Länge von 2.6 und 9.9 Kilobasen und werden als pKJS-(X)630 bezeichnet. Sie sind damit eindeutig von dem Ausgangsproddkt pKJSB330 zu unterscheiden.

Beispiel 8: Herstellung der Plasmide pTJSS'035 und pTJSS'036

a) Isolierung der Plasmide pT7-5, pT7-6 und pKJS32

Zwei Stämme von E. coli HMS174, von denen einer das Plasmid pT7-5, der andere das Plasmid pT7-6 enthält, werden in je 400 ml LB-Medium nach T. Maniatis et al. (vgl. oben) mit einem Zusatz von 50 μg/ml Ampicillin 16 Stunden bei 37 °C angezogen. Aus den abzentrifugierten Zellen wird die Plasmid-DNA durch das bekannte Verfahren der alkalischen Lyse nach T. Maniatis isoliert. Die Isolierung von pKJS32 wird in Beispiel 5 beschrieben.

b) Klonierung des SacI/Sal I-Fragments aus pKJS32 in pT7-5 und pT7-6

Ein Reaktionsgemisch (A), bestehend aus 20 μl (1 μg) pKJS32, 15 μl Wasser, 4 μl TAS-Puffer und je 0.5 μl (2 Units) der Enzyme SacI und Sal I, werden 1 20 min bei 37 °C inkubiert. Zwei weitere Reaktionsgemische, bestehend aus entweder 2 μl (1 μg) pT7-5 (B) oder 2 μl (1 μg) pT7-6 (C), 15 μl Wasser, 2 μl TAS-Puffer und je 0.5 μl (2 Units) der Enzyme SacI und SalI, werden unter den gleichen Bedingungen inkubiert. Alle drei Reaktionen werden durch Erhitzen auf 68 °C für 15 min abgestoppt. Je 13 μl des Restriktionsansatzes A (pKJS32) werden mit 7 μl des Restriktionsansatzes B (pT7-5) bzw. 7 μl des

Restriktionsansatzes C (pT7-6) gemischt. Zu den beiden Mischungen werden je 50 $\mu$l Wasser, 8 $\mu$l 10-fach konzentrierter Ligationspuffer und 2 $\mu$l (2 Units) T4-DNA-Ligase gegeben. Beide Ligationsansätze werden 16 Stunden bei 14 °C inkubiert. Je 0.2 ml kompetente Zellen von E. Coli LE392, hergestellt nach dem Verfahren T.J. Silhavy et al. (Experiments with gene fusions. Cold Spring Harbour Labbratory, Cold Spring Harbour, New York, 1984), werden mit je 10 $\mu$l der Ligationsansätze gemischt, 40 min auf Eis belassen, 3 min auf 42 °C erhitzt, mit 2 ml LB-Medium gemischt und 60 min bei 37 °C inkubiert. Portionen von 50 $\mu$l bis 200 $\mu$l werden auf LB-Agar-Platten ausgestrichen, die 50 $\mu$g/ml Ampicillin enthalten. Die Platten werden 16 Stunden bei 37 °C inkubiert.

c) Identifizierung rekombinanter Plasmide

Aus den so erhaltenen Ampicillin-resistenten Klonen wird die Plasmid-DNA nach dem Bekannten Schnellverfahren der alkalischen Lyse nach T. Maniatis et al. (vgl. oben), isoliert. Durch Restriktion der Plasmide mit BglII und BamHI, sowie durch doppelte Restriktion mit BamHI und HindIII und anschließende elektrophoretische Auftrennung der Fragmente in einem 0.7%igen Agarosegel werden Plasmide identifiziert, die aus einem der beiden Vektorplasmide pT7-5 oder pT7-6 und dem kleinen SacI/SalI-Fragment von pKJS32 bestehen. Diese neukombinierten Plasmide liefern dabei im Falle von pT7-5 als Ausgangsprodukt zwei BglII-Fragmente der Größen 3.0 Kilobasen und 2.2 Kilobasen, ein BamHI-Fragment der Größe 5.2 Kilobasen und zwei BamHI/HindIII-Fragmente der Größen 3.2 und 2.0 Kilobasen und werden als pTJSS'035 bezeichnet. Im Falle von pT7-6 als Ausgangsprodukt ergeben sich zwei BglII-Fragmente der Größen 2.8 Kilobasen und 2.2 Kilobasen, ein BanHI-Fragment der Größe 5.0 Kilobasen und zwei BamHI/HindIII-Fragmente der Größen 3.0 Kilobasen und 2.0 Kilobasen; sie werden als pTJSS'036 bezeichnet. Die rekombinanten Plasmide unterscheiden sich damit eindeutig von ihren Ausgangsprodukten pKJS32 und pT7-5 bzw. pT7-6.

Beispiel 9: Herstellung der Plasmide pTJS'B435 und pTJS'B436

a) Isolierung der Plasmide pT7-5, pT7-6 und pKJSB330

Die Isolierung von pT7-5 und pT7-6 wird bereits in Beispiel 8 beschrieben, die Isolierung von pKJSB330 wird in Beispiel 7 beschrieben.

b) Klonierung des kleinen SalI/BamHI-Fragments aus pKJSB330 in pT7-5 und pT7-6

Eine Reaktionsmischung (A), beschehend aus 40 $\mu$l (2 $\mu$g) pKJSB330, 5 $\mu$l TAS-Puffer und je 2.5 $\mu$l (4 Units) von verdünnten Lösungen der Enzyme SalI und BamHI, wird 120 min bei 37 °C inkubiert. Zwei weitere Reaktionsmischungen, bestehend aus 2 $\mu$l (1 $\mu$g) pT7-5 (B) bzw. 2 $\mu$l (1 $\mu$g) pT7-6 (C), 20 $\mu$l Wasser, 3 $\mu$l TAS-Puffer und je 2.5 $\mu$l (4 Units) von verdünnten Lösungen der Enzyme Sal I und BamHI, werden unter den gleichen Bedingungen inkubiert. Die Reaktionen werden durch Erhitzen auf 68 °C für 15 min abgestoppt. Der gesamte Restriktionsansatz A (pKJSB330) wird in einem Agarosegel [0.7% Agarose in TBE-Puffer nach T. Maniatis et al. (vgl. oben), das 0.5 $\mu$g/ml Ethidiumbromid enthält, nach T. Maniatis et al. (vgl. oben)] elektrophoretisch aufgetrennt. Von den beiden unter UV-Licht (300 nm) sichtbaren DNA-Banden wird die kleinere, 2.0 Kilobasen große Bande durch das Verfahren der DEAE-Membranelution auf folgende Weise aus dem Gel isoliert: Ein geeignet dimensioniertes Stück einer DEAE-Membran (Schleicher und Schuell S&S NA-45) wird in einem Einschnitt 1 bis 2 mm unterhalb der zu eluierenden Bande plaziert. Die Elektrophorese wird fortgesetzt, bis die betreffende Bande ganz von der Membran absorbiert worden ist. Die Membran wird anschließend aus dem Gel herausgenommen und 10 min mit 10 ml NET-Puffer (150 mM NaCl, 0.1 mM EDTA, 20 mM Tris-HCl, pH 8.O) gewaschen. Die gewaschene Membran wird in 150 $\mu$l HNET-Puffer (1 M NaCl, 0.1 mM EDTA, 20 mM Tris-HCl, pH 8.0) 30 min bei 68 °C inkubiert. Die Lösung wird abgenommen und die Membran weitere 10 min mit 50 $\mu$l HNET-Puffer inkubiert. Die vereinigten Elutionslösungen (200 $\mu$l) werden mit 200 $\mu$l Wasser verdünnt, mit 800 $\mu$l kaltem (-20 °C) Ethanol gemischt und 30 min bei -20 °C aufbewahrt. Die gefällte DNA wird abzentrifugiert und der Niederschlag in 90 $\mu$l Wasser aufgenommen. Nach Zusatz von 10 $\mu$l 3 M Natriumacetatlösung nach T. Maniatis et al. (vgl. oben) wird mit 200 $\mu$l kaltem Ethanol erneut gefällt (30 min, -20 °C), abzentrifugiert, der Niederschlag mit kaltem 70%igen Ethanol gewaschen, im Vakuum getrocknet und in 80 $\mu$l Ligationspuffer aufgenommen. Davon werden je 40 $\mu$l mit 3 $\mu$l (100 ng) des Restriktionsansatzes B (pT7-5) bzw. 3 $\mu$l (100 ng) des Restriktionsansatzes C (pT7-6) und 2 $\mu$l (1 Unit) T4-DNA-Ligase versetzt und 16 Stunden bei 14 °C inkubiert. 0.2 ml kompetente Zellen von E. coli LE392, hergestellt nach dem Verfahren von T.J. Silhavy et al. (Experiments with gene fusions. Cold Spring Harbour Laboratory. Cold Spring Harbour, New York, 1984), werden mit 15 µl des Ligationsansatzes gemischt, 40 min auf Eis belassen, 3 min auf 42 °C erhitzt, mit 2 ml LB-Medium gemischt und 60 min bei 37 °C inkubiert. Portionen von 50 µl bis 200 µl werden auf LB-Agar-Platten ausgestrichen, die 50 µg/ml Ampicillin enthalten. Die Platten werden 16 Stunden bei 37 °C inkubiert.

c) Identifizierung rekombinanter Plasmide

Aus den so erhaltenen Ampicillin-resistenen Klonen wird die DNA nach dem bekannten Schnellverfahren der alkalischen Lyse nach T. Maniatis et al. (vgl. oben) isoliert. Durch Restriktion der Plasmide mit EcoRI und anschließender elektrophoretischer Auftrennung der Fragmente in einem 0.7%igen Agarosegel werden Plasmide identifiziert, die das kleine SalI/BamHI-Fragment aus pKJSB330 in einem der beiden Vektorplasmide enthalten. Rekombinante Plasmide mit pT7-5 als Ausgangsprodukt ergeben zwei EcoRI-Fragmente der Größen 3.0 Kilobasen und 1.5 Kilobasen und werden als pTJS'B435 bezeichnet, solche mit pT7-6 als Ausgangsprodukt liefern zwei EcoRI-Fragmente der Größen 2.8 Kilobasen und 1.5 Kilobasen und werden mit pTJS'B436 benannt.

Beispiel 10: Herstellung der Plasmide pTJS'X535 und pTJS'X536

a) Isolierung der Plasmide pT7-5, pT7-6 und pTJSS'035

E. coli LE392, der das in Beispiel 8 hergestellte Plasmid pTJSS'035 enthält, wird in 400 ml LB-Medium mit einem Zusatz von 50 µg/ml Ampicillin 16 Stunden bei 37 °C angezogen. Aus den abzentrifugierten Zellen wird die Plasmid-DNA nach T. Maniatis et al. (vgl. oben) isoliert. Die Isolierung von pT7-5 und pT7-6 wird in Beispiel 8 beschrieben.

b) Klonierung des kleinen SalI/XbaI-Fragments aus pTJSS'035 in pT7-5 und pT7-6

Ein Reaktionsgemisch (A), bestehend aus 6 µl (3 µg) pTJSS'035, 15 µl Wasser, 3 µl TAS-Puffer und je 2 µl (5 Units) von verdünnten Lösungen der Enzyme XbaI, SalI und BamHI, wird 120 min bei 37 °C inkubiert. Zwei weitere Reaktionsgemische, bestehend aus entweder 2 µl (1 µg) pT7-5 (B) oder 2 µl (1 µg) pT7-6 (C), 23 µl Wasser, 3 µl TAS-Puffer und je 1 µl (2.5 Units) von verdünnten Lösungen der Enzyme XbaI und SalI, werden in gleicher Weise inkubiert. Die Reaktionen werden durch Erhitzen auf 68 °C für 15 min gestoppt. In zwei verschiedenen Ligationsansätzen werden je 5 µl (500 ng) des Restriktionsansatzes A (pTJSS'035) mit entweder 15 µl des Restriktionsansatzes B (pT7-5) oder 15 µl des Restriktionsansatzes C (pT7-6), 50 µl Wasser, 8 µl Ligatlonspuffer und 2 µl (2 Units) T4-DNA-Ligase gemischt und 16 Stunden bei 14 °C inkubiert. 0.2 ml kompetente Zellen von E. coli LE392, hergestellt nach dem Verfahren von T.J. Silhavy et al. (Experiments with gene fusions. Cold Spring Harbour Laboratory. Cold Spring Harbour, New York, 1984), werden mit 15 µl des Ligationsansatzes gemischt, 40 min auf Eis belassen, 3 min auf 42 °C erhitzt, mit 2 ml LB-Medium gemischt und 60 min bei 37 °C inkubiert. Portionen von 50 µl bis 200 µl werden auf LB-Agar-Platten ausgestrichen, die 50 µg/ml Ampicillin enthalten.Die Platten werden 16 Stunden bei 37 °C inkubiert.

c) Identifizierung rekombinanter Plasmide

Aus den so erhaltenen Ampicillin-resistenten Klonen wird die Plasmid-DNA nach T. Maniatis et al. (vgl. oben) isoliert. Durch Restriktion mit den Enzymen SmaI und EcoRI, sowie durch doppelte Restriktion mit EcoRI und SalI werden Plasmide identifiziert, die das kleine, 1.4 Kilobasen große SalI/XbaI-Fragment aus pTJSS'035 in einem der beiden Vektorplasmide pT7-5 bzw. pT7-6 enthalten. Rekombinante Plasmide mit pT7-5 als Ausgangsprodukt ergeben zwei EcoRI-Fragmente der Größen 3.0 und 0.8 Kilobasen, zwei SmaI-Fragmente der Größen 2,4 und 1.4 Kilobasen und drei EcoRI/SalI-Fragmente der Größen 2.4, 0.8 und 0.6 Kilobasen; sie werden als pTJS'X535 bezeichnet. Rekombinante Plasmide mit pT7-6 als Ausgangsprodukt liefern zwei EcoRI-Fragmente der Größen 2.8 und 0.8 Kilobasen, zwei SmaI-Fragmente der Größen 2.2 und 1.4 Kilobasen und drei EcoRI/SalI-Fragmente der Größen 2.2, 0.8 und 0.6 Kilobasen; sie werden mit pTJS'X536 benannt. Die rekombinanten Plasmide unterscheiden sich damit eindeutig von ihren Ausgangsprodukten.

16

Beispiel 11: Herstellung des Plasmids pTJS'X535omega

a) Isolierung der Plasmide pTJS'X535 und pDOC37

Zwei E. coli Stämme LE392, wovon einer das in Beispiel 10 hergestellte Plasmid pTJS'X535, der andere das Plasmid pDOC37 enthält, werden in je 400 ml LB-Medium mit einem Zusatz von 50 µg/ml Ampicillin 16 Stunden bei 37 °C angezogen. Aus den abzentrifugierten Zellen wird die Plasmid-DNA nach T. Maniatis et al. (vgl. oben) isoliert. Das Plasmid pDOG37 enthält das Omega-Fragment zwischen zwei EcoRI-Restriktionsstellen. Anstelle von pDOC37 kann in gleicher Weise das von Prentki und Krisch, 1984, in Gene 29:103, beschriebene Plasmid pHP45omega als Quelle für das Omega-Fragment dienen.

b) Klonierung des Omega-Fragments aus pDOC37 in die Bgl II-Stelle von pTJS'X535

Zwei Reaktionsmischungen, davon eine bestehend aus 2 µl (1 µg) pTJS'X535, 14 µl Wasser, 2 µl TAS-Puffer und 2 µl (3 Units) Bgl II, die andere bestehend aus 2 µl (2 µg) pDOC37, 14 µl Wasser, 2 µl TAS-Puffer und 2 µl (4 Units) einer verdünnten BamHI-Lösung, werden 120 min bei 37 °C inkubiert. Die Reaktionen werden durch Erhitzen auf 68 °C für 15 min gestoppt. Je 10 µl aus beiden Restriktionsansätzen werden vereinigt und mit 80 µl Ligationspuffer und 1 µl (1 Unit) T4-DNA-Ligase versetzt. Der Ligationsansatz wird 16 Stunden bei 14 °C inkubiert. 0.2 ml kompetente Zellen von E. coli LE 392, hergestellt nach T.J. Silhavy et al. (Experiments with gene fusions. Cold Spring Harbour Laboratory. Cold Spring Harbour, New York, 1984), werden mit 10 µl des Ligationsansatzes gemischt, 40 min auf Eis belassen, 3 min auf 42 °C erhitzt, mit 2 ml LB-Medium gemischt und 60 min bei 37 °C inkubiert. Portionen von 50 µl bis 200 µl werden auf LB-Agar-Platten ausgestrichen, die 50 µg/ml Ampicillin und 80 µg/ml Spectinomycin enthalten. Die Platten werden 16 Stunden bei 37 °C inkubiert. Durch Selektion mittels Spectinomycin wird sichergestellt, daß alle wachsenden Klone ein Plasmid mit dem Omega-Fragment enthalten, da dieses das Gen für die Expression der Spectinomycinresistenz enthält.

c) Identifizierung rekombinanter Plasmide

Aus den so erhaltenen Ampicillin- und Spectinomycin-resistenen Klonen wird die Plasmid-DNA nach T. Maniatis et al. (vgl. oben) isoliert. Durch Restriktion mit HindIII werden die neukombinierten Plasmide identifiziert. Ergeben sich dabei drei HindIII-Fragmente der Größen 0.6, 2.0 und 3.2 Kilobasen, so handelt es sich dabei um Derivate von pTJS'X535, die das Omegafragment an der ehemaligen Bgl II-Schnittstelle eingebaut haben. Durch Ligation der kompatiblen überlappenden Enden der Bgl II- und BamHI-Fragmente gehen dabei die Restriktionsstellen Bgl II und BamHI an der Verknüpfungsstelle verloren. Diese neukombinierten Plasmide werden als pTJS'X535omega bezeichnet.

Beispiel 12: Herstellung der Phagen MJSS'030 und MJSS'031

a) Isolierung des Plasmids pKJS32 und der doppelsträngigen Formen der Phagen M13tg130 und M13tg131

Der Stamm E. coli JM101 [J.Messing et al. Nucl. Acids Res. 9:309 (1981)] sowie Doppelstrang-DNA der Vektoren M13tg130 und M13tg131 [M.P. Kieny et al. Gene 26:91 (1983)] können von Amersham Buchler GmbH & Co. KG, Braunschweig, bezogen werden. Doppelstrang-DNA kann auch nach folgendem Verfahren gewonnen werden: 0.2 ml kompetente Zellen von E. coli JM101, hergestellt nach T.J. Silhavy et al. (Experiments with gene fusions. Cold Spring Harbour Laboratory. Cold Spring Harbour, New York, 1984), werden mit Einzelstrang- oder Doppel-Strang-DNA der M13-Vektoren nach einem literaturbekannten Verfahren transformiert, mit 3 ml geschmolzenem (45 °C) H-Top-Agar (10 g/l Trypton, 8 g/l NaCl, 8 g/l Agar) gemischt und auf H-Agar-Platten (10 g/l Trypton, 8 g/l NaCl, 12 g/l Agar) ausgegossen. Die erkalteten Platten werden 16 Stunden bei 37 °C inkubiert. 2 ml TY-Medium (16 g/l Trypton, 10 g/l Hefeextrakt, 5 g/l NaCl) werden mit 20 µl einer über-Nacht-Kultur (16 Stunden, 37 °C) von E. coli JM101 in TY-Medium und einem einzelnen Plaque von der H-Agar-Platte angeimpft. Nach 16 Stunden Wachstum bei 37 °C werden die phageninfizierten Zellen zusammen mit 2 ml einer frischen über-Nacht-Kultur von E. coli JM101 in 400 ml TY-Medium überimpft und 16 Stunden bei 37 °C angezogen. Aus den abzentrifugierten Zellen wird die Doppelstrang-DNA nach T. Maniatis et al. (vgl. oben) isoliert. Die Isolierung von Plasmid pKJS32 wird bereits in Beispiel 5 beschrieben.

b) Klonierung des SacI/SalI-Fragments aus pKJS32 in M13tg130 und M13tg131

Eine Reaktionsmischung (A), bestehend aus 30 $\mu$l (1.5 $\mu$g) pKJS32, 20 $\mu$l Wasser, 6 $\mu$l TAS-Puffer und je 2 $\mu$l (4 Units) von verdünnten Lösungen der Enzyme SacI und SalI, wird 120 min bei 37 °C inkubiert. Zwei weitere Reaktionsmischungen, bestehend aus 10 $\mu$l (300 ng) Doppelstrang-DNA von M13tg130 (B) oder M13tg131 (C), 25 $\mu$l Wasser, 4 $\mu$l TAS-Puffer und je 0.5 $\mu$l (1 Unit) von verdünnten Lösungen der Enzyme SacI und SalI, werden in gleicher Weise inkubiert und zum Abstoppen der Reaktionen für 15 min auf 68 °C erhitzt. Der gesamte Restriktionsansatz A wird in einem Agarosegel (0,7% Agarose, 0.5 $\mu$g/ml Ethidiumbromid in TBE-Puffer nach T. Maniatis et al. (vgl. oben)) elektrophoretisch aufgetrennt. Von den beiden im UV-Licht sichtbaren DNA-Banden wird die kleinere, 2.8 Kilobasen große SacI/SalI-Bande in gleicher Weise mittels einer DEAE-Membran aus dem Gel isoliert, wie dies für die 2.0 Kilobasen große Bande in Beispiel 9 beschrieben wird. Die DNA wird zuletzt in 80 $\mu$l Wasser aufgenommen, davon werden je 40 $\mu$l mit 20 $\mu$l der Restriktionsansätze A (M13tg130) bzw. B (M13tg131) und 40 $\mu$l Wasser versetzt, mit 100 $\mu$l gepuffertem Phenol/Chloroform nach T. Maniatis et al. (vgl. oben) gemischt und zentrifugiert. Die oberen wäßrigen Phasen werden abgenommen und nach Zusatz von 10 $\mu$l 3 M Natiumacetatlösung nach T. Maniatis et al. (vgl. oben) mit je 200 $\mu$l kaltem (-20 °C) Ethanol gemischt. Die Mischungen werden 30 min bei -20 °C belassen, anschließend zentrifugiert, die Niederschläge mit kaltem 70%igen Ethanol gewaschen, im Vakuum getrocknet und in 40 $\mu$l Ligationspuffer aufgenommen. Die beiden Ansätze werden mit je 1 $\mu$l (1 Unit) T4-DNA-Ligase versetzt und 16 Stunden bei 14 °C inkubiert. Je 0.2 ml kompetente Zellen von E. coli JM101, hergestellt nach T.J. Silhavy et al. (Experiments with gene fusions. Cold Spring Harbour Laboratory. Cold Spring Harbour, New York, 1984), werden mit 10 $\mu$l der Ligationsansätze gemischt, 40 min auf Eis belassen, 3 min auf 42 °C erhitzt und mit je 3 ml geschmolzenem (45 °C) H-Top-Agar gemischt. Die Mischungen werden mit je 40 $\mu$l IPTG (100 mM) und 40 $\mu$l X-Gal (2% in Dimethylformamid) versetzt und auf H-Agar-Platten ausgegossen. Die erkalteten Platten werden 16 Stunden bei 37 °C inkubiert.

c) Identifizierung der rekombinanten Phagen

Von den so erhaltenen Plaques stammen diejenigen von rekombinanten Phagen, welche keine blaue Färbung aufweisen. Zur weiteren Identifizierung wird die Doppelstrang- und die Einzelstrang-DNA aus den farblosen Plaques nach literaturbekannten Verfahren, zusammengefaßt im "M13 Cloning and Sequencing Handbook" von Amersham, isoliert. Durch Restriktion der Doppel-Strang-DNA mit BglII und anschließende elektrophoretische Auftrennung der DNA-Fragmente werden Derivate von M13tg130 bzw. M13tg131 identifiziert, die das kleine SacI/SalI-Fragment aus PKJS32 enthalten. Rekombinante Phagen mit M13tg130 als Vektor ergeben drei BglII-Fragmente der Größen 0.7, 2.2 und 7.1 Kilobasen und werden als MJSS'030 bezeichnet, solche mit M13tg131 ergeben vier BglII-Fragmente der Größen 0.6, 0.7, 2.2 und 6.6 Kilobasen und werden als MJSS'031 bezeichnet. Sie unterscheiden sich damit eindeutig von ihren Ausangsprodukten.

Beispiel 13: Herstellung der tfdA-Mutanten Alcaligenes eutrophus JMP134: Tn5-2 und JMP134:Tn5-4

a) Transposonmutagenese von Alcaligenes eutrophus JMP134

10 ml PYE-Medium (3 g/l Pepton, 3 g/l Hefeextrakt) werden mit 0.5 ml einer über-Nacht-Kultur (16 Stunden, 30 °C) von Alcaligenes eutrophus JMP134 [R.H. Don et al. J. Bacteriol. 145:681 (1981)] angeimpft und 8 Stunden bei 30 °C geschüttelt. 10 ml LB-Medium nach T. Maniatis et al. (vgl. oben) werden mit 0.1 ml einer über-Nacht-Kultur von E. coli S17-1 [Biotechnology 1:784 1983)], die das Plasmid pSUP2021 enthält, angeimpft und 5 Stunden bei 37 °C geschüttelt. Von beiden Kulturen wird anschließend im Photometer die optische Dichte bei 600 nm Wellenlänge bestimmt. Die Kulturen werden im Verhältnis 1:1 bezüglich ihrer optischen Dichte gemischt und 1.5 ml der Mischung werden auf einer PYE-Agar-Platte [R.H. Don et al. J. Bacteriol. 145:681 (1981)] verteilt. Die Platte wird 16 Stunden bei 30 °C inkubiert. Anschließend werden die Bakterien mit 1.5 ml steriler 0.7%iger NaCl-Lösung von der Platte gespült und die Bakteriensuspension wird in zwei Schritten mit 0.7%iger NaCl-Lösung jeweils 1:10 (0.1 ml + 0.9 ml) verdünnt. Von jeder Verdünnung werden Portionen von 50 $\mu$l, 100 $\mu$l, 200 $\mu$l und 400 $\mu$l auf Minimal-Agar-Platten (1.6 g/l Dikaliumhydrogenphosphat, 0.4 g/l Kaliumdihydrogensulfat, 1 g/l Ammoniussulfat, 0.05 g/l Magnesiumsulfatx7Wasser, 0.01 g/l Eisen(II)sulfatx7Wasser, 15 g/l Agar) ausgestrichen, die zusätzlich 2 g/l Fructose und 380 $\mu$g/ml Kanamycin enthalten. Die Platten werden 3 bis 7 Tage bei 30 °C inkubiert.

b) Test der transposonhaltigen Stämme auf 2,4-D-Abbau

Die so erhaltenen Kanamycin-resistenten Klone stellen Abkömmlinge des Stammes JMP134 dar, die das Transposon Tn5 stabil in ihrem Genom integriert haben. Sie werden parallel auf zwei Minimal-Agar-Platten auzgestrichen, von denen eine 1 mM 2,4-Dichlorphenoxyessigsäure, Natriumsalz (2,4-D), die andere 2 g/l Fructose und 380 $\mu$g/ml Kanamycin enthält. Die Platten werden 3 Tage bei 30 °C inkubiert. Stämme, die eine Mutation in einem der für den 2,4-D-Abbau verantwortlichen Gene aufweisen, können 2,4-D nicht als Wachstumssubstrat verwenden (2,4-D-negativ). Sie treten mit einer Häufigkeit von 0,1 bis 1 % auf, bezogen auf die Gesamtheit der transposonhaltigen, Kanamycin-resistenten Klone.

c) Identifizierung des Gendefekts der 2,4-D-negativen Mutanten

Die so erhaltenen 2,4-D-negativen Mutanten werden parallel auf zwei Minimal-Agar-Platten ausgestrichen, von denen eine 2 mM 3-Chlorbenzoesäure, Natriumsalz (3-CB), die andere 2 g/l Fructose und 380 $\mu$g/ml Kanamycin enthält. Die Platten werden 3 Tage bei 30 °C inkubiert. 2,4-D-negative Transposonmutanten vom Stamm JMP134, die eine Mutation in den Genen tfdC, tfdD und tfdE des 2,4-D-Abbaues aufweisen, können 3-CB nicht als Wachstumssubstrat verwenden (3-CB-negative), wie Den et al. [J. Bacteriol. 161:85 (1985)] zeigen konnten. Mutanten in den Genen tfdA und tfdB hingegen können auf 3-CB als einziger Kohlenstoffquelle wachsen (3-CB-positiv). Transposonmutanten, die sich in diesem Test als 3-CB-positiv erweisen, werden demnach weiterhin mittels eines Enzymtests auf Defekte in den Genen tfdA oder tfdB untersucht. Dazu werden die Stämme in 250 ml Minimal-Medium (wie oben, ohne Agar) mit einem Zusatz von 15 mM Natriumpyruvat und 1 mM 3-CB 16 Stunden bei 30 °C angezogen. Die Zellen werden durch Zentrifugation bei 4 °C geerntet, dreimal in 10 ml kaltem (4 °C) Minimal-Medium (ohne Kohlenstoffquelle) gewaschen und rezentrifugiert, und zuletzt in soviel Minimal-Medium suspendiert, daß sich eine optische Dichte bei 420 nm von 30 ergibt. Für den Enzymtest der 2,4-D-monooxigenase bzw. der 2,4-Dichlorphenolhydroxylase wird 1 Volumenanteil der Zellsuspension mit 9 Volumenteilen eines sauerstoffgesättigten Minimal-Mediums gemischt, so daß sich eine optische Dichte bei 420 nm von 3 ergibt. Mit Hilfe einer handelsüblichen Sauerstoffelektrode wird dann über 10 min die Sauerstoffaufnahme ohne Substratzusatz verfolgt. Anschließend wird 2,4-D zu einer Endkonzentration von 1 mM oder 2,4-Dichlorphenol zu einer Endkonzentration von 0.2 mM zugegeben und die Abnahme der Sauerstoffkonzentration über 20 min verfolgt. Durch diesen Test lassen sich tfdA-Mutanten von allen anderen Mutationstypen sowie vom Wildtypstamm JmP134 unterscheiden: Sie zeigen nach Zusatz von 2,4-D keinerlei Erhöhung des Sauerstoffverbrauchs, während nach Zusatz von 2,4-Dichlorphenol eine signifikante Steigerung der Sauerstoffaufnahme eintritt. Der Wildtypstamm sowie tfdB-Mutanten zeigen nach Zusatz von 2,4-D als Substrat einen gesteigerten Sauerstoffverbrauch und damit eine intakte 2,4-D-monooxigenase. Bei den beiden Transposonmutanten JMP134:Tn5-2 und JMP134:Tn5-4 handelt es sich um zwei 2,4-D-negative, 3-CB-positive Mutanten mit einer nachweisbaren 2,4-Dichlorphenolhydroxylase aber ohne nachweisbare 2,4-D-monooxigenase.

Sie können damit als tfdA-Mutanten identifiziert werden. Diese Zuordnung wird durch weitere in den folgenden Beispielen beschriebene Versuche bestätigt.

Beispiel 14: Expression klonierter tfdA-Gene in anderen gram-negativen Bakterien als E. coli

a) Herstellung von Donorstämmen für den konjugativen Transfer tfdA-haltiger Plasmide

Die auf der Grundlage von mobilisierbaren Vektoren mit breitem Wirtsbereich wie pVK101 [V.C.Knauf et al. Plazmid 8:45 (1982)], pGSS33 [G.S. Sharpe, Gene 29:93 (1984)] und pKT231 [M. Bagdasarian et al. Current Topics in Microbiology and Immunology 96:47 (1982)] konstruierten Plasmide, deren Herstellung in den Beispielen 1 bis 7 beschrieben ist, können aus dem mobilisierenden Stamm E. coli S17-1 [Biotechnology 1:784 (1983)] durch Konjugation auf andere gram-negative Bakterien wie z.B. Alcaligenes eutrophus und Pseudomonas putida übertragen werden. Dazu müssen sie erst durch Transformation in E. coli S17-1 gebracht werden, sofern sie nicht bereits in diesem Stamm kloniert wurden. Zu diesem Zweck wird aus den Klonen von E. coli LE392, die das betreffende Plasmid enthalten, die Plasmid-DNA nach T. Maniatis et al. (vgl . oben) isoliert. 0.2 ml kompetente Zellen von E. coli LE392, hergestellt nach T.J. Silhavy et al. (Experiments with gene fusions. Cold Spring Harbour Laboratory. Cold Spring Harbour, New York, 1984), werden mit 1 $\mu$l der isolierten Plasmid-DNA gemischt, 40 min auf Eis belassen, 3 min auf 42 °C erhitzt, mit 2 ml LB-Medium gemischt und 60 min bei 37 °C inkubiert. Portionen von 50 $\mu$l bis 200 $\mu$l werden auf LB-Agar-Platten ausgestrichen, die das entsprechende zur Selektion geeignete Antibiotikum enthalten. Die Platten werden 16 Stunden bei 37 °C inkubiert.

b) Konjugativer Transfer

Ein so erhaltener Stamm von E. coli S17-1, der eines der in den Beispielen 1 bis 7 beschriebenen Plasmide enthält, wird 16 Stunden bei 37 °C in 5 ml LB-Medium angezogen, das ein zur Selektion geeignetes Antibiotikum enthält. Als Rezipient wird einer der beiden in Beispiel 13 isolierten tfdA-Mutanten (Fall A), der pJP4-freie Stamm Alcaligenes eutrophus JMP222 [R.H. Don et al. J. Bacteriol. 145:681 (1981)] (Fall B) oder Pseudomonas spec. B13 [E. Dorn et al. Arch. Microbiol. 99:61 (1974)] (Fall C) in 5 ml PYE-Medium (3 g/1 Pepton, 3 g/1 Hefeextrakt) 16 Stunden bei 30 °C angezogen. Die Zellen des Donorstammes werden abzentrifugiert und in dem doppelten Volumen PYE-Medium suspendiert. Die Suspension der Donorzellen und die Kultur des Rezipienten werden zu gleichen Teilen gemischt. 100 µl der Mischung werden auf einen Membranfilter (Millipore HA 0.45 µm) pipettiert, der sich auf der Oberfläche einer PYE-Agar-Platte befindet. Anschließend wird die Platte 6 Stunden bei 30 °C inkubiert. Dann werden die Zellen mit 1 ml 0.7%iger NaCl-Lösung vom Filter abgewaschen und die Zellsuspension wird in 7 Schritten jeweils 1:10 (0.1 ml + 0.9 ml) mit 0.7%iger NaCl-Lösung verdünnt. Von jeder Verdünnung werden 100 µl auf einer Minimal-Agar-Platte ausgestrichen, die eines der folgenden Wachstumssubstrate enthält: 1 mM 2,4-D im Fall A (tfdA-Mutante), 4 mM Phenoxyessigsäure-Na im Fall B (Alcaligenes eutrophus JMP222) oder 1 mM 4-Chlorphenoxyessigsäure-Na im Fall C (Pseudomonas B13). Die Platten werden im Fall A 14 Tage, im Fall B und C 4 Tage bei 30 °C inkubiert.

c) Eigenschaften der neu entstandenen Stämme

Mobilisierbare Plasmide mit breitem Wirtsbereich aus der oben genannten Gruppe, welche ein intaktes tfdA-Gen enthalten, ermöglichen nach konjugativem Transfer dem jeweiligen Rezipienten-Stamm, eine funktionsfähige 2,4-D-monooxigenase zu synthetisieren. Dies führt im Fall A (tfdA-Mutante) zur Komplementation der Mutation und damit zur Wiederherstellung der Wildtypeigenschaft in Bezug auf die Verwertung von 2,4-D, d.h. es entstehen 2,4-D-positive Stämme, die auf 2,4-D-Minimal-Agar-Platten wachsen. Die von tfdA kodierte 2,4-D-monooxigenase ist außerdem in der Lage, neben 2,4-D auch die mit 2,4-D chemisch verwandten Verbindungen Phenox yessigsäure und 4-Chlorphenoxyessigsäure enzymatisch umzusetzen, wobei als Produkte Phenol bzw. 4-Chlorphenol gebildet werden. Da nun der Stamm Alcaligenes eutrophus JMP222 Gene für die vollständige Metabolisierung von Phenol besitzt, ergeben sich nach konjugativem Transfer tfdA-haltiger Plasmide im Fall B Stämme mit der neuen Eigenschaft, Phenoxyessigsäure als Wachstumssubstrat zu nutzen. In gleicher Weise ermöglicht es ein tfdA-haltiges Plasmid einen damit ausgestatteten Pseudomonas B13, welcher komplette Abbauwege für Phenol und 4-Chlorphenol besitzt, auf den Substraten Phenoxyessigsäure und 4-Chlorphenoxyessigsäure zu wachsen (Fall C). Der Test auf Verwertung eines bestimmten Substrates, wie er oben beschrieben wird, eignet sich besonders zum Nachweis der Expression des tfdA-Gens durch in mobilisierbaren Broad-Host-Range-Vektoren klonierte DNA-Fragmente und deren verkürzte Derivate, deren Herstellung in den Beispielen 1 bis 7 beschrieben wird. Dabei zeigt sich, daß die Plasmide pVJH21, pGJS3, pKJS31, pKJS32, pKJSE330, pKJS32RHΔS' und PKJS(X)630 alle ein intaktes tfdA-Gen enthalten, während das Plasmid pKJEΔB130 kein funktionsfähiges Genproddkt kodiert.

Beispiel 15: Anreicherung von tfdA-kodierter 2,4-D-monooxigenase in E. coli

a) Herstellung hochproduzierender E. coli Stämme

Die auf der Grundlage der von Tabor konstruierten Vektoren pT7-5 und pT7-6 hergestellten rekombinanten Plasmide, die in den Beispielen 8, 9, 10 und 11 beschrieben werden, enthalten klonierte DNA-Fragmente im Anschluß an einen starken Promotor des Phagen T7. Plasmide aus dieser Gruppe, welche ein intaktes tfdA-Gen in der richtigen Orientierung zum Promotor enthalten, können unter dem Einfluß der von dem Plasmid PGP1-2 [S. Tabor et al. Proc. Natl. Acad. Sci. USA 82:1074 (1985)] edprimierten T7-RNA-Polymerase in E. coli K38 in großen Mengen 2,4-D-monooxigenase produzieren. Da der Promotor spezifisch nur von der T7-RNA-Polymerase erkannt wird, und da diese auf dem Plasmid pGP1-2 unter der Kontrolle eines hitzesensitiven lambda-Repressors steht, kann die Synthese des Genproduktes durch Hitze induziert werden. Durch Zugabe von Rifampicin können außerdem die bakteriellen RNA-Polymerasen gehemmt werden. Zur Herstellung der hochproduzierenden Stämme wird die Plasmid-DNA aus den in den Beispielen 8 bis 11 erhaltenen plasmidhaltigen Stämmen von E. coli LE392 nach T. Maniatis et al. (vgl. oben) isoliert. E. coli K38, der das Plasmid pGP1-2 enthält, wird 16 Stunden bei 30 °C in LB-Medium angezogen, das zur Selektion auf pGP1-2 mit 50 µg/ml Kanamycin versetzt worden ist. Aus der gewachsenen Kultur werden kompetente Zellen nach dem Verfahren von T.J. Silhavy et al. (Experiments with gene fusions. Cold Spring Harbour Laboratory. Cold Spring Harbour, New York, 1984), hergestellt. 0.2 ml kompetente Zellen werden mit 1 µl der isolierten DNA gemischt, 40 min auf Eis belassen, 5 min auf 37 °C erhitzt, mit 2 ml LB-Medium gemischt und 60 min bei 30 °C inkubiert. Portionen von 50 µl bis 200 µl werden auf LB-Agar-Platten ausgestrichen, die 50 µg/ml Ampicillin und 50 ug/ml Kanamycin enthalten. Die Platten werden 16 Stunden bei 30 °C inkubiert.

b) Induzierte Produktion und radioaktive Markierung des tfdA-Genprodukts

Die so erhaltenen Ampicillin- und Kanamycin-resistenten Klone enthalten das Plasmid pGP1-2 und eines der rekombinanten pT7-Derivate. Sie werden in 10 ml LB-Medium mit einem Zusatz von 50 µg/ml Ampicillin und 50 µg/ml Kanamycin bei 30 °C angezogen. Bei einer optischen Dichte von 0.5, gemessen bei 590 nm, werden 0.2 ml Kultur abzentrifugiert. Die Zellen werden in 5 ml M9-Medium nach T. Maniatis et al. (vgl. oben) gewaschen, wieder abzentrifugiert und schließlich in 1 ml M9-Medium suspendiert, das 20 µg/ml Thiamin und je 0.01 % aller proteinogenen Aminosäuren außer Methionin und Cystein (insgesamt 18) enthält. Die Zellsuspension wird 60 min bei 30 °C geschüttelt, dann wird die Temperatur auf 42 °C erhöht. Nach 15 min werden 10 µl Rifampicin-Lösung (20 mg/ml in Methanol) zugegeben und der Ansatz wird weitere 10 min bei 42 °C belassen. Danach wird die Temperatur wieder auf 30 °C erniedrigt, nach 20 min wird der Ansatz mit 10 µCi L-($^{35}$S) Methionin (Amersham, cell labeling grade) versetzt und 5 min bei Raumtemperatur belassen. Die Zellen werden anschließend abzentrifugiert, in 120 µl Auftragspuffer (60 mM Tris-HCl, PH 6,8, 1 % Natriumlaurylsulfat, 1 % 2-Mercaptoethanol, 10 % Glycerin, 0.01 % Bromphenolblau) suspendiert und 3 min auf 95 °C erhitzt.

c) Identifizierung des spezifisch radioaktiv markierten tfdA-Genproduktes

Die so erhaltenen Proben werden auf ein 12.5%iges Polyacrylamid-Gel [U.K. Laemmli, Nature 227:680 (1970)] geladen und die gesamten bakteriellen Proteine werden nach dem dort beschriebenen literaturbekannten Verfahren elektrophoretisch aufgetrennt. Nach beendeter Elektrophorese wird das Gel 30 min in einer Mischung aus 2.5 g SERVA Blau G, 454 ml Methanol, 92 ml Essigsäure und 454 ml Wasser gefärbt und anschließend 24 Stunden in einer Mischung aus 50 ml Methanol, 75 ml Essigsäure und 875 ml Wasser entfärbt. Das Gel wird auf einem Filterpapier (Whatman 3MM) mit Hilfe eines handelsüblichen Geltrockners getrocknet, anschließend wird nach T. Maniatis et al. (vgl. oben) die Autoradiographie mit einem Röntgenfilm (Kodak Industrex AX) durchgeführt und der Film entwickelt. Auf den Autoradiogrammen wird ein einzelnes markiertes Protein bei denjenigen Stämmen identifiziert, welche neben pGP1-2 eines der Plasmide pTJSS'035, pTJS'B435, PTJS'X535 oder PTJS' X535omega enthalten. Allen diesen homologen Plasmiden ist gemeinsam, daß das klonierte Fragment von der T7-RNA-Polymerase zum Sall-Ende hin transkribiert wird. Bei Plasmiden mit der umgekehrten Orientierung des Inserts (pTJSS'036, pTJS'B435 und pTJS'X535) wird kein spezifisch markiertes Protein gefunden. Durch Vergleich mit Standardproteinen bekannter Größe wird das Molekulargewicht der markierten Proteine bestimmt. Dabei ergibt sich für die von pTJSS'035, pTJS'B435 und pTJS'X535 exprimierten Genprodukte ein Molekulargewicht von 32 000, für das von pTJS'X535omega exprimierte Genprodukt ein Molekulargewicht von 29 000.

d) Induzierte Produktion mit Nachweis der enzymatischen Aktivität

Die oben erhaltenen Stämme von E. coli K38, welche pGP1-2 und eines der Plasmide pTJSS'035, pTJS'B435, pTJS'X535 oder pTJS'X535omega enthalten, werden bei 30 °C in 20 ml LB-Medium mit einem Zusatz von 50 µg/ml Ampicillin und 50 µg/ml Kanamycin angezogen. Bei einer optischen Dichte von 1.0 bei 590 nm wird die Temperatur auf 42 °C erhöht und die Kultur 25 min geschüttelt. Danach werden 100 µl Rifampicin-Lösung (20 mg/ml in Methanol) zugegeben und die Kultur 2 Stunden bei 37 °C geschüttelt. Der Nachweis der enzymatischen Aktivität der 2,4-D-monooxigenase in E. coli folgt dem von Amy et al. [Appl. Env. Microbiol. 49:1237 (1985)] beschriebenen Verfahren des radioaktiven Enzymtests mit folgenden Änderungen: Die Zellen einer induzierten 20 ml-Kultur werden durch Zentrifugieren geerntet, mit 10 ml EM-Medium gewaschen, wieder abzentrifugiert und schließlich in 10 ml EM-Medium suspendiert. Die Zellsuspension wird in einem 250 ml Warburg-Kolben mit 10 ml Transformationspuffer gemischt, mit 200 µg unmarkiertem 2,4-D und 0.1 µCi 2,4-Dichlorphenoxy(2-$^{14}$C)essigsäure (Amersham, 55 mCi/mmol) versetzt und im verschlossenen Kolben 4 Stunden bei 21 °C geschüttelt. Anschließend werden 0.5 ml beta-Phenylethylamin in das zentrale Gefäß des Warburg-Kolbens pipettiert und 2 ml 1 M Schwefelsäure werden in die Zellsuspension injiziert. Nach 1 Stunde Schütteln bei 21 °C wird das beta-Phenylethylamin in 5 ml Zählflüssigkeit (rotiszint 22, Roth) aufgenommen und die Radioaktivität der Probe im Szintillationszähler gemessen. Stämme von E. coli K38, welche neben pGP1-2 eines der Plasmide pTJSS'035, pTJS'B435 oder pTJS'X535 enthalten, weisen in diesem Test hohe Enzymaktivität auf, während solche mit dem Plasmid pTJS'X535omega keine Enzymaktivität exprimieren.

Beispiel 16: Bestimmung der Basensequenz der in M13 klonierten DNA

a) Herstellung deletierter Phagen aus MJSS'030 und MJSS'031

Zur Sequenzierung des 2 Kilobasen großen BamHI/Sall-Fragments der rekombin anten Phagen nach der Methode von Sanger [Proc. Natl. Acad. Sci. USA 74:5463 (1977)] wird zunächst durch gerichtete Deletion nach einem literaturbekannten Verfahren [G. Henikoff, Gene 28:351 (1984)] eine Serie von unterschiedlich verkürzten Phagen hergestellt, die es erlauben, durch überlappende Sequenzierung von jeweils 200 bis 300 Basen die gesamte Basensequenz zu ermitteln. Dazu wird aus Stämmen von E. coli JM101, welche die in Beispiel 12 hergestellten Phagen MJSS'030 bzw. MJSS'031 enthalten, nach dem dort für die Phagenvektoren M13tg130 und M13tg131 beschriebenen Verfahren die Doppelstrang-DNA isoliert. 10 $\mu$g Doppelstrang-DNA des Phagen MJSS'030 werden mit den Ehzymen PstI und SalI geschnitten, 10 $\mu$g Doppelstrang-DNA des Phagen MJSS'031 werden mit den Enzymen BamHI und SacI geschnitten. Die Restriktionsansätze werden mit Phenol extrahiert und die geschnittene DNA wird mit Ethanol gefällt. Die anschließende Verdauung der DNA mit Exonuclease III und S1-Nuclease, die Klenow-Füllung der überstehenden DNA-Enden und die Ligation erfolgen genau nach dem von Henikoff beschriebenen Verfahren mit folgender Änderung: Anstelle von S1-Nuclease wird Mung Bean Nuclease (Pharmacia) verwendet. Portionen von 0.2 ml kompetenter Zellen von E. coli JM101 [J. Messing et al. Nucl. Acids Res. 9:309 (1981)], hergestellt nach dem Verfahren von T.J. Silhavy et al. (Experiments with gene fusions. Cold Spring Harbour Laboratory. Cold Spring Harbour, New York, 1984), werden mit je 20 $\mu$l der Ligationsansätze versetzt, 40 min auf Eis belassen, 3 min auf 42 °C erhitzt, mit je 3 ml geschmolzenem (45 °C) H-Top-Agar (Beispiel 12) gemischt und auf H-Agar-Platten (Beispiel 12) ausgegossen. Die Platten werden 16 Stunden bei 37 °C inkubiert.

b) Identifizierung deletierter Phagen

E. coli JM101 wird in 2 ml TY-Medium (Beispiel 12) 16 Stunden bei 37 °C angezogen. 1 ml dieser Kultur wird mit 100 ml TY-Medium verdünnt und je 2 ml der verdünnten Zellsuspension werden mit einem der oben erhaltenen Plaques infiziert. Die Kulturen werden 5 Stunden bei 37 °C geschüttelt und dann zentrifugiert. Aus den Überständen der Zentrifugation wird die Einzelstrang-DNA nach dem von Amersham [M13 Cloning and Sequencing Handbook, 1984] angegebenen Verfahren gewonnen. Aus den abzentrifugierten Zellen wird die Doppelstrang-DNA nach T. Maniatis et al. (vgl. oben) isoliert. Die Doppelstrang-DNA der aus MJSS'030 hervorgegangenen verkürzten Phagen wird mit BglII, diejenige der aus MJSS'031 hervorgegangen Phagen mit EcoRI und SalI verdaut. Durch anschließende elektrophoretische Auftrennung der Bruchstücke auf einem 2%igen Agarosegel nach T. Maniatis et al. (vgl. oben) wird die Größe der jeweiligen Deletion bestimmt.

c) Sequenzierung der Einzelstrang-DNA

Von der oben erhaltenen Einzelstrang-DNA der verürzten Phagen sowie von der in Beispiel 12 hergestellten Einzelstrang-DNA von MJSS'030 und MJSS'031 wird die Basensequenz nach dem literaturbekannten Verfahren der Didesoxynukleotid-Sequenzierung [Proc. Natl. Acad. Sci. USA 74:5463 (1977)] bestimmt. Dabei wird die von Amersham gegebene Vorschrift [M13 Cloning and Sequencing Handbook, 1094] befolgt. Zur Auftrennung der DNA-Fragmente wird ein 55 cm langes Gel mit einer von 0.1 mm bis 0.4 mm ansteigenden Dicke verwendet. Die identifizierten DNA-Sequenzen der verkürzten Phagen werden mit Hilfe eines Computer-Programns [C. Queen et al. Nucl. Acids Res. 12:581 (1984)] an überlappenden Regionen zu einer DNA-Sequenz zusammengefügt,die den gesamten Abschnitt zwischen der BamHI-Schnittstelle und der Sall-Schnittstelle des in M13tg130 bzw. M13tg131 klonierten Inserts umfaßt. Durch Vergleich der beiden so erhaltenen komplementären DNA-Stränge wird die Basensequenz bestätigt.

d) Eigenschaften der sequenzierten DNA

Mit Hilfe des obengenannten Computerprogramms werden alle Eigenschaften der DNA ermittelt, die sich unmittelbar aus der Basenfolge ergeben. Darunter sind als wichtigste zu nennen: die Lage der Erkennungsstellen für Restriktionsendonukleasen, die Lage und Länge der offenen Leserahmen als mögliche ködierende Regionen eines Gens, die Häufigkeit bestimmter Basen und deren Verteilung, und das Vorkommen bestimmter funktioneller DNA-Sequenzen. Die Analyse der DNA-Sequenz zeigt einen offenen Leserahmen, dessen Lage, Länge und Transkriptionsrichtung mit den in den Beispielen 14 und 15 beschriebenen Eigenschaften des tfdA-Gens übereinstimmt. Er beginnt an einem GTG-Startkodon mit der Base Nr. 748 und endet vor einem TAG-Stopkodon mit der Base Nr. 1608 und hat somit eine Länge von 861 Basen, welche mit der Länge des von tfdA kodierten Proteins (Beispiel 15) korrespondiert. Die Insertion von Transkriptions- und Translationsstopsignalen in die einzige BglII-Schnittstelle des sequenzierten Fragments, wie es die Klonierung des Omega-Fragments in pTJS'X535 darstellt (Beispiel 11), verkürzt diesen offenen Leserahmen rechnerisch auf 768 Basen, was mit der Expression eines verkürzten und enzymatisch inaktiven Genprodukts durch das Plasmid pTJS'X535omega (Beispiel 15) übereinstimmt.

```
          10        20        30        40        50        60
GGATCCTGTCTCAGCTGGCGCGCAATGCTCGAACCCGCTGCGATATACAGCCGTTCGTAG

          70        80        90       100       110       120
TGCAGGTGCTCCACCGTGATTCCAGGCTCCTGGGGGTAGAAGCGGCCGACACCGAGATGG

         130       140       150       160       170       180
ATGGTGCCGGCACGCAGGGCCTCGATCTGCCGCACCTTGGGCATCAGGGCCAGAGACAGC

         190       200       210       220       230       240
GTCGCCCCGGGACCGCCTGCGTGAACGCATGGAGCAATGCCGGGACGGTCTGGTAGATC

         250       260       270       280       290       300
GCCGTGCCGAGGTAGCCGATATCGAGTTGGCCGATCTCGCCCCGGCTGGCGGCGCGGGAC

         310       320       330       340       350       360
CGGTCCACGGAAGTCCGACCCAGTTCGAGCATGCGCCGTGCATCTTCGAGAAACGCGGCC

         370       380       390       400       410       420
CGGCGGGCGTGAGCTGCACGCCGCGCGCGCTGCGCTCGAACAACAACACGCCCAGATGC

         430       440       450       460       470       480
TGTTCGAGCGCGTGAATCTGTCGCGTGACCGGGGGCTGGGAAATATGCAGCCGCCGCGCG

         490       500       510       520       530       540
GCGGCACCGACGTTGCCCTCCTCCGCGGCAGCAACGAAATAGCGAAGCTGTCGAAACTCC

         550       560       570       580       590       600
ATTCTTCACTCCTGGTGGCTGGCTCCGGCTGCCGGAGAGCCATACCGATCCCGTATCGCT

         610       620       630       640       650       660
CGCGCTGATGGAAGGTATTAGACCATATGGCCCGGCATTTCTAGACTACCGCCATGATAA

         670       680       690       700       710       720
AACTCGGCTGCTCTCTCGTCTGCTGGAACATCTTCAGGCGCGCTGAGCCGTCTTTTTGAA

         730       740       750       760       770       780
ACAGTCTCTTAGAAAAGGAGCAAAAAAGTGAGCGTCGTCGCAAATCCCCTTCATCCTCTT

         790       800       810       820       830       840
TTCGCCGCAGGGGTCGAAGACATCGACCTTCGAGAGGCCTTGGGTTCGACCGAGGTCCGA

         850       860       870       880       890       900
GAGATCGAACGGCTAATGGACGAGAAGTCGGTGCTGGTGTTCCGGGGGCAGCCCCTGAGT

         910       920       930       940       950       960
CAGGATCAGCAGATCGCCTTCGCGCGCAATTTCGGGCCACTCGAAGGCGGTTTCATCAAG

         970       980       990      1000      1010      1020
GTCAATCAAAGACCTTCGAGATTCAAGTACGCGGAGTTGGCGGACATCTCGAACGTCAGT

        1030      1040      1050      1060      1070      1080
CTCGACGGCAAGGTCGCGCAACGCGATGCGCGCGAGGTGGTCGGGAACTTCGCGAACCAG

        1090      1100      1110      1120      1130      1140
CTCTGGCACAGCGACAGCTCCTTTCAGCAACCTGCTGCCCGCTACTCGATGCTCTCCGCG

        1150      1160      1170      1180      1190      1200
GTGGTGGTTCCGCCGTCGGGCGGCGACACCGAGTTCTGCGACATGCGTGCGGCATACGAC

        1210      1220      1230      1240      1250      1260
GCGCTGCCTCGGGACCTCCAATCCGAGTTGGAAGGGCTGCGTGCCGAGCACTACGCACTG
```

24

```
              1280      1290      1300      1310      1320
AACTCCCGCTTCCTGCTCGGCGACACCGACTATTCGGAAGCGCAACGCAATGCCATGCCG

     1330      1340      1350      1360      1370      1380
CCGGTCAACTGGCCGCTGGTTCGAACCCACGCCGGCTCCGGGCGCAAGTTTCTCTTCATC

     1390      1400      1410      1420      1430      1440
GGCGCGCACGCGAGCCACGTCGAAGGCCTTCCGGTGGCCGAAGGCCGGATGCTGCTTGCG

     1450      1460      1470      1480      1490      1500
GAGCTTCTCGAGCACGCGACACAGCGGGAATTCGTGTACCGGCATCGCTGGAACGTGGGA

     1510      1520      1530      1540      1550      1560
GATCTGGTGATGTGGGACAACCGCTGCGTTCTTCACCGCGGACGCAGGTACGACATCTCG

     1570      1580      1590      1600      1610      1620
GCCAGGCGTGAGCTGCGCCGGGCGACCACCCTGGACGATGCCGTCGTCTTAGCGCACGCCA

     1630      1640      1650      1660      1670      1680
TGGCGCACGCCCTTTTCGCGAAGGCCCCACAAGATGTACGCAACCCTGATCAGCGGCAGC

     1690      1700      1710      1720      1730      1740
CGTAGCCTGGACGGCGACACCTTGGCGCAGCGCGTCCTTCGAGCGGCGGGCGGCCTGGCG

     1750      1760      1770      1780      1790      1800
GCATGGGGATTGAGGCCCGGTGATGTCGTCGCCATCCTCATGCGCAATGACTTTCCGGTG

     1810      1820      1830      1840      1850      1860
CTCGAAATGACGCTGGCCGCGAACCGCGCCGGCATCGTTGCGGTGCCTTTGAACTGGCAT

     1870      1880      1890      1900      1910      1920
GCGAACCGGGACGAGATCGCCTTCATCCTCGAGGACTGCAAAGCGCGTGTGCTCGTCGCG

     1930      1940      1950      1960      1970      1980
CACACCGATCTGCTCAAGGGCGTTGCATCCGCGGTGCCCGAGGCCTGCAAGGTGCTGGAA

     1990      2000      2010      2020      2030      2040
GCCGCGTCGCCGCCCGAGATCCGGCAGGCCTATCGGCTGTCCGATGCGTCGTGCACGGCG

     2050
AACCCGGGCACGGTCGAC
```

25

# Formblatt für die Hinterlegung von Mikroorganismen    **M3**

Internationales Aktenzeichen: PCT/

---

### MIKROORGANISMEN

Angaben über den auf Seite .13...., Zeile .12.–26.der Beschreibung genannten Mikroorganismus[1]

**A. KENNZEICHNUNG DER HINTERLEGUNG[2]**

Weitere Hinterlegungen sind auf einem zusätzlichen Blatt angegeben ☐ [3]

Name der Hinterlegungsstelle[4]

**Deutsche Sammlung vo Mikroorganismen**

Anschrift der Hinterlegungsstelle (einschließlich Postleitzahl und Land)[4]

**Grisebachstr. 8**
**D – 3400 Göttingen**

| Datum der Hinterlegung[5] | Eingangsnummer[6] |
|---|---|
| 28. August 1986 | DSM 3829 – 3843 . |

**B. WEITERE ANGABEN[7]** (die Angaben werden auf einem gesonderten Blatt fortgesetzt ☐)

**C. BESTIMMUNGSSTAATEN, FÜR DIE ANGABEN GEMACHT WERDEN[3]** (falls die Angaben nicht alle Bestimmungsstaaten betreffen)

**D. NACHREICHUNG VON ANGABEN[8]**

Folgende Angaben werden später beim Internationalen Büro eingereicht[9] (allgemeine Bezeichnung der Angaben, z.B. "Eingangsnummer der Hinterlegung")

**E.** ☒ Dieses Blatt wurde zusammen mit der internationalen Anmeldung eingereicht (vom Anmeldeamt nachzuprufen)

Dipl.-Verwaltungswirt
(Bevollmächtigter Beamter)   R. KONVALIN

Regierungsamtmann

☐ Datum des Eingangs dieses (vom Anmelder nachgereichten) Blattes beim Internationalen Büro[10]

_____
(Bevollmächtigter Beamter)

---

Formblatt PCT/RO/134 (Januar 1985)     Anmerkungen siehe Beiblatt     (Januar 1986)

# Formblatt für die Hinterlegung von Mikroorganismen

# M3

Internationales Aktenzeichen: PCT/ DE 87 /00392

---

### MIKROORGANISMEN

Angaben über den auf Seite ....13.... Zeile ....12.... der Beschreibung genannten Mikroorganismus[1]

**A. KENNZEICHNUNG DER HINTERLEGUNG[2]**

Weitere Hinterlegungen sind auf einem zusätzlichen Blatt angegeben  [x] [3]

Name der Hinterlegungsstelle[4]

Deutsche Sammlung von Mikroorganismen (DSM)

Anschrift der Hinterlegungsstelle (einschließlich Postleitzahl und Land)[4]

Gisebachstr. 8, D- 3400 Göttingen, Deutschland

| Datum der Hinterlegung[5] | Eingangsnummer[6] |
|---|---|
| 28.08.1986 | DSM 3829 |

**B. WEITERE ANGABEN[7]** (die Angaben werden auf einem gesonderten Blatt fortgesetzt  [ ] )

Für diejenigen Bestimmungen, in denen um ein europäisches Patent nachgesucht wird, wird bis zur Veröffentlichung des Hinweises auf die Erteilung des europäischen Patents oder bis zu dem Tag, an dem die Anmeldung zurückgewiesen oder zurückgenommen wird oder als zurückgenommen gilt, der Zugang zu dem hinterlegten Mikroorganismus nur durch Herausgabe einer Probe des Mikroorganismus an einen Sachverständigen hergestellt, der von der Person, die die Probe beantragt, benannt wird (Regel 28 Absatz 4 EPÜ).

**C. BESTIMMUNGSSTAATEN, FÜR DIE ANGABEN GEMACHT WERDEN[3]** (falls die Angaben nicht alle Bestimmungsstaaten betreffen)

**D. NACHREICHUNG VON ANGABEN[8]**

Folgende Angaben werden später beim Internationalen Büro eingereicht[9] (allgemeine Bezeichnung der Angaben, z.B. "Eingangsnummer der Hinterlegung")

**E.** [X]  Dieses Blatt wurde zusammen mit der internationalen Anmeldung eingereicht (vom Anmeldeamt nachzuprüfen)

am 03. 11. 87

Dipl.-Verwaltungswirt
(Bevollmächtigter Beamter)  R. KONVALIN
Regierungsamtmann

[ ]  Datum des Eingangs dieses (vom Anmelder nachgereichten) Blattes beim Internationalen Büro[10]

_____
(Bevollmächtigter Beamter)

Formblatt PCT/RO/134 (Januar 1985)          Anmerkungen siehe Beiblatt          (Januar 1986)

**EP 0 334 841 B1**

# Formblatt für die Hinterlegung von Mikroorganismen    **M3**

DE 87/00392

Internationales Aktenzeichen: PCT/

| **MIKROORGANISMEN** |
| --- |

Angaben über den auf Seite . . . . . . . . 13 . ., Zeile . . . . . . . 13 . . . der Beschreibung genannten Mikroorganismus[1]

**A. KENNZEICHNUNG DER HINTERLEGUNG[2]**

Weitere Hinterlegungen sind auf einem zusätzlichen Blatt angegeben   [x] [3]

Name der Hinterlegungsstelle[4]

    Deutsche Sammlung von Mikroorganismen (DSM)

Anschrift der Hinterlegungsstelle (einschließlich Postleitzahl und Land)[4]

    Gisebachstr. 8, D- 3400 Göttingen, Deutschland

| Datum der Hinterlegung[5] | Eingangsnummer[6] |
| --- | --- |
| 28.08.1986 | DSM 3830 |

**B. WEITERE ANGABEN[7]** (die Angaben werden auf einem gesonderten Blatt fortgesetzt [ ])

Für diejenigen Bestimmungen, in denen um ein europäisches Patent nachgesucht wird, wird bis zur Veröffentlichung des Hinweises auf die Erteilung des europäischen Patents oder bis zu dem Tag, an dem die Anmeldung zurückgewiesen oder zurückgenommen wird oder als zurückgenommen gilt, der Zugang zu dem hinterlegten Mikroorganismus nur durch Herausgabe einer Probe des Mikroorganismus an einen Sachverständigen hergestellt, der von der Person, die die Probe beantragt, benannt wird (Regel 28 Absatz 4 EPÜ).

**C. BESTIMMUNGSSTAATEN, FÜR DIE ANGABEN GEMACHT WERDEN[3]** (falls die Angaben nicht alle Bestimmungsstaaten betreffen)

**D. NACHREICHUNG VON ANGABEN[8]**

Folgende Angaben werden später beim Internationalen Büro eingereicht[9] (allgemeine Bezeichnung der Angaben, z.B. "Eingangsnummer der Hinterlegung")

E. [X] Dieses Blatt wurde zusammen mit der internationalen Anmeldung eingereicht (vom Anmeldeamt nachzuprüfen)

    am 12. 1. 87

           Dipl.-Verwaltungswirt
      —————————————   R. KONVALIN
      (Bevollmächtigter Beamter)   Regierungsamtmann

[ ] Datum des Eingangs dieses (vom Anmelder nachgereichten) Blattes beim Internationalen Büro[10]

————————————————
(Bevollmächtigter Beamter)

Formblatt PCT/RO/134 (Januar 1985)     Anmerkungen siehe Beiblatt     (Januar 1986)

# Formblatt für die Hinterlegung von Mikroorganismen　**M3**

DE 87/00392

Internationales Aktenzeichen: PCT/

| MIKROORGANISMEN |
|---|

Angaben über den auf Seite ...13..., Zeile ...14... der Beschreibung genannten Mikroorganismus[1]

**A. KENNZEICHNUNG DER HINTERLEGUNG[2]**

Weitere Hinterlegungen sind auf einem zusätzlichen Blatt angegeben　[x][3]

Name der Hinterlegungsstelle[4]

Deutsche Sammlung von Mikroorganismen (DSM)

Anschrift der Hinterlegungsstelle (einschließlich Postleitzahl und Land)[4]

Gisebachstr. 8, D- 3400 Göttingen, Deutschland

| Datum der Hinterlegung[5] | Eingangsnummer[6] |
|---|---|
| 28.08.1986 | DSM 3831 |

**B. WEITERE ANGABEN[7]** (die Angaben werden auf einem gesonderten Blatt fortgesetzt ☐)

Für diejenigen Bestimmungen, in denen um ein europäisches Patent nachgesucht wird, wird bis zur Veröffentlichung des Hinweises auf die Erteilung des europäischen Patents oder bis zu dem Tag, an dem die Anmeldung zurückgewiesen oder zurückgenommen wird oder als zurückgenommen gilt, der Zugang zu dem hinterlegten Mikroorganismus nur durch Herausgabe einer Probe des Mikroorganismus an einen Sachverständigen hergestellt, der von der Person, die die Probe beantragt, benannt wird (Regel 28 Absatz 4 EPÜ).

**C. BESTIMMUNGSSTAATEN, FÜR DIE ANGABEN GEMACHT WERDEN[3]** (falls die Angaben nicht alle Bestimmungsstaaten betreffen)

**D. NACHREICHUNG VON ANGABEN[8]**

Folgende Angaben werden später beim Internationalen Büro eingereicht[9] (allgemeine Bezeichnung der Angaben, z.B. "Eingangsnummer der Hinterlegung")

E. [X] Dieses Blatt wurde zusammen mit der internationalen Anmeldung eingereicht (vom Anmeldeamt nachzuprüfen)

*am* ...

(Bevollmächtigter Beamter)

☐ Datum des Eingangs dieses (vom Anmelder nachgereichten) Blattes beim Internationalen Büro[10]

(Bevollmächtigter Beamter)

Formblatt PCT/RO/134 (Januar 1985)　　Anmerkungen siehe Beiblatt　　(Januar 1986)

# Formblatt für die Hinterlegung von Mikroorganismen    **M3**

Internationales Aktenzeichen: PCT/    DE 87/00392

---

### MIKROORGANISMEN

Angaben über den auf Seite ....13...., Zeile ....15.... der Beschreibung genannten Mikroorganismus[1]

**A. KENNZEICHNUNG DER HINTERLEGUNG[2]**

Weitere Hinterlegungen sind auf einem zusätzlichen Blatt angegeben   [x] [3]

Name der Hinterlegungsstelle[4]

    Deutsche Sammlung von Mikroorganismen (DSM)

Anschrift der Hinterlegungsstelle (einschließlich Postleitzahl und Land)[4]

    Gisebachstr. 8, D- 3400 Göttingen, Deutschland

| Datum der Hinterlegung[5] | Eingangsnummer[6] |
|---|---|
| 28.08.1986 | DSM 3832 |

**B. WEITERE ANGABEN[7]** (die Angaben werden auf einem gesonderten Blatt fortgesetzt [ ])

Für diejenigen Bestimmungen, in denen um ein europäisches Patent nachgesucht wird, wird bis zur Veröffentlichung des Hinweises auf die Erteilung des europäischen Patents oder bis zu dem Tag, an dem die Anmeldung zurückgewiesen oder zurückgenommen wird oder als zurückgenommen gilt, der Zugang zu dem hinterlegten Mikroorganismus nur durch Herausgabe einer Probe des Mikroorganismus an einen Sachverständigen hergestellt, der von der Person, die die Probe beantragt, benannt wird (Regel 28 Absatz 4 EPÜ).

**C. BESTIMMUNGSSTAATEN, FÜR DIE ANGABEN GEMACHT WERDEN[3]** (falls die Angaben nicht alle Bestimmungsstaaten betreffen)

**D. NACHREICHUNG VON ANGABEN[8]**

Folgende Angaben werden später beim Internationalen Büro eingereicht[9] (allgemeine Bezeichnung der Angaben, z.B. "Eingangsnummer der Hinterlegung")

**E.** [✓] Dieses Blatt wurde zusammen mit der internationalen Anmeldung eingereicht (vom Anmeldeamt nachzuprüfen)

    am L    27

Dipl.-Verwaltungswirt
R. KON ALIN
Regierungsamtmann

(Bevollmächtigter Beamter)

[ ] Datum des Eingangs dieses (vom Anmelder nachgereichten) Blattes beim Internationalen Büro[10]

(Bevollmächtigter Beamter)

---

Formblatt PCT/RO/134 (Januar 1985)     Anmerkungen siehe Beiblatt     (Januar 1986)

# Formblatt für die Hinterlegung von Mikroorganismen

# M3

Internationales Aktenzeichen: PCT/   DE 87/00392

| MIKROORGANISMEN |
|---|
| Angaben über den auf Seite ..13...., Zeile ...16.... der Beschreibung genannten Mikroorganismus[1] |

**A. KENNZEICHNUNG DER HINTERLEGUNG[2]**

Weitere Hinterlegungen sind auf einem zusätzlichen Blatt angegeben   ☒ [3]

Name der Hinterlegungsstelle[4]

Deutsche Sammlung von Mikroorganismen (DSM)

Anschrift der Hinterlegungsstelle (einschließlich Postleitzahl und Land)[4]

Gisebachstr. 8, D- 3400 Göttingen, Deutschland

| Datum der Hinterlegung[5] | Eingangsnummer[6] |
|---|---|
| 28.08.1986 | DSM 3833 |

**B. WEITERE ANGABEN[7]** (die Angaben werden auf einem gesonderten Blatt fortgesetzt ☐ )

Für diejenigen Bestimmungen, in denen um ein europäisches Patent nachgesucht wird, wird bis zur Veröffentlichung des Hinweises auf die Erteilung des europäischen Patents oder bis zu dem Tag, an dem die Anmeldung zurückgewiesen oder zurückgenommen wird oder als zurückgenommen gilt, der Zugang zu dem hinterlegten Mikroorganismus nur durch Herausgabe einer Probe des Mikroorganismus an einen Sachverständigen hergestellt, der von der Person, die die Probe beantragt, benannt wird (Regel 28 Absatz 4 EPÜ).

**C. BESTIMMUNGSSTAATEN, FÜR DIE ANGABEN GEMACHT WERDEN[3]** (falls die Angaben nicht alle Bestimmungsstaaten betreffen)

**D. NACHREICHUNG VON ANGABEN[8]**

Folgende Angaben werden später beim Internationalen Büro eingereicht[9] (allgemeine Bezeichnung der Angaben, z.B. "Eingangsnummer der Hinterlegung")

**E.** ☒ Dieses Blatt wurde zusammen mit der internationalen Anmeldung eingereicht (vom Anmeldeamt nachzuprüfen)

am 13. 11. 87

(Bevollmächtigter Beamter)

☐ Datum des Eingangs dieses (vom Anmelder nachgereichten) Blattes beim Internationalen Büro[10]

(Bevollmächtigter Beamter)

Formblatt PCT/RO/134 (Januar 1985)     Anmerkungen siehe Beiblatt     (Januar 1986)

# Formblatt für die Hinterlegung von Mikroorganismen

# M3

Internationales Aktenzeichen: PCT/ DE 87/00392

---

### MIKROORGANISMEN

Angaben über den auf Seite ..13.... , Zeile ....17... der Beschreibung genannten Mikroorganismus[1]

**A. KENNZEICHNUNG DER HINTERLEGUNG[2]**

Weitere Hinterlegungen sind auf einem zusätzlichen Blatt angegeben  [X] [3]

Name der Hinterlegungsstelle[4]

Deutsche Sammlung von Mikroorganismen (DSM)

Anschrift der Hinterlegungsstelle (einschließlich Postleitzahl und Land)[4]

Gisebachstr. 8, D- 3400 Göttingen, Deutschland

| Datum der Hinterlegung[5] | Eingangsnummer[6] |
|---|---|
| 28.08.1986 | DSM 3834 |

**B. WEITERE ANGABEN[7]** (die Angaben werden auf einem gesonderten Blatt fortgesetzt  [ ] )

Für diejenigen Bestimmungen, in denen um ein europäisches Patent nachgesucht wird, wird bis zur Veröffentlichung des Hinweises auf die Erteilung des europäischen Patents oder bis zu dem Tag, an dem die Anmeldung zurückgewiesen oder zurückgenommen wird oder als zurückgenommen gilt, der Zugang zu dem hinterlegten Mikroorganismus nur durch Herausgabe einer Probe des Mikroorganismus an einen Sachverständigen hergestellt, der von der Person, die die Probe beantragt, benannt wird (Regel 28 Absatz 4 EPÜ).

**C. BESTIMMUNGSSTAATEN, FÜR DIE ANGABEN GEMACHT WERDEN[3]** (falls die Angaben nicht alle Bestimmungsstaaten betreffen)

**D. NACHREICHUNG VON ANGABEN[8]**

Folgende Angaben werden später beim Internationalen Büro eingereicht[9] (allgemeine Bezeichnung der Angaben, z.B. "Eingangsnummer der Hinterlegung")

E. [X] Dieses Blatt wurde zusammen mit der Internationalen Anmeldung eingereicht (vom Anmeldeamt nachzuprüfen)

am

(Bevollmächtigter Beamter)

Dipl.-Verwaltungswirt
R. KONV.....N
Regierungsamtmann

[ ] Datum des Eingangs dieses (vom Anmelder nachgereichten) Blattes beim Internationalen Büro[10]

(Bevollmächtigter Beamter)

Formblatt PCT/RO/134 (Januar 1985)          Anmerkungen siehe Beiblatt

(Januar 1986)

# Formblatt für die Hinterlegung von Mikroorganismen

# M3

Internationales Aktenzeichen: PCT/   DE 87/00392

| MIKROORGANISMEN |
|---|

Angaben über den auf Seite ..13...., Zeile ........18........ der Beschreibung genannten Mikroorganismus[1]

**A. KENNZEICHNUNG DER HINTERLEGUNG[2]**

Weitere Hinterlegungen sind auf einem zusätzlichen Blatt angegeben   [X] [3]

Name der Hinterlegungsstelle[4]

Deutsche Sammlung von Mikroorganismen (DSM)

Anschrift der Hinterlegungsstelle (einschließlich Postleitzahl und Land)[4]

Gisebachstr. 8, D- 3400 Göttingen, Deutschland

| Datum der Hinterlegung[5] | Eingangsnummer[6] |
|---|---|
| 28.08.1986 | DSM 3835 |

**B. WEITERE ANGABEN[7]** (die Angaben werden auf einem gesonderten Blatt fortgesetzt [ ])

Für diejenigen Bestimmungen, in denen um ein europäisches Patent nachgesucht wird, wird bis zur Veröffentlichung des Hinweises auf die Erteilung des europäischen Patents oder bis zu dem Tag, an dem die Anmeldung zurückgewiesen oder zurückgenommen wird oder als zurückgenommen gilt, der Zugang zu dem hinterlegten Mikroorganismus nur durch Herausgabe einer Probe des Mikroorganismus an einen Sachverständigen hergestellt, der von der Person, die die Probe beantragt, benannt wird (Regel 28 Absatz 4 EPÜ).

**C. BESTIMMUNGSSTAATEN, FÜR DIE ANGABEN GEMACHT WERDEN[3]** (falls die Angaben nicht alle Bestimmungsstaaten betreffen)

**D. NACHREICHUNG VON ANGABEN[8]**

Folgende Angaben werden später beim Internationalen Büro eingereicht[9] (allgemeine Bezeichnung der Angaben, z.B. "Eingangsnummer der Hinterlegung")

**E.** [✓] Dieses Blatt wurde zusammen mit der internationalen Anmeldung eingereicht (vom Anmeldeamt nachzuprüfen)

am ................

Dipl.-Verwaltungswirt

(Bevollmächtigter Beamter)

[ ] Datum des Eingangs dieses (vom Anmelder nachgereichten) Blattes beim Internationalen Büro[10]

(Bevollmächtigter Beamter)

Formblatt PCT/RO/134 (Januar 1985)     Anmerkungen siehe Beiblatt     (Januar 1986)

# Formblatt für die Hinterlegung von Mikroorganismen

# M3

Internationales Aktenzeichen: PCT/ DE 87/00392

---

## MIKROORGANISMEN

Angaben über den auf Seite ..13...., Zeile .....19. der Beschreibung genannten Mikroorganismus[1]

### A. KENNZEICHNUNG DER HINTERLEGUNG[2]

Weitere Hinterlegungen sind auf einem zusätzlichen Blatt angegeben  [X] [3]

Name der Hinterlegungsstelle[4]

Deutsche Sammlung von Mikroorganismen (DSM)

Anschrift der Hinterlegungsstelle (einschließlich Postleitzahl und Land)[4]

Gisebachstr. 8, D- 3400 Göttingen, Deutschland

| Datum der Hinterlegung[5] | Eingangsnummer[6] |
|---|---|
| 28.08.1986 | DSM 3836 |

### B. WEITERE ANGABEN[7] (die Angaben werden auf einem gesonderten Blatt fortgesetzt [ ])

Für diejenigen Bestimmungen, in denen um ein europäisches Patent nachgesucht wird, wird bis zur Veröffentlichung des Hinweises auf die Erteilung des europäischen Patents oder bis zu dem Tag, an dem die Anmeldung zurückgewiesen oder zurückgenommen wird oder als zurückgenommen gilt, der Zugang zu dem hinterlegten Mikroorganismus nur durch Herausgabe einer Probe des Mikroorganismus an einen Sachverständigen hergestellt, der von der Person, die die Probe beantragt, benannt wird (Regel 28 Absatz 4 EPÜ).

### C. BESTIMMUNGSSTAATEN, FÜR DIE ANGABEN GEMACHT WERDEN[3] (falls die Angaben nicht alle Bestimmungsstaaten betreffen)

### D. NACHREICHUNG VON ANGABEN[8]

Folgende Angaben werden später beim Internationalen Büro eingereicht[9] (allgemeine Bezeichnung der Angaben, z.B. "Eingangsnummer der Hinterlegung")

### E. [X] Dieses Blatt wurde zusammen mit der internationalen Anmeldung eingereicht (vom Anmeldeamt nachzuprüfen)

am 3. 5. 11. 87

(Bevollmächtigter Beamter)

Dipl.-Verwalt. R. KÖNIG Regierungs...

[ ] Datum des Eingangs dieses (vom Anmelder nachgereichten) Blattes beim Internationalen Büro[10]

(Bevollmächtigter Beamter)

Formblatt PCT/RO/134 (Januar 1985)     Anmerkungen siehe Beiblatt     (Januar 1986)

# Formblatt für die Hinterlegung von Mikroorganismen

# M3

Internationales Aktenzeichen: PCT/     DE 87/00392

| MIKROORGANISMEN |
|---|

Angaben über den auf Seite ..13..... Zeile ...20...... der Beschreibung genannten Mikroorganismus[1]

### A. KENNZEICHNUNG DER HINTERLEGUNG[2]

Weitere Hinterlegungen sind auf einem zusätzlichen Blatt angegeben    [X] [3]

Name der Hinterlegungsstelle[4]

     Deutsche Sammlung von Mikroorganismen (DSM)

Anschrift der Hinterlegungsstelle (einschließlich Postleitzahl und Land)[4]

     Gisebachstr. 8, D- 3400 Göttingen, Deutschland

| Datum der Hinterlegung[5] | Eingangsnummer[6] |
|---|---|
| 28.08.1986 | DSM 3837 |

### B. WEITERE ANGABEN[7] (die Angaben werden auf einem gesonderten Blatt fortgesetzt [ ])

Für diejenigen Bestimmungen, in denen um ein europäisches Patent nachgesucht wird, wird bis zur Veröffentlichung des Hinweises auf die Erteilung des europäischen Patents oder bis zu dem Tag, an dem die Anmeldung zurückgewiesen oder zurückgenommen wird oder als zurückgenommen gilt, der Zugang zu dem hinterlegten Mikroorganismus nur durch Herausgabe einer Probe des Mikroorganismus an einen Sachverständigen hergestellt, der von der Person, die die Probe beantragt, benannt wird (Regel 28 Absatz 4 EPÜ).

### C. BESTIMMUNGSSTAATEN, FÜR DIE ANGABEN GEMACHT WERDEN[3] (falls die Angaben nicht alle Bestimmungsstaaten betreffen)

### D. NACHREICHUNG VON ANGABEN[8]

Folgende Angaben werden später beim Internationalen Büro eingereicht[9] (allgemeine Bezeichnung der Angaben, z.B. "Eingangsnummer der Hinterlegung")

E. [X] Dieses Blatt wurde zusammen mit der internationalen Anmeldung eingereicht (vom Anmeldeamt nachzuprüfen)

     *am*    37

(Bevollmächtigter Beamter)

[ ] Datum des Eingangs dieses (vom Anmelder nachgereichten) Blattes beim Internationalen Büro[10]

(Bevollmächtigter Beamter)

Formblatt PCT/RO/134 (Januar 1985)      Anmerkungen siehe Beiblatt      (Januar 1986)

# Formblatt für die Hinterlegung von Mikroorganismen

# **M3**

Internationales Aktenzeichen: PCT/    DE 87 /00392

| MIKROORGANISMEN |
|---|
| Angaben über den auf Seite ..13..... Zeile .....21...... der Beschreibung genannten Mikroorganismus[1] |

**A. KENNZEICHNUNG DER HINTERLEGUNG[2]**

Weitere Hinterlegungen sind auf einem zusätzlichen Blatt angegeben   ☒   [3]

Name der Hinterlegungsstelle[4]

Deutsche Sammlung von Mikroorganismen (DSM)

Anschrift der Hinterlegungsstelle (einschließlich Postleitzahl und Land)[4]

Gisebachstr. 8, D- 3400 Göttingen, Deutschland

| Datum der Hinterlegung[5] | Eingangsnummer[6] |
|---|---|
| 28.08.1986 | DSM 3838 |

**B. WEITERE ANGABEN[7]** (die Angaben werden auf einem gesonderten Blatt fortgesetzt ☐ )

Für diejenigen Bestimmungen, in denen um ein europäisches Patent nachgesucht wird, wird bis zur Veröffentlichung des Hinweises auf die Erteilung des europäischen Patents oder bis zu dem Tag, an dem die Anmeldung zurückgewiesen oder zurückgenommen wird oder als zurückgenommen gilt, der Zugang zu dem hinterlegten Mikroorganismus nur durch Herausgabe einer Probe des Mikroorganismus an einen Sachverständigen hergestellt, der von der Person, die die Probe beantragt, benannt wird (Regel 28 Absatz 4 EPÜ).

**C. BESTIMMUNGSSTAATEN, FÜR DIE ANGABEN GEMACHT WERDEN[3]** (falls die Angaben nicht alle Bestimmungsstaaten betreffen)

**D. NACHREICHUNG VON ANGABEN[8]**

Folgende Angaben werden später beim Internationalen Büro eingereicht[9] (allgemeine Bezeichnung der Angaben, z.B. "Eingangsnummer der Hinterlegung")

E. ☒   Dieses Blatt wurde zusammen mit der internationalen Anmeldung eingereicht (vom Anmeldeamt nachzuprüfen)

am       Dipl.-Verwaltungswirt

R. Ko...

(Bevollmächtigter Beamter)    Regierungs...

☐   Datum des Eingangs dieses (vom Anmelder nachgereichten) Blattes beim Internationalen Büro[10]

(Bevollmächtigter Beamter)

Formblatt PCT/RO/134 (Januar 1985)      Anmerkungen siehe Beiblatt      (Januar 1986)

## Formblatt für die Hinterlegung von Mikroorganismen          **M3**

Internationales Aktenzeichen: PCT/    DE 87/00392

---

**MIKROORGANISMEN**

Angaben über den auf Seite ..13...., Zeile ......22. der Beschreibung genannten Mikroorganismus[1]

**A. KENNZEICHNUNG DER HINTERLEGUNG[2]**

Weitere Hinterlegungen sind auf einem zusätzlichen Blatt angegeben   [x] [3]

Name der Hinterlegungsstelle[4]

Deutsche Sammlung von Mikroorganismen (DSM)

Anschrift der Hinterlegungsstelle (einschließlich Postleitzahl und Land)[4]

Gisebachstr. 8, D- 3400 Göttingen, Deutschland

| Datum der Hinterlegung[5] | Eingangsnummer[6] |
|---|---|
| 28.08.1986 | DSM 3839 |

**B. WEITERE ANGABEN[7]** (die Angaben werden auf einem gesonderten Blatt fortgesetzt [ ])

Für diejenigen Bestimmungen, in denen um ein europäisches Patent nachgesucht wird, wird bis zur Veröffentlichung des Hinweises auf die Erteilung des europäischen Patents oder bis zu dem Tag, an dem die Anmeldung zurückgewiesen oder zurückgenommen wird oder als zurückgenommen gilt, der Zugang zu dem hinterlegten Mikroorganismus nur durch Herausgabe einer Probe des Mikroorganismus an einen Sachverständigen hergestellt, der von der Person, die die Probe beantragt, benannt wird (Regel 28 Absatz 4 EPÜ).

**C. BESTIMMUNGSSTAATEN, FÜR DIE ANGABEN GEMACHT WERDEN[3]** (falls die Angaben nicht alle Bestimmungsstaaten betreffen)

**D. NACHREICHUNG VON ANGABEN[8]**

Folgende Angaben werden später beim Internationalen Büro eingereicht[9] (allgemeine Bezeichnung der Angaben, z.B. "Eingangsnummer der Hinterlegung")

**E.** [✓] Dieses Blatt wurde ~~zusammen mit der internationalen Anmeldung~~ eingereicht (vom Anmeldeamt nachzuprüfen)

*an* 1. 11. 87

(Bevollmächtigter Beamter)

[ ] Datum des Eingangs dieses (vom Anmelder nachgereichten) Blattes beim Internationalen Büro[10]

(Bevollmächtigter Beamter)

---

Formblatt PCT/RO/134 (Januar 1985)          Anmerkungen siehe Beiblatt          (Januar 1986)

# Formblatt für die Hinterlegung von Mikroorganismen  **M3**

Internationales Aktenzeichen: PCT/  DE 87/00392

| MIKROORGANISMEN |
|---|

Angaben über den auf Seite ..13...., Zeile ...23.... der Beschreibung genannten Mikroorganismus[1]

**A. KENNZEICHNUNG DER HINTERLEGUNG[2]**

Weitere Hinterlegungen sind auf einem zusätzlichen Blatt angegeben  [X] [3]

Name der Hinterlegungsstelle[4]

Deutsche Sammlung von Mikroorganismen (DSM)

Anschrift der Hinterlegungsstelle (einschließlich Postleitzahl und Land)[4]

Gisebachstr. 8, D- 3400 Göttingen, Deutschland

| Datum der Hinterlegung[5] | Eingangsnummer[6] |
|---|---|
| 28.08.1986 | DSM 3840 |

**B. WEITERE ANGABEN[7]** (die Angaben werden auf einem gesonderten Blatt fortgesetzt [ ])

Für diejenigen Bestimmungen, in denen um ein europäisches Patent nachgesucht wird, wird bis zur Veröffentlichung des Hinweises auf die Erteilung des europäischen Patents oder bis zu dem Tag, an dem die Anmeldung zurückgewiesen oder zurückgenommen wird oder als zurückgenommen gilt, der Zugang zu dem hinterlegten Mikroorganismus nur durch Herausgabe einer Probe des Mikroorganismus an einen Sachverständigen hergestellt, der von der Person, die die Probe beantragt, benannt wird (Regel 28 Absatz 4 EPÜ).

**C. BESTIMMUNGSSTAATEN, FÜR DIE ANGABEN GEMACHT WERDEN[3]** (falls die Angaben nicht alle Bestimmungsstaaten betreffen)

**D. NACHREICHUNG VON ANGABEN[8]**

Folgende Angaben werden später beim Internationalen Büro eingereicht[9] (allgemeine Bezeichnung der Angaben, z.B. "Eingangsnummer der Hinterlegung")

E. [X] Dieses Blatt wurde zusammen mit der internationalen Anmeldung eingereicht (vom Anmeldeamt nachzuprüfen)

an [18. 11. 87]

Dipl.-Verwaltungswirt
R. KOWALSKI
(Bevollmächtigter Beamter)  Regierungsamtmann

[ ] Datum des Eingangs dieses (vom Anmelder nachgereichten) Blattes beim Internationalen Büro[10]

(Bevollmächtigter Beamter)

Formblatt PCT/RO/134 (Januar 1985)       Anmerkungen siehe Beiblatt                (Januar 1986)

# Formblatt für die Hinterlegung von Mikroorganismen **M3**

Internationales Aktenzeichen: PCT/ DE 87/00392

| MIKROORGANISMEN |
|---|
| Angaben über den auf Seite ..13..... Zeile .....24... der Beschreibung genannten Mikroorganismus[1] |

| A. KENNZEICHNUNG DER HINTERLEGUNG[2] |
|---|
| Weitere Hinterlegungen sind auf einem zusätzlichen Blatt angegeben [x] [3] |

Name der Hinterlegungsstelle[4]

**Deutsche Sammlung von Mikroorganismen (DSM)**

Anschrift der Hinterlegungsstelle (einschließlich Postleitzahl und Land)[4]

**Gisebachstr. 8, D- 3400 Göttingen, Deutschland**

| Datum der Hinterlegung[5] | Eingangsnummer[6] |
|---|---|
| 28.08.1986 | DSM 3841 |

**B. WEITERE ANGABEN**[7] (die Angaben werden auf einem gesonderten Blatt fortgesetzt [ ] )

Für diejenigen Bestimmungen, in denen um ein europäisches Patent nachgesucht wird, wird bis zur Veröffentlichung des Hinweises auf die Erteilung des europäischen Patents oder bis zu dem Tag, an dem die Anmeldung zurückgewiesen oder zurückgenommen wird oder als zurückgenommen gilt, der Zugang zu dem hinterlegten Mikroorganismus nur durch Herausgabe einer Probe des Mikroorganismus an einen Sachverständigen hergestellt, der von der Person, die die Probe beantragt, benannt wird (Regel 28 Absatz 4 EPÜ).

**C. BESTIMMUNGSSTAATEN, FÜR DIE ANGABEN GEMACHT WERDEN**[3] (falls die Angaben nicht alle Bestimmungsstaaten betreffen)

**D. NACHREICHUNG VON ANGABEN**[8]

Folgende Angaben werden später beim Internationalen Büro eingereicht[9] (allgemeine Bezeichnung der Angaben, z.B. "Eingangsnummer der Hinterlegung")

E. [X] Dieses Blatt wurde zusammen mit der internationalen Anmeldung eingereicht (vom Anmeldeamt nachzuprüfen)

am _____ _____ Dipl.-Vers._____
(Bevollmächtigter Beamter)

[ ] Datum des Eingangs dieses (vom Anmelder nachgereichten) Blattes beim Internationalen Büro[10]

_____
(Bevollmächtigter Beamter)

Formblatt PCT/RO/134 (Januar 1985)     Anmerkungen siehe Beiblatt     (Januar 1986)

**EP 0 334 841 B1**

# Formblatt für die Hinterlegung von Mikroorganismen

## M3

Internationales Aktenzeichen: PCT/ DE 87/00392

---

### MIKROORGANISMEN

Angaben über den auf Seite ..13.... Zeile .....25. der Beschreibung genannten Mikroorganismus[1]

---

**A. KENNZEICHNUNG DER HINTERLEGUNG[2]**

Weitere Hinterlegungen sind auf einem zusätzlichen Blatt angegeben [X] [3]

Name der Hinterlegungsstelle[4]

Deutsche Sammlung von Mikroorganismen (DSM)

---

Anschrift der Hinterlegungsstelle (einschließlich Postleitzahl und Land)[4]

Gisebachstr. 8, D- 3400 Göttingen, Deutschland

---

| Datum der Hinterlegung[5] | Eingangsnummer[6] |
|---|---|
| 28.08.1986 | DSM 3842 |

---

**B. WEITERE ANGABEN[7]** (die Angaben werden auf einem gesonderten Blatt fortgesetzt [ ] )

Für diejenigen Bestimmungen, in denen um ein europäisches Patent nachgesucht wird, wird bis zur Veröffentlichung des Hinweises auf die Erteilung des europäischen Patents oder bis zu dem Tag, an dem die Anmeldung zurückgewiesen oder zurückgenommen wird oder als zurückgenommen gilt, der Zugang zu dem hinterlegten Mikroorganismus nur durch Herausgabe einer Probe des Mikroorganismus an einen Sachverständigen hergestellt, der von der Person, die die Probe beantragt, benannt wird (Regel 28 Absatz 4 EPÜ).

---

**C. BESTIMMUNGSSTAATEN, FÜR DIE ANGABEN GEMACHT WERDEN[3]** (falls die Angaben nicht alle Bestimmungsstaaten betreffen)

---

**D. NACHREICHUNG VON ANGABEN[8]**

Folgende Angaben werden später beim Internationalen Büro eingereicht[9] (allgemeine Bezeichnung der Angaben, z.B. "Eingangsnummer der Hinterlegung")

---

E. [X] Dieses Blatt wurde zusammen mit der internationalen Anmeldung eingereicht (vom Anmeldeamt nachzuprüfen)

am 13. 11. 87

_____
(Bevollmächtigter Beamter)

[ ] Datum des Eingangs dieses (vom Anmelder nachgereichten) Blattes beim Internationalen Büro[10]

_____
(Bevollmächtigter Beamter)

---

Formblatt PCT/RO/134 (Januar 1985)     Anmerkungen siehe Beiblatt                    (Januar 1986)

# Formblatt für die Hinterlegung von Mikroorganismen

# M3

Internationales Aktenzeichen: PCT/    DE 87/00392

| MIKROORGANISMEN |
| --- |

Angaben über den auf Seite ..13..... , Zeile ..26.... der Beschreibung genannten Mikroorganismus[1]

### A. KENNZEICHNUNG DER HINTERLEGUNG[2]

Weitere Hinterlegungen sind auf einem zusätzlichen Blatt angegeben   [X] [3]

Name der Hinterlegungsstelle[4]

Deutsche Sammlung von Mikroorganismen (DSM)

Anschrift der Hinterlegungsstelle (einschließlich Postleitzahl und Land)[4]

Gisebachstr. 8, D- 3400 Göttingen, Deutschland

| Datum der Hinterlegung[5] | Eingangsnummer[6] |
| --- | --- |
| 28.08.1986 | 3843 |

### B. WEITERE ANGABEN[7] (die Angaben werden auf einem gesonderten Blatt fortgesetzt [ ])

Für diejenigen Bestimmungen, in denen um ein europäisches Patent nachgesucht wird, wird bis zur Veröffentlichung des Hinweises auf die Erteilung des europäischen Patents oder bis zu dem Tag, an dem die Anmeldung zurückgewiesen oder zurückgenommen wird oder als zurückgenommen gilt, der Zugang zu dem hinterlegten Mikroorganismus nur durch Herausgabe einer Probe des Mikroorganismus an einen Sachverständigen hergestellt, der von der Person, die die Probe beantragt, benannt wird (Regel 28 Absatz 4 EPÜ).

### C. BESTIMMUNGSSTAATEN, FÜR DIE ANGABEN GEMACHT WERDEN[3] (falls die Angaben nicht alle Bestimmungsstaaten betreffen)

### D. NACHREICHUNG VON ANGABEN[8]

Folgende Angaben werden später beim Internationalen Büro eingereicht[9] (allgemeine Bezeichnung der Angaben, z.B. "Eingangsnummer der Hinterlegung")

E. [X] Dieses Blatt wurde zusammen mit der internationalen Anmeldung eingereicht (vom Anmeldeamt nachzuprüfen)

am 19. 11. 87

Dipl.-Verwaltungswirt
R. KONWALLI
(Bevollmächtigter Beamter) Regierungsamtmann

[ ] Datum des Eingangs dieses (vom Anmelder nachgereichten) Blattes beim Internationalen Büro[10]

(Bevollmächtigter Beamter)

Formblatt PCT/RO/134 (Januar 1985)     Anmerkungen siehe Beiblatt     (Januar 1986)

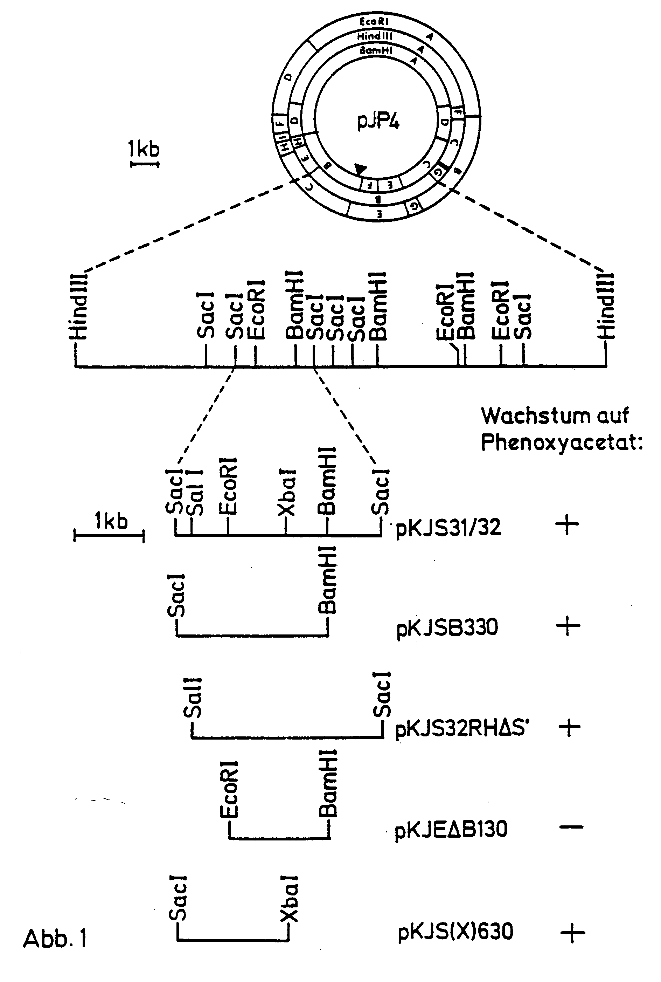

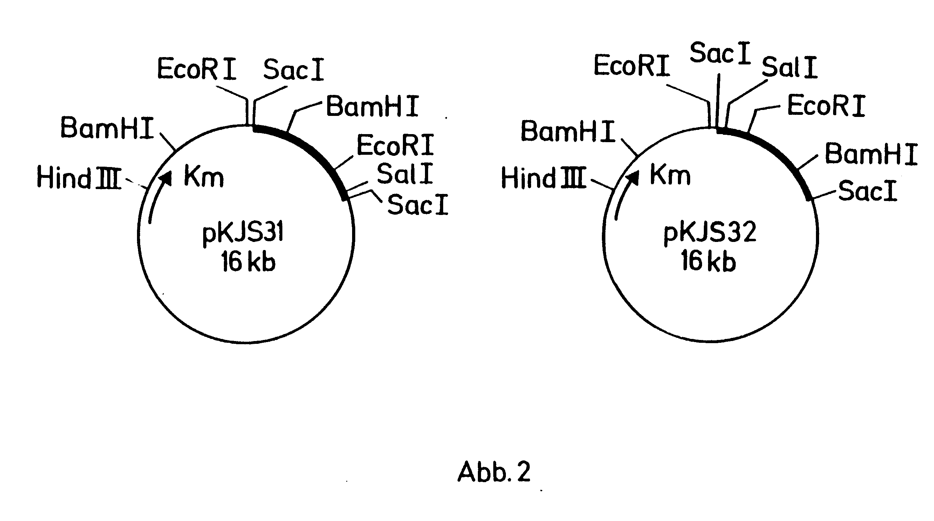

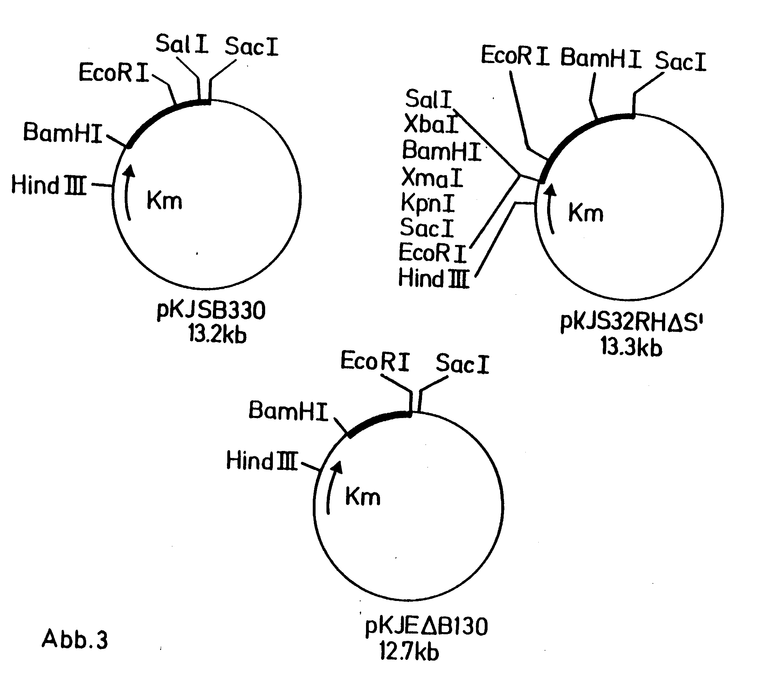

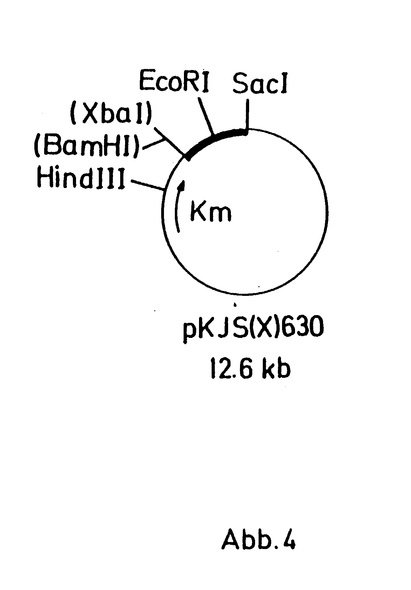

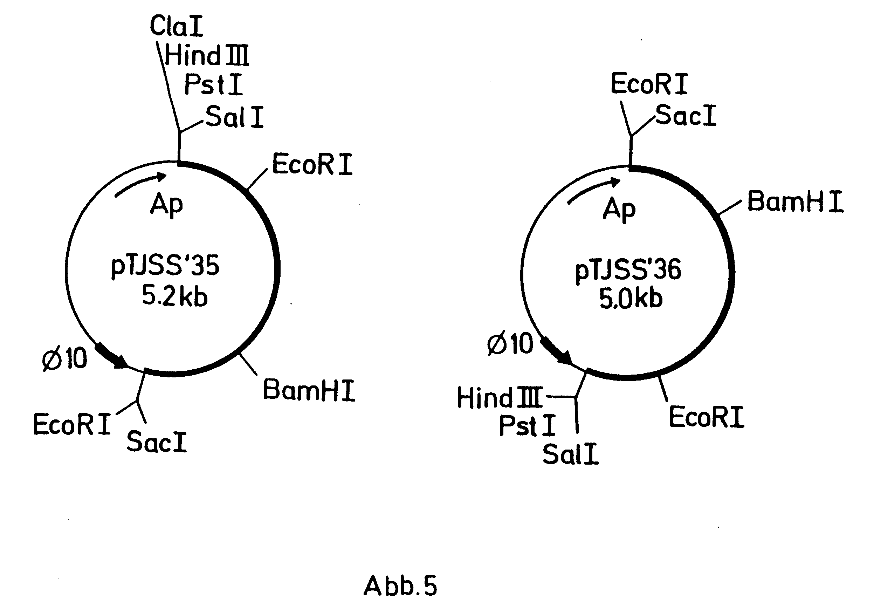

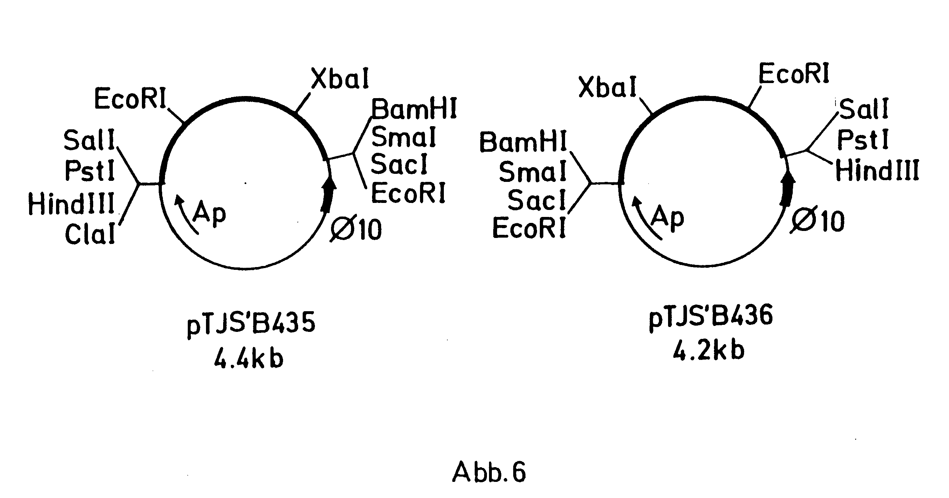

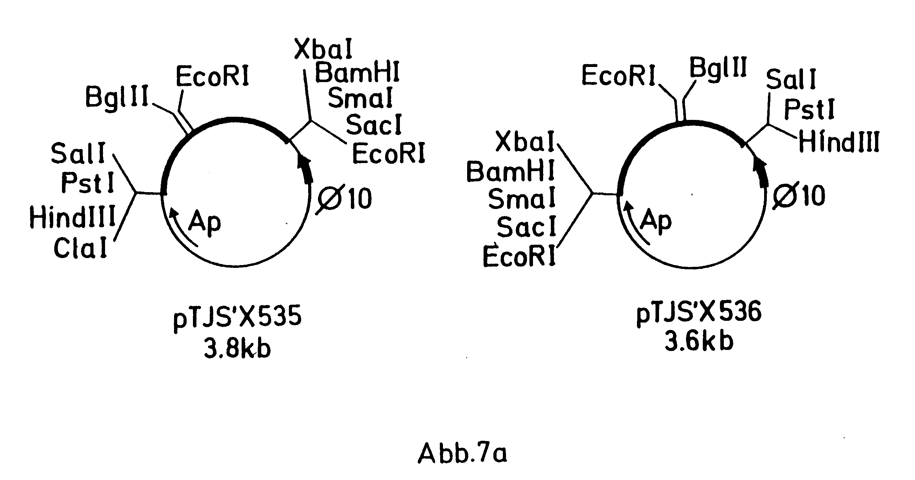

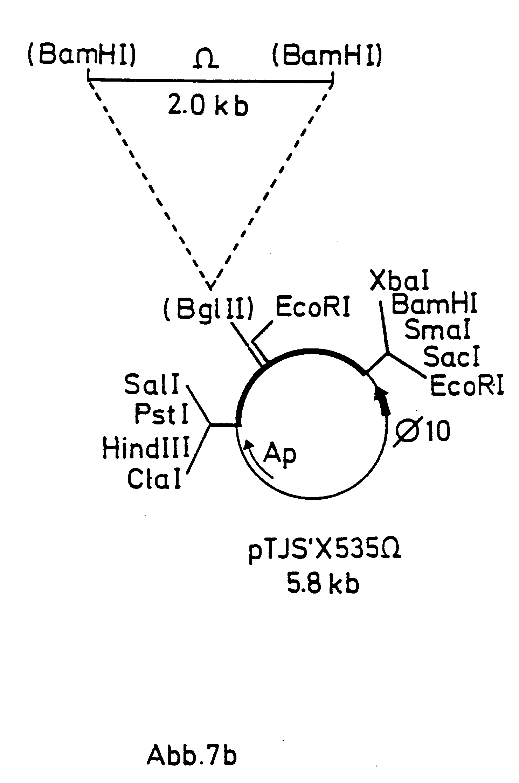

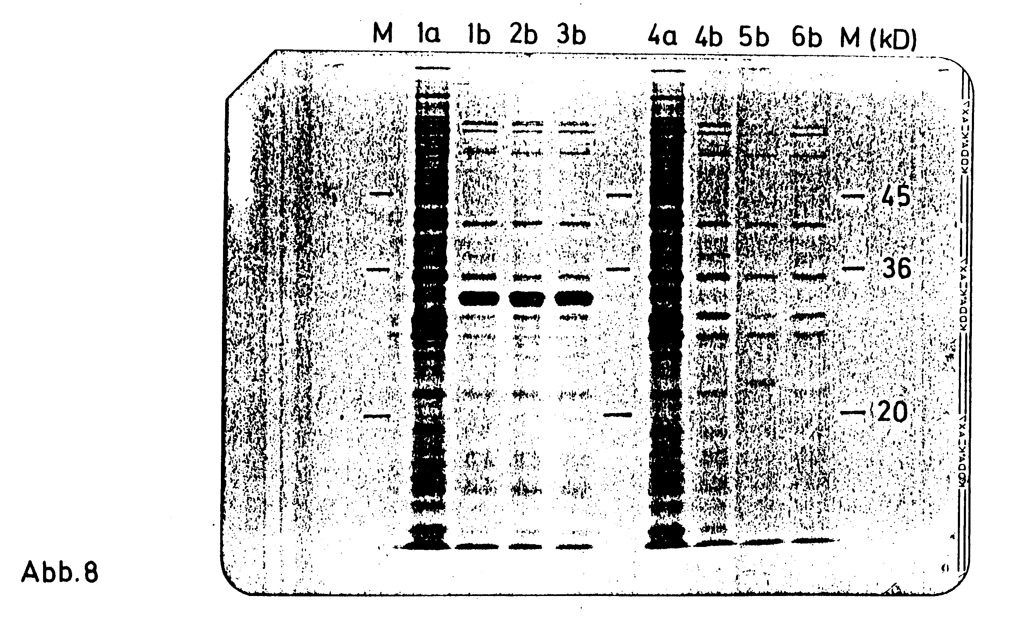

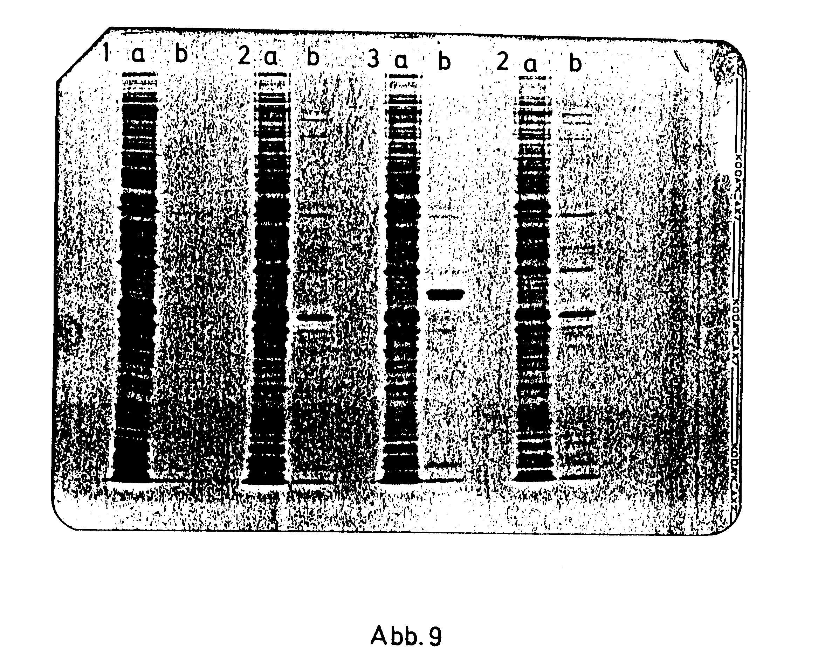

## Patentansprüche

1. tfdA-2,4-monooxigenase-Genmutante Alcaligenes eutrophus JMP 134:Tn 5-2 (DSM 3842).

2. tfdA-2,4-D-monooxigenase-Genmutante Alcaligenes eutrophus JMP 134:Tn 5-4 (DSM 3843).

**3.** Verwendung der tfdA-Mutanten Alcaligenes eutrophus JMP 134:Tn 5-2 und JMP 134:Tn 5-4 zur Identifizierung und Isolierung tfdA-Gene enthaltender Plasmide.

**4.** Rekombinierte DNA, dadurch gekennzeichnet, daß sie die für das 2,4-D spaltende Protein der angegebenen Aminosäuresequenz 1 bis 287 codierende Basensequenz 1 bis 861

```
                 .             .           .30          .              .          60
GTGAGCGTCGTCGCAAATCCCCTTCATCCTCTTTTCGCCGCAGGGGTCGAAGACATCGAC
ValSerValValAlaAsnProLeuHisProLeuPheAlaAlaGlyValGluAspIleAsp

                 .             .           90           .              .          120
CTTCGAGAGGCCTTGGGTTCGACCGAGGTCCGAGAGATCGAACGGCTAATGGACGAGAAG
LeuArgGluAlaLeuGlySerThrGluValArgGluIleGluArgLeuMetAspGluLys

                 .             .           150          .              .          180
TCGGTGCTGGTGTTCCGGGGGCAGCCCCTGAGTCAGGATCAGCAGATCGCCTTCGCGCGC
SerValLeuValPheArgGlyGlnProLeuSerGlnAspGlnGlnIleAlaPheAlaArg

                 .             .           210          .              .          240
AATTTCGGGCCACTCGAAGGCGGTTTCATCAAGGTCAATCAAAGACCTTCGAGATTCAAG
AsnPheGlyProLeuGluGlyGlyPheIleLysValAsnGlnArgProSerArgPheLys

                 .             .           270          .              .          300
TACGCGGAGTTGGCGGACATCTCGAACGTCAGTCTCGACGGCAAGGTCGCGCAACGCGAT
TyrAlaGluLeuAlaAspIleSerAsnValSerLeuAspGlyLysValAlaGlnArgAsp

                 .             .           330          .              .          360
GCGCGCGAGGTGGTCGGGAACTTCGCGAACCAGCTCTGGCACAGCGACAGCTCCTTTCAG
AlaArgGluValValGlyAsnPheAlaAsnGlnLeuTrpHisSerAspSerSerPheGln

                 .             .           390          .              .          420
CAACCTGCTGCCCGCTACTCGATGCTCTCCGCGGTGGTGGTTCCGCCGTCGGGCGGCGAC
GlnProAlaAlaArgTyrSerMetLeuSerAlaValValValProProSerGlyGlyAsp

                 .             .           450          .              .          480
ACCGAGTTCTGCGACATGCGTGCGGCATACGACGCGCTGCCTCGGGACCTCCAATCCGAG
ThrGluPheCysAspMetArgAlaAlaTyrAspAlaLeuProArgAspLeuGlnSerGlu
```

```
                                        510                          540
TTGGAAGGGCTGCGTGCCGAGCACTACGCACTGAACTCCCGCTTCCTGCTCGGCGACACC
LeuGluGlyLeuArgAlaGluHisTyrAlaLeuAsnSerArgPheLeuLeuGlyAspThr

                                        570                          600
GACTATTCGGAAGCGCAACGCAATGCCATGCCGCCGGTCAACTGGCCGCTGGTTCGAACC
AspTyrSerGluAlaGlnArgAsnAlaMetProProValAsnTrpProLeuValArgThr

                                        630                          660
CACGCCGGCTCCGGGCGCAAGTTTCTCTTCATCGGCGCGCACGCGAGCCACGTCGAAGGC
HisAlaGlySerGlyArgLysPheLeuPheIleGlyAlaHisAlaSerHisValGluGly

                                        690                          720
CTTCCGGTGGCCGAAGGCCGGATGCTGCTTGCGGAGCTTCTCGAGCACGCGACACAGCGG
LeuProValAlaGluGlyArgMetLeuLeuAlaGluLeuLeuGluHisAlaThrGlnArg

                                        750                          780
GAATTCGTGTACCGGCATCGCTGGAACGTGGGAGATCTGGTGATGTGGGACAACCGCTGC
GluPheValTyrArgHisArgTrpAsnValGlyAspLeuValMetTrpAspAsnArgCys

                                        810                          840
GTTCTTCACCGCGGACGCAGGTACGACATCTCGGCCAGGCGTGAGCTGCGCCGGGCGACC
ValLeuHisArgGlyArgArgTyrAspIleSerAlaArgArgGluLeuArgArgAlaThr

ACCCTGGACGATGCCGTCGTC
ThrLeuAspAspAlaValVal
```

oder ein nach dem genetischen Code für die gleiche Aminosäuresequenz codierendes Äquivalent davon enthält.

5. Plasmide, die ein Gen oder Subfragmente eines Gens gemäß Anspruch 4 enthalten.

6. Plasmid pVJH21, hergestellt gemäß Beispiel 1 aus den Plasmiden pJP4 und pVK101.

7. Plasmid pGJS3, hergestellt gemäß Beispiel 2 aus den Plasmiden PVJH21 und PGSS33.

8. Plasmid pKJS31, hergestellt aus den Plasmiden pGJS3 und pKT231 (hinterlegt in E. coli S17-1, DSM 3835)

9. Plasmid pKJS32, hergestellt gemäß Beispiel 3 aus den Plasmiden pGJS3 und pKT231.

10. Plasmid pKJSB330, hergestellt gemäß Beispiel 4 aus dem Plasmid pKJS31.

11. Plasmid pKJS(X)630, hergestellt aus dem Plasmid gemäß Anspruch 10 (hinterlegt in E. coli S17-1, DSM 3837).

12. Plasmid pKJEΔB130, hergestellt gemäß Beispiel 6 aus den Plasmiden pKT 231 und pKJS32.

13. Plasmid pTJS'B435, hergestellt gemäß Beispiel 9 aus den Plasmiden pT7-5 und pKJSB330.

14. Plasmid pTJS'B436, hergestellt gemäß Beispiel 9 aus den Plasmiden pT7-6 und pKJSB330.

15. Plasmid pKJS32RH S', hergestellt aus den Plasmiden pKJS32 und pRME1 (hinterlegt in E. coli S17-1, DSM 3836).

**16.** Plasmid pTJSS'035, hergestellt aus den Plasmiden pT7-5 und pKJS32 (hinterlegt in E. coli LE 392, DSM 3832).

**17.** Plasmid pTJSS'036, hergestellt gemäß Beispiel 8 aus den Plasmiden pT7-6 und pKJS32.

**18.** Plasmid pTJS'535, hergestellt aus den Plasmiden pT7-5 und pTJSS'035 (hinterlegt als E. coli K38 (pGT1-2/pTJSX535), DSM 3839)

**19.** Plasmid pTJS'X536, hergestellt gemäß Beispiel 10 aus den Plasmiden pT7-6 und pTJSS'035.

**20.** Plasmid pTJS'X535omega, hergestellt gemäß Beispiel 11 aus den Plasmiden pTJS'X535 und pDOC37.

**21.** Phagen MJSS'030, hergestellt gemäß Beispiel 12 aus dem Phagen M13tg130 und dem Plasmid pKJS32.

**22.** Phagen MJSS'031, hergestellt gemäß Beispiel 12 aus dem Phagen M13tg131 und dem Plasmid pKJS32.

**23.** E. coli-Stämme, enthaltend Plasmide nach Anspruch 5.

**24.** Pseudomonas-Stämme, enthaltend Plasmide nach Anspruch 5, dadurch gekennzeichnet, daß diese Stämme 4-Chlorphenoxyessigsäure verwerten können.

**25.** Verwendung von tfdA-Genen oder Teilen dieser Gene enthaltenden Plasmide als Ausgangsprodukt zur Herstellung von 2,4-D-abbauenden Pflanzen, dadurch gekennzeichnet, daß diese Pflanzen gegen die Wuchshemmwirkung von 2,4-D resistent sind.

**Claims**

**1.** tfdA-2,4-monooxygenase gene mutant Alcaligenes eutrophus JMP 134:Tn 5-2 (DSM 3842).

**2.** tfdA-2,4-D-monooxygenase gene mutant Alcaligenes eutrophus JMP 134:Tn 5-4 (DSM 3843).

**3.** Use of the tfdA-mutants Alcaligenes eutrophus JMP 134:Tn 5-2 and JMP134:Tn 5-4 for the identification and isolation of plasmids comprising tfdA-genes.

**4.** Recombined DNA characterized by containing the 2,4-D-degrading protein of the indicated amino acid sequenz 1 to 287 of the coding base sequence 1 to 861

```
                .              .             .30            .              .            60
GTGAGCGTCGTCGCAAATCCCCTTCATCCTCTTTTCGCCGCAGGGGTCGAAGACATCGAC
ValSerValValAlaAsnProLeuHisProLeuPheAlaAlaGlyValGluAspIleAsp

                .              .             90            .              .           120
CTTCGAGAGGCCTTGGGTTCGACCGAGGTCCGAGAGATCGAACGGCTAATGGACGAGAAG
LeuArgGluAlaLeuGlySerThrGluValArgGluIleGluArgLeuMetAspGluLys

                .              .            150            .              .           180
TCGGTGCTGGTGTTCCGGGGGCAGCCCCTGAGTCAGGATCAGCAGATCGCCTTCGCGCGC
SerValLeuValPheArgGlyGlnProLeuSerGlnAspGlnGlnIleAlaPheAlaArg

                .              .            210            .              .           240
AATTTCGGGCCACTCGAAGGCGGTTTCATCAAGGTCAATCAAAGACCTTCGAGATTCAAG
AsnPheGlyProLeuGluGlyGlyPheIleLysValAsnGlnArgProSerArgPheLys

                .              .            270            .              .           300
TACGCGGAGTTGGCGGACATCTCGAACGTCAGTCTCGACGGCAAGGTCGCGCAACGCGAT
TyrAlaGluLeuAlaAspIleSerAsnValSerLeuAspGlyLysValAlaGlnArgAsp

                .              .            330            .              .           360
GCGCGCGAGGTGGTCGGGAACTTCGCGAACCAGCTCTGGCACAGCGACAGCTCCTTTCAG
AlaArgGluValValGlyAsnPheAlaAsnGlnLeuTrpHisSerAspSerSerPheGln

                .              .            390            .              .           420
CAACCTGCTGCCCGCTACTCGATGCTCTCCGCGGTGGTGGTTCCGCCGTCGGGCGGCGAC
GlnProAlaAlaArgTyrSerMetLeuSerAlaValValValProProSerGlyGlyAsp

                .              .            450            .              .           480
ACCGAGTTCTGCGACATGCGTGCGGCATACGACGCGCTGCCTCGGGACCTCCAATCCGAG
ThrGluPheCysAspMetArgAlaAlaTyrAspAlaLeuProArgAspLeuGlnSerGlu
```

45

```
                                     510              540
ITGGAAGGGCTGCGTGCCGAGCACTACGCACTGAACTCCCGCTTCCTGCTCGGCGACACC
LeuGluGlyLeuArgAlaGluHisTyrAlaLeuAsnSerArgPheLeuLeuGlyAspThr

                                     570              600
GACTATTCGGAAGCGCAACGCAATGCCATGCCGCCGGTCAACTGGCCGCTGGTTCGAACC
AspTyrSerGluAlaGlnArgAsnAlaMetProProValAsnTrpProLeuValArgThr

                                     630              660
CACGCCGGCTCCGGGCGCAAGTTTCTCTTCATCGGCGCGCACGCGAGCCACGTCGAAGGC
HisAlaGlySerGlyArgLysPheLeuPheIleGlyAlaHisAlaSerHisValGluGly

                                     690              720
CTTCCGGTGGCCGAAGGCCGGATGCTGCTTGCGGAGCTTCTCGAGCACGCGACACAGCGG
LeuProValAlaGluGlyArgMetLeuLeuAlaGluLeuLeuGluHisAlaThrGlnArg

                                     750              780
GAATTCGTGTACCGGCATCGCTGGAACGTGGGAGATCTGGTGATGTGGGACAACCGCTGC
GluPheValTyrArgHisArgTrpAsnValGlyAspLeuValMetTrpAspAsnArgCys

                                     810              840
GTTCTTCACCGCGGACGCAGGTACGACATCTCGGCCAGGCGTGAGCTGCGCCGGGCGACC
ValLeuHisArgGlyArgArgTyrAspIleSerAlaArgArgGluLeuArgArgAlaThr

ACCCTGGACGATGCCGTCGTC
ThrLeuAspAspAlaValVal
```

or an equivalent thereof, coding according to the genetic code of the same amino acid sequence.

5. Plasmids comprising a gene or subfragments of a gene, according to claim 4.

6. Plasmid pVJH21 prepared from the plasmids pJP4 and pVK101, according to Example 1.

7. Plasmid pGJS3,prepared from the plasmids PVJH21 and PGSS33, according to Example 2.

8. Plasmid pKJS31,prepared from the plasmids pGJS3 and PKT231 (deposited in E. Coli S17-1, DSM 3835).

9. Plasmid pKJS32, prepared from the plasmids pGJS3 and pKT231, according to Example 3.

10. Plasmid pKJSB330, prepared from the plasmid pKJS31, according to Example 4.

11. Plasmid pKJS(X)630, prepared from the plasmid according to claim 10 (deposited in E. coli S17-1, DSM 3837).

12. Plasmid PKJEΔB130,prepared from the plasmids pKT 231 and pKJS32, according to Example 6.

13. Plasmid pTJS'B435, prepared from the plasmids pT7-5 and pKJSB330, according to Example 9.

14. Plasmid pTJS'B436,prepared from the plasmids pT7-6 and pKJSB330, according to Example 9.

15. Plasmid pKJS32RH S',prepared from the plasmids pKJS32 and pRME1 (deposited in E. coli S17-1, DSM 3836).

**16.** Plasmid pTJSS'035,prepared from the plasmids pT7-5 and pKJS32 (deposited in E. coli LE 392, DSM 3832).

**17.** Plasmid pTJSS'036,prepared from the plasmids pT7-6 and pKJS32, according to Example 8.

**18.** Plasmid pTJS'535,prepared from the plasmids pT7-5 and pTJSS'035 (deposited as E. coli K38 (pGT1-2/pTJSX535), DSM 3839).

**19.** Plasmid pTJS'X536,prepared from the plasmids pT7-6 and pTJSS'035, according to Example 10.

**20.** Plasmid pTJS'X535omega,prepared from the plasmids pTJS'X535 and pDOC37, according to Example 11.

**21.** Phages MJSS'030,prepared from the phages M13tg130 and the plasmid pKJS32, according to Example 12.

**22.** Phages MJSS'031,prepared from the phages M13tg131 and the plasmid pKJS32, according to Example 12.

**23.** E. coli strains comprising plasmids according to claim 5.

**24.** Pseudomonas strains comprising plasmids according to claim 5, characterized by these strains can utilize 4-chlorophenoxyacetic acid.

**25.** Use of tfdA-genes or fragments of these genes comprising plasmids as starting product for the preparation of 2,4-D-degrading plants, characterized by these plants are resistant to the growth inhibition activity of 2,4-D.

**Revendications**

**1.** Mutant de gène de tfdA-2,4-monooxygénase Alcaligenes eutrophus JMP 134:Tn 5-2 (DSM 3842).

**2.** Mutant de gène de tfdA-2,4-D-monooxygénase Alcaligenes eutrophus JMP 134:Tn 5-4 (DSM 3843).

**3.** Emploi des mutants tfdA Alcaligenes eutrophus JMP 134:Tn 5-2 et JMP 134:Tn 5-4 pour identifier et isoler des plasmides contenant des gènes de tfdA.

**4.** ADN recombinié caractérisé en ce qu'il comprend la séquence de bases 1 à 861 codant pour la protéine dissociant 2,4D, de la séquence des aminoacides indiquée 1 à 287.

```
               .              .       .30              .              .    60
   GTGAGCGTCGTCGCAAATCCCCTTCATCCTCTTTTCGCCGCAGGGGTCGAAGACATCGAC
   ValSerValValAlaAsnProLeuHisProLeuPheAlaAlaGlyValGluAspIleAsp

               .              .      90               .              .   120
   CTTCGAGAGGCCTTGGGTTCGACCGAGGTCCGAGAGATCGAACGGCTAATGGACGAGAAG
   LeuArgGluAlaLeuGlySerThrGluValArgGluIleGluArgLeuMetAspGluLys

               .              .     150              .              .   180
   TCGGTGCTGGTGTTCCGGGGGCAGCCCCTGAGTCAGGATCAGCAGATCGCCTTCGCGCGC
   SerValLeuValPheArgGlyGlnProLeuSerGlnAspGlnGlnIleAlaPheAlaArg

               .              .     210              .              .   240
   AATTTCGGGCCACTCGAAGGCGGTTTCATCAAGGTCAATCAAAGACCTTCGAGATTCAAG
   AsnPheGlyProLeuGluGlyGlyPheIleLysValAsnGlnArgProSerArgPheLys

               .              .     270              .              .   300
   TACGCGGAGTTGGCGGACATCTCGAACGTCAGTCTCGACGGCAAGGTCGCGCAACGCGAT
   TyrAlaGluLeuAlaAspIleSerAsnValSerLeuAspGlyLysValAlaGlnArgAsp

               .              .     330              .              .   360
   GCGCGCGAGGTGGTCGGGAACTTCGCGAACCAGCTCTGGCACAGCGACAGCTCCTTTCAG
   AlaArgGluValValGlyAsnPheAlaAsnGlnLeuTrpHisSerAspSerSerPheGln

               .              .     390              .              .   420
   CAACCTGCTGCCCGCTACTCGATGCTCTCCGCGGTGGTGGTTCCGCCGTCGGGCGGCGAC
   GlnProAlaAlaArgTyrSerMetLeuSerAlaValValValProProSerGlyGlyAsp

               .              .     450              .              .   480
   ACCGAGTTCTGCGACATGCGTGCGGCATACGACGCGCTGCCTCGGGACCTCCAATCCGAG
   ThrGluPheCysAspMetArgAlaAlaTyrAspAlaLeuProArgAspLeuGlnSerGlu
```

48

```
                                          510                            540
TTGGAAGGGCTGCGTGCCGAGCACTACGCACTGAACTCCCGCTTCCTGCTCGGCGACACC
LeuGluGlyLeuArgAlaGluHisTyrAlaLeuAsnSerArgPheLeuLeuGlyAspThr

                                          570                            600
GACTATTCGGAAGCGCAACGCAATGCCATGCCGCCGGTCAACTGGCCGCTGGTTCGAACC
AspTyrSerGluAlaGlnArgAsnAlaMetProProValAsnTrpProLeuValArgThr

                                          630                            660
CACGCCGGCTCCGGGCGCAAGTTTCTCTTCATCGGCGCGCACGCGAGCCACGTCGAAGGC
HisAlaGlySerGlyArgLysPheLeuPheIleGlyAlaHisAlaSerHisValGluGly

                                          690                            720
CTTCCGGTGGCCGAAGGCCGGATGCTGCTTGCGGAGCTTCTCGAGCACGCGACACAGCGG
LeuProValAlaGluGlyArgMetLeuLeuAlaGluLeuLeuGluHisAlaThrGlnArg

                                          750                            780
GAATTCGTGTACCGGCATCGCTGGAACGTGGGAGATCTGGTGATGTGGGACAACCGCTGC
GluPheValTyrArgHisArgTrpAsnValGlyAspLeuValMetTrpAspAsnArgCys

                                          810                            840
GTTCTTCACCGCGGACGCAGGTACGACATCTCGGCCAGGCGTGAGCTGCGCCGGGCGACC
ValLeuHisArgGlyArgArgTyrAspIleSerAlaArgArgGluLeuArgArgAlaThr

ACCCTGGACGATGCCGTCGTC
ThrLeuAspAspAlaValVal
```

ou un équivalent de cette séquence codant pour la même séquence d'aminoacides suivant le code génétique.

5. Plasmides contenant un gène ou des sousfragments d'un gène selon la revendication 4.

6. Plasmide pVJH21, formé suivant l'exemple 1 à partir des plasmides pJP4 et pVK101.

7. Plasmide pGJS3, formé suivant l'exemple 2 à partir des plasmides PVJH21 et PGSS33.

8. Plasmide pKJS31, formé à partir des plasmides pGJS3 et pKT231 (dépôt en E. coli S17-1, DSM 3835).

9. Plasmide pKJS32, formé suivant l'exemple 3 à partir des plasmides pGJS3 et pKT231.

10. Plasmide pKJSB330, formé suivant l'exemple 4 à partir du plasmide pKJS31.

11. Plasmide pKJS(X)630, formé à partir du plasmide de la revendication 10 (dépôt en E. coli S17-1, DSM 3837).

12. Plasmide pKJEΔB130, formé suivant l'exemple 6 à partir des plasmides pKT231 et pKJS32.

13. Plasmide pTJS'B435, formé suivant l'exemple 9 à partir des plasmides pT7-5 et pKJSB330.

14. Plasmide pTJS'B436, formé suivant l'exemple 9 à partir des plasmides pT7-6 et pKJSB330.

15. Plasmide pKJS32RH S', formé à partir des plasmides pKJS32 et pRME1 (dépôt en E. coli S17-1, DSM 3836).

16. Plasmide pTJSS'035, formé à partir des plasmides pT7-5 et pKJS32 (dépôt en E. coli LE 392, DSM 3832).

**17.** Plasmide pTJSS'036, formé suivant l'exemple 8 à partir des plasmides pT7-6 et pKJS32.

**18.** Plasmide pTJS'535, formé à partir des plasmides pT7-5 et pTJSS'035 (dépôt en E. coli K38 (pGT1-2/pTJSX535), DSM 3839).

**19.** Plasmide pTJS'X536, formé suivant l'exemple 10 à partir des plasmides pT7-6 et pTJSS'035.

**20.** Plasmide pTJS'X535oméga, formé suivant l'exemple 11 à partir des plasmides pTJS'X535 et pDOC37.

**21.** Phage MJSS'030, formé suivant l'exemple 12 à partir du phage M13tg130 et du plasmide pKJS32.

**22.** Phage MJSS'031, formé suivant l'exemple 12 à partir du phage M13tg131 et du plasmide pKJS32.

**23.** Souches de E. coli contenant des plasmides de la revendication 5.

**24.** Souches de Pseudomonas contenant des plasmides de la revendication 5, caractérisées en ce qu'elles peuvent exploiter l'acide 4-chlorophénoxyacétique.

**25.** L'emploi de plasmides contenant des gènes de tfdA ou des parties de ces gènes pour obtenir des plantes dégradant 2,4-D, caractérisé en ce ces plantes sont résistantes à l'action inhibitrice de croissance de 2,4-D.

Abb. 1

Abb. 2

Abb.3

EP 0 334 841 B1

EcoRI SacI
(XbaI)
(BamHI)
HindIII

Km

pKJS(X)630

12.6 kb

Abb.4

EP 0 334 841 B1

Abb.5

EP 0 334 841 B1

pTJS'B435
4.4kb

pTJS'B436
4.2kb

Abb. 6

pTJS'X535
3.8kb

pTJS'X536
3.6kb

Abb.7a

EP 0 334 841 B1

(BamHI)  Ω  (BamHI)

2.0 kb

(BgI II)  EcoRI

XbaI
BamHI
SmaI
SacI
EcoRI

SalI
PstI
HindIII
ClaI

Ap

Ø10

pTJS'X535Ω
5.8 kb

Abb.7b

M  1a  1b  2b  3b    4a  4b  5b  6b  M (kD)

— 45

— 36

— 20

Abb. 8

Abb. 9